(19) Europäisches Patentamt
European Patent Office
Office européen des brevets

(11) **EP 4 528 281 A2**

(12) **EUROPEAN PATENT APPLICATION**

(43) Date of publication:
**26.03.2025   Bulletin 2025/13**

(21) Application number: 25153368.3

(22) Date of filing: **19.02.2021**

(51) International Patent Classification (IPC):
***G01N 33/74*** *(2006.01)*

(52) Cooperative Patent Classification (CPC):
**G01N 33/6893; C07K 16/22; G01N 33/6896;
G01N 33/74;** G01N 2333/51; G01N 2800/2821;
G01N 2800/2871; G01N 2800/325; G01N 2800/326

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **20.02.2020   EP 20158636**

(62) Document number(s) of the earlier application(s) in
accordance with Art. 76 EPC:
**21705555.7 / 4 107 182**

(71) Applicants:
• **Universiteit Maastricht**
**6211 LK  Maastricht (NL)**
• **F. Hoffmann-La Roche AG**
**4070 Basel (CH)**
Designated Contracting States:
**AL AT BE BG CH CY CZ DK EE ES FI FR GB GR
HR HU IE IS IT LI LT LU LV MC MK MT NL NO PL
PT RO RS SE SI SK SM TR**
• **Roche Diagnostics GmbH**
**68298 Mannheim (DE)**
Designated Contracting States:
**DE**
• **Academisch ziekenhuis Maastricht**
**6229 HX Maastricht (NL)**

(72) Inventors:
• **Engel, Alfred**
**82377 Penzberg (DE)**
• **Gerg, Michael**
**82377 Penzberg (DE)**
• **Jucknischke, Ute**
**82377 Penzberg (DE)**

• **Karl, Johann**
**82377 Penzberg (DE)**
• **Kastner, Peter**
**82377 Penzberg (DE)**
• **Meier, Thomas**
**82377 Penzberg (DE)**
• **Hillringhaus, Lars**
**82377 Penzberg (DE)**
• **Schotten, Ulrich**
**6229 GT Maastricht (NL)**
• **Rolny, Vinzent**
**82377 Penzberg (DE)**
• **Wienhues-Thelen, Ursula-Henrike**
**82377 Penzberg (DE)**
• **Ziegler, André**
**6343 Rotkreuz (CH)**
• **Latini, Roberto**
**20129 Milano (IT)**
• **Meessen, Jennifer Marie Theresia Anna**
**20129 Milano (IT)**

(74) Representative: **Altmann Stößel Dick
Patentanwälte PartG mbB
Theodor-Heuss-Anlage 2
68165 Mannheim (DE)**

Remarks:
•The complete document including Reference
Table(s) and the Sequence Listing(s) can be
downloaded from the EPO website
•This application was filed on 22-01-2025 as a
divisional application to the application mentioned
under INID code 62.

(54) **DETECTION METHOD OF CIRCULATING BMP10 (BONE MORPHOGENETIC PROTEIN 10)**

(57)     The present invention relates to a method for assessing atrial fibrillation in a subject, said method comprising the steps of determining the amount of BMP10 in a sample from the subject, and comparing the amount of BMP10 to a reference amount, whereby atrial fibrillation is to be assessed. Moreover, the present invention relates to a method for diagnosing heart failure based on the determination of BMP10 in a sample from a subject. Further, the present invention relates to a method for predicting the risk of a subject of hospitalization due to heart failure based on the determination of a BMP10-type peptide in a sample from a subject. The present invention further pertains to antibodies which bind to one or more BMP10-type peptides such as NT-proBMP10.

EP 4 528 281 A2

**Figure 1**

**Description**

The present invention relates to a method for assessing atrial fibrillation in a subject, said method comprising the steps of determining the amount of one or more BMP10-type peptides in a sample from the subject, and comparing the amount of the one or more BMP10-type peptides to a reference amount, whereby atrial fibrillation is to be assessed. Moreover, the present invention relates to a method for diagnosing heart failure based on the determination of one or more BMP10-type peptides in a sample from a subject. Further, the present invention relates to a method for predicting the risk of a subject of hospitalization due to heart failure based on the determination of one or more BMP10-type peptides in a sample from a subject. The present invention further pertains to antibodies which bind to one or more BMP10-type peptides such as to NT-proBMP10.

BACKGROUND SECTION

Atrial fibrillation (AF) is the most common type of heart arrhythmia and one of the most widespread conditions among the elderly population. Atrial fibrillation is characterized by irregular heart beating and often starts with brief periods of abnormal beating that can increase over time and may become a permanent condition. An estimated 2.7-6.1 million people in the United States have Atrial Fibrillation and approximately 33 million people globally (Chugh S.S. et al., Circulation 2014;129:837-47).

The diagnosis of heart arrhythmia such as atrial fibrillation typically involves determination of the cause of the arrhythmia, and classification of the arrhythmia. Guidelines for the classification of atrial fibrillation according to the American College of Cardiology (ACC), the American Heart Association (AHA), and the European Society of Cardiology (ESC) are mainly based on simplicity and clinical relevance. The first category is called "first detected AF". People in this category are initially diagnosed with AF and may or may not have had previous undetected episodes. If a first detected episode stops on its own in less than one week, but is followed by another episode later on, the category changes to "paroxysmal AF". Although patients in this category have episodes lasting up to 7 days, in most cases of paroxysmal AF the episodes will stop in less than 24 hours. If the episode lasts for more than one week, it is classified as "persistent AF". If such an episode cannot be stopped, i.e. by electrical or pharmacologic cardioversion, and continues for more than one year, the classification is changed to "permanent AF". An early diagnosis of atrial fibrillation is highly desired because atrial fibrillation is an important risk factor for stroke and systemic embolism (Hart et al., Ann Intern Med 2007; 146(12): 857-67; Go AS et al. JAMA 2001; 285(18): 2370-5). Stroke ranks after ischemic heart disease second as a cause of lost disability -adjusted - life years in high income countries and as a cause of death worldwide. In order to reduce the risk of stroke, anticoagulation therapy appears the most appropriate therapy.

Biomarkers which allow for the assessment of atrial fibrillation are highly desired.

Latini R. et al. (J Intern Med. 2011 Feb; 269(2): 160-71) measured various circulating biomarkers (hsTnT, NT-proBNP, MR-proANP, MR-proADM, copeptin, and CT-proendothelin-1) in patients with atrial fibrillation.

Bone Morphogenetic Protein 10 (abbreviated BMP10) is a ligand of the TGF-beta (transforming growth factor-beta) superfamily of proteins. Ligands of this family bind various TGF-beta receptors leading to recruitment and activation of certain transcription factors that regulate gene expression. BMP10 binds to the activin receptor-like kinase 1 (ALK1) and has been shown to be a functional activator of this kinase in endothelial cells (David et al., Blood. 2007, 109(5):1953-61).

Bone Morphogenetic Proteins are circulating in blood in different forms (uncleaved, processed or complexed). Kienast et.al., J. Biol. Chem. (2016) 293(28) 10963-10974 examined circulating variants of BMP9 and found that mature BMP9 represents only 0.5% of total BMP9 in human plasma compared to the other BMP9 variants.

Human preproBMP10 comprises a short signal peptide (amino acids 1 to 21) which is enzymatically cleaved off to release proBMP10, an inactive precursor protein (amino acids 22 to 424 of human preproBMP10). proBMP10 is cleaved by proteases resulting in the non-glycosylated C-terminal peptide of 108 aa (~14 kDa; BMP10, amino acids 317 to 424) and an N-terminal prosegment of ~50 kDa (amino acids 22 to 424, Susan-Resiga et al., J Biol Chem. 2011 Jul 1;286(26):22785-94). Both the mature BMP10 and the N-terminal prosegment of BMP-10 remain in structural proximity forming homo- or hetero-dimers of BMP10 or in combination with other BMP-family proteins (Yadin et al., CYTOGFR 2016, 27 (2016) 13-34). The dimerization occurs by formation of Cys-Cys bridge or strong adhesion in the C-terminal peptides of both binding partners. Thus, an architecture consisting of two subunits is formed.

US 8,287,868 discloses an isolated monoclonal antibody that binds to a mature humanized or fully human antibody BMP10 peptide and competes with BMP10 propeptide for binding to the mature BMP10. Further antibodies against BMP10 or precursors thereof are disclosed in US 5,932,216.

It has been shown that BMP10 plays a role in cardiovascular development including cardiomyocyte proliferation and regulation of heart size, closure of the ductus arteriosus, angiogenesis and ventricular trabeculation.

Being involved in the regulation of tissue repair, soluble BMP10 has been found as a diagnostic and treatment target involved in tissue fibrosis also in cardiovascular diseases (see e.g. US2013209490) Involvement of BMP10 has been described in vascular fibrosis and cardiac fibrosis.

US 2012/0213782 discloses BMP10 propeptides can be used for treating heart disorders.

A general role of BMP10 is in the developmental regulation of vascular remodeling (Ricard et al., Blood. 2012 Jun 21; 119(25): 6162-6171). Moreover, BMP10 is a heart developmental factor (Huang et al., J Clin Invest. 2012;122(10):3678-3691) and induces cardiomyocyte proliferation upon myocardial infarction (Sun et al., J Cell Biochem. 2014;115(11):1868-1876). It is described to also originate from endothelial cells (Jiang et al., JBC 2016, 291(6): 2954-2966).

Transcriptomic analyses reveal that BMP10 mRNA in healthy conditions is strongly expressed in heart right atrium and right atrial appendage. It is expressed predominantly in the right compared to the left atrial appendage (Kahr et al., Plos ONE, 2010, 6(10): e26389).

During the ESC (European Society of Cardiology) Congress held in Paris from August 29, 2019 to September 2, 2019, Larissa Fabritz held a presentation on biomarkers in atrial fibrillation. One of the mentioned biomarkers was BMP (FP Number: 2365).

International patent application PCT/EP2019/072042 discloses circulating BMP10 (Bone Morphogenetic Protein 10) in the assessment of Atrial Fibrillation.

There is a need for reliable methods for the assessment of atrial fibrillation including the diagnosis of atrial fibrillation, the risk stratification of patients with atrial fibrillation (such as occurrence of stroke), the assessment of the severity of atrial fibrillation, and the assessment of a therapy in patients with atrial fibrillation. Furthermore, there is a need for reliably assessing heart failure.

Tillet et.al. (J. Biol. Chem. (2018) 293(28) 10963-10974) found that a heterodimer of BMP9 and BMP10 is responsible for most of the biological BMP activity found in plasma. However, Tillet et al. found lower levels of BMP10 in plasma and suggested the presence of masking proteins in plasma.

The technical problem underlying the present invention can be seen as the provision of methods for complying with the aforementioned needs. The technical problem is solved by the embodiments characterized in the claims and herein below.

Advantageously, it was found in the context of the studies of the present invention that the determination of the amount of one or more BMP10-type peptides in a sample from a subject allows for an improved assessment of atrial fibrillation and heart failure. Therefore, it can be e.g. diagnosed whether a subject suffers from atrial fibrillation or heart failure, is at risk of suffering from stroke associated with atrial fibrillation, or is at risk for recurrent Afib after therapeutic interventions.

Further, it was shown in the studies described herein that amount of one or more BMP10-type peptides correlates with existence of white matter lesions (WML) in patients. Since WML extent can be caused by clinically silent strokes (Wang Y, Liu G, Hong D, Chen F, Ji X, Cao G. White matter injury in ischemic stroke. Prog Neurobiol. 2016;141:45-60), the BMP10-type peptides can be used for the assessment of the extent of white matter lesions and for the assessment whether a subject has experienced one or more silent strokes, i.e. clinically silent strokes, in the past. Since WMI, are associated with the risk of prediction of dementia, the one or more BMP10-type peptides also allow the prediction of dementia, such as vascular dementia and/or Alzheimer's disease.

Advantageously, it was found in the studies described herein that the detection of BMP10-type peptides in blood, serum or plasma samples is superior, when using antibodies against amino acid region 22 to 316 of human preproBMP10 (i.e. against the N-terminal prosegment of BMP10) as compared to when using antibodies against the mature BMP10 hormone itself. Therefore, the use of detection agents which bind within amino acid region 22 to 316 of human preproBMP10, rather than to mature BMP10, allows for an improved assessment of atrial fibrillation and heart failure. Furthermore, the inventors have identified subregions within amino acid region 22 to 316 of human preproBMP10 which are particularly suitable target regions for detection agents. In this context, antibodies were identified which allow for an improved detection of BMP10-type peptides. The antibodies are capable of binding NT-proBMP10 and any BMP10-type peptides which comprise the NT-proBMP10 fragment, such as preproBMP 10 and proBMP 10.

SUMMARY OF THE PRESENT INVENTION

The present invention relates to a method for assessing atrial fibrillation in a subject, comprising the steps of

   a) determining, in at least one sample from the subject, the amount of one or more BMP10-type peptides (Bone Morphogenetic Protein 10-type peptides) and, optionally, the amount of at least one further biomarker selected from the group consisting of a natriuretic peptide, ESM-1 (Endocan), Ang2 (Angiopoietin 2) and FABP3 (Fatty Acid Binding Protein 3), and

   b) comparing the amount of the one or more BMP10-type peptides to a reference amount for the one or more BMP10-type peptides and, optionally, comparing the amount of the at least one further biomarker to a reference amount for said at least one further biomarker, whereby atrial fibrillation is to be assessed.

The present invention further relates to a method of aiding in the assessment of atrial fibrillation, said method comprising the steps of:

a) providing at least one sample from a subject,

b) determining, in the at least one sample provided in step a), the amount of one or more BMP10-type peptides (Bone Morphogenetic Protein 10-type peptides) and, optionally, the amount of at least one further biomarker selected from the group consisting of a natriuretic peptide, ESM-1 (Endocan), Ang2 and FABP3 (Fatty Acid Binding Protein 3), and

c) providing information on the determined amount of the one or more BMP10-type peptides and optionally on the determined amount of the at least one further biomarker to a physician, thereby aiding in the assessment of atrial fibrillation.

Further, the present invention contemplates a method for aiding in the assessment of atrial fibrillation, comprising:

a) providing an assay for one or more BMP10-type peptides and, optionally, at least one further assay for a further biomarker selected from the group consisting of a natriuretic peptide, ESM-1 (Endocan), Ang2 and FABP3 (Fatty Acid Binding Protein 3), and

b) providing instructions for using of the assay results obtained or obtainable by said assay(s) in the assessment of atrial fibrillation.

Furthermore, the present invention relates to method for assessing the extent of white matter lesions in a subject, said method comprising

a) determining the amount of one or more BMP10-type peptides in a sample from the subject, and

b) assessing the extent of white matter lesions in a subject based on the amount determined in step a).

Moreover, the present invention relates to a method for the assessment whether a subject has experienced one or more silent strokes, said method comprising

a) determining the amount one or more BMP10-type peptides in a sample from the subject,

b) comparing the amount determined in step a) to a reference, and

c) assessing whether a subject has experienced one or more silent strokes.

Furthermore, the present invention relates to a method for predicting dementia, such as vascular dementia and/or Alzheimer's disease in a subject, said method comprising

a) determining the amount of one or more BMP10-type peptides in a sample from the subject,

b) comparing the amount determined in step a) to a reference, and

c) predicting the risk of dementia in said subject.

The present invention also contemplates a method for predicting the risk of AFib recurrence after therapeutic interventions (such as pulmonary vein isolation (PVI) or ablation therapy in atrial fibrillation) in a samples taken before the therapeutic intervention, comprising:

a) providing an assay for one or more BMP10-type peptides and, optionally, at least one further assay for a further biomarker selected from the group consisting of a natriuretic peptide, ESM-1 (Endocan), Ang2 and FABP3 (Fatty Acid Binding Protein 3), and

b) providing instructions for using of the assay results obtained or obtainable by said assay(s) in the assessment of risk of recurrence for atrial fibrillation.

Also encompassed by the present invention is computer-implemented method for assessing atrial fibrillation, comprising

a) receiving, at a processing unit, a value for the amount of one or more BMP10-type peptides, and, optionally at least one further value for the amount of at least one further biomarker selected from the group consisting of a natriuretic peptide, ESM-1 (Endocan), Ang2 and FABP3 (Fatty Acid Binding Protein 3), wherein said amount of the one or more BMP10-type peptides and, optionally, the amount of the at least one further biomarker have been determined in a sample from a subject,

b) comparing, by said processing unit, the value or values received in step (a) to a reference or to references, and
c) assessing atrial fibrillation based in the comparison step b).

The present invention further relates to a method for diagnosing heart failure, said method comprising the steps of

(a) determining, in at least one sample from the subject, the amount of one or more BMP10-type peptides (Bone Morphogenetic Protein 10-type peptides) and, optionally, the amount of at least one further biomarker selected from the group consisting of a natriuretic peptide, ESM-1 (Endocan), Ang2, and FABP3 (Fatty Acid Binding Protein 3), and
(b) comparing the amount of the one or more BMP10-type peptides to a reference amount for the BMP10-type peptide and, optionally, comparing the amount of the at least one further biomarker to a reference amount for said at least one further biomarker, whereby heart failure is to be diagnosed.

The present invention further relates to a method for predicting the risk of a subject of hospitalization due to heart failure, said method comprising the steps of

(a) determining, in at least one sample from the subject, the amount of one or more BMP10-type peptides (Bone Morphogenetic Protein 10-type peptides) and, optionally, the amount of at least one further biomarker selected from the group consisting of a natriuretic peptide, ESM-1 (Endocan), Ang2, and FABP3 (Fatty Acid Binding Protein 3),
(b) comparing the amount of the one or more BMP10-type peptides to a reference amount and, optionally, comparing the amount of the at least one further biomarker to a reference amount for said at least one further biomarker, and
(c) predicting the risk of a subject of hospitalization due to heart failure.

In a preferred embodiment of the methods of the present invention, the determination of the amount of the one or more BMP10-type peptides comprises contacting the sample with at least one agent that binds (i.e. is capable of binding to) the N-terminal prosegment of BMP10 (NT-proBMP10, sometimes also referred to as N-terminal prodomain of BMP10). Thus, it shall not bind mature BMP10. Accordingly, said agent binds (i.e. is capable of binding) within amino acid region 22 to 316 of the polypeptide shown in SEQ ID NO: 1. For example, the sample is contacted with at least one agent that binds within amino acid region 37 to 299 of the polypeptide shown in SEQ ID NO: 1.

The present invention further relates to a method for determining the amount of one or more BMP10-type peptides, comprising the steps of contacting a sample containing one or more BMP10-type peptides with at least one agent that binds the N-terminal prosegment of BMP10, and thus within amino acid region 22 to 316 of the polypeptide shown in SEQ ID NO: 1, thereby allowing the formation of a complex of the BMP10-type peptide and the at least one agent, and determining the amount of the complex formed.

In some embodiments, the method for determining the amount of one or more BMP10-type peptides is performed by a sandwich immunoassay.

The present invention further relates to a kit comprising at least one agent which specifically binds to one or more BMP10-type peptides, such as an agent which binds within amino acid region 22 to 316 of the polypeptide shown in SEQ ID NO: 1, and at least one further agent selected from the group consisting of an agent which specifically binds to a natriuretic peptide, an agent which specifically binds to ESM-1, an agent which specifically binds Ang2 and an agent which specifically binds to FABP3.

Moreover, the present invention relates to the *in vitro* use of

i) one or more BMP10-type peptides and optionally of at least one further biomarker selected from the group consisting of a natriuretic peptide, ESM-1 (Endocan), Ang2 and FABP3 (Fatty Acid Binding Protein 3), and/or
ii) at least one agent that specifically binds to one or more BMP10-type peptides, and, optionally, at least one further agent selected from the group consisting of an agent which specifically binds to a natriuretic peptide, an agent which specifically binds to ESM-1, an agent which specifically binds to Ang2 and an agent which specifically binds to FABP3, for assessing atrial fibrillation, for predicting the risk of stroke, or for diagnosing heart failure, or for predicting the risk of a subject of hospitalization due to heart failure.

In a preferred embodiment of the aforementioned uses, the at least one agent that specifically binds to one or more BMP10-type peptides is an agent which binds to NT-proBMP10, i.e. to the amino acid region 22 to 316 of the polypeptide shown in SEQ ID NO: 1. The amino acid sequence of NT-proBMP 1 0 is also shown in SEQ ID NO: 6.

The present invention further provides agents which bind (i.e. which are capable of binding) within amino acid region 22 to 316 of the polypeptide shown in SEQ ID NO: 1, such as an antibody, or antigen-binding fragment thereof. In some embodiments, the antibody is a monoclonal antibody.

In some embodiments, the agent is an which binds to an epitope contained in amino acid region 37 to 47 of the polypeptide shown in SEQ ID NO: 1 (SLFGDVFSEQD, SEQ ID NO 2).

In some embodiments, the agent is an which binds to an epitope contained in amino acid region 171 to 185 of SEQ ID NO: 1 (LESKGDNEGERNMLV, SEQ ID NO: 3), such as an agent which binds to an epitope contained in amino acid region 173 to 181 of SEQ ID NO: 1 (SKGDNEGER, SEQ ID NO: 4).

In some embodiments, the agent is an which binds to an epitope contained in amino acid region 291 to 299 of SEQ ID NO: 1 (SSGPGEEAL, SEQ ID NO: 5).

The present invention also relates to an antibody (such as monoclonal antibody) or fragment thereof which binds one or more BMP10-type peptides.

In an embodiment, the antibody or fragment thereof, comprises a heavy chain variable domain that is at least 80%, 85%, 90%, 95%, 98%, 99%, or 100% identical to the heavy chain variable domain comprising a sequence shown in SEQ ID NO: 7, 8, 9, 10, 11, 12, 13, 14 or 15 (see Table A) and/or a light chain variable domain that is, in increasing order of preference at least 80%, 85%, 90%, 95%, 98%, 99% or 100% identical to the light chain variable domain comprising a sequence as shown in SEQ ID NO: 16, 17, 18, 19, 20, 21, 22, 23 or 24 (see Table B).

Additionally or alternatively, the antibody, or fragment thereof, of the present invention comprises

(a) a light chain variable domain comprising

(a1) a light chain CDR1 shown in Table D (and thus a light chain CDR1 sequence selected from SEQ ID NOs:34-42),
(a2) a light chain CDR2 shown in Table D (and thus a light chain CDR2 sequence selected from SEQ ID NOs:52-60), and
(a3) a light chain CDR3 shown in Table D (and thus a light chain CDR3 sequence selected from SEQ ID NOs:70-78),
and

(b) a heavy chain variable domain comprising

(b1) a heavy chain CDR1 shown in Table C (and thus a heavy chain CDR1 sequence selected from SEQ ID NOs:25-33),
(b2) a heavy chain CDR2 shown in Table C (and thus a heavy chain CDR2 sequence selected from SEQ ID NOs:43-51), and
(b3) a heavy chain CDR3 shown in Table C (and thus a heavy chain CDR3 sequence selected from SEQ ID NOs:61-69).

DETAILED SUMMARY OF THE PRESENT INVENTION / DEFINITIONS

The present invention relates to a method for assessing atrial fibrillation in a subject, comprising the steps of

a) determining, in at least one sample from the subject, the amount of one or more BMP10-type peptides (Bone Morphogenetic Protein 10-type peptides), and
b) comparing the amount of the BMP 10-type peptide to a reference amount for the BMP10-type peptide, whereby atrial fibrillation is to be assessed.

The BMP10-type peptide is preferably selected from the group consisting of BMP10, N-terminal prosegment of BMP 10 (N-terminal proBMP 10), proBMP 10, and preproBMP 10. More preferably, the BMP10-type peptide is selected from the group consisting of N-terminal prosegment of BMP10 (N-terminal proBMP10), proBMP10, and preproBMP10. Even more preferably, the BMP10-type peptide is proBMP10 and/or N-terminal proBMP 10. Most preferably, the BMP 10-type peptide is NT-proBMP 10.

In accordance with the present invention, the amounts of one or more BMP10-type peptides shall be determined. As set forth elsewhere herein, the determination is preferably carried out with at least one agent which binds NT-proBMP10, or specific subregions therein. Thereby, the amount (i.e. the combined) of all BMP10-type peptides can be determined that comprise the NT-proBMP10 peptide sequence, such as proBMP10, preproBMP10 and NT-proBMP10. Thus, the expression "determining the amount of one or more BMP10-type peptides", preferably means that the combined amounts (i.e. the sum of the amounts) of polypeptides is determined, which have an amino acid sequence comprising NT-proBMP10 amino acid sequence, in particular that the combined amounts (i.e. the sum of the amounts) of proBMP10, preproBMP10 and NT-proBMP10 is determined. Since preproBMP10 is, in principle, not present in many samples, the expression may also mean that the sum of NT-proBMP10 and proBMP10 is determined. Thus, the amount of NT-proBMP10 and/or BMP10 is determined.

In an embodiment of method of the present invention, the method further comprises the determination of the amount of at least one further biomarker selected from the group consisting of a natriuretic peptide, ESM-1 (Endocan), Ang2 (Angiopoietin 2) and FABP-3 (Fatty acid binding protein 3) in a sample from the subject in step a) and the comparison of the amount of the at least one further biomarker to a reference amount in step b).

Accordingly, the present invention relates to a method for assessing atrial fibrillation in a subject, comprising the steps of

a) determining, in at least one sample from the subject, the amount of one or more BMP10-type peptides (Bone Morphogenetic Protein 10-type peptides) and, optionally, the amount of at least one further biomarker selected from the group consisting of a natriuretic peptide, ESM-1 (Endocan), Ang2 (Angiopoietin 2) and FABP-3 (Fatty acid binding protein 3), and

b) comparing the amount of the one or more BMP10-type peptides to a reference amount for the BMP10-type peptide and, optionally, comparing the amount of the at least one further biomarker to a reference amount for said at least one further biomarker, whereby atrial fibrillation is to be assessed.

The assessment of atrial fibrillation (AF) shall be based on the results of the comparison step b).

Accordingly, the present invention preferably comprises the steps of

a) determining, in at least one sample from the subject, the amount of one or more BMP10-type peptides and, optionally, the amount of at least one further biomarker selected from the group consisting of a natriuretic peptide, ESM-1 (Endocan), Ang2 (Angiopoietin 2) and FABP-3 (Fatty acid binding protein 3),

b) comparing the amount of the one or more BMP10-type peptides to a reference amount for the one or more BMP10-type peptides and, optionally, comparing the amount of the at least one further biomarker to a reference amount for said at least one further biomarker, and

c) assessing atrial fibrillation based on the results of the comparison step b).

The method as referred to in accordance with the present invention includes a method which essentially consists of the aforementioned steps or a method which includes further steps. Moreover, the method of the present invention, preferably, is an ex vivo and more preferably an in vitro method. Moreover, it may comprise steps in addition to those explicitly mentioned above. For example, further steps may relate to the determination of further markers and/or to sample pre-treatments or evaluation of the results obtained by the method. The method may be carried out manually or assisted by automation. Preferably, step (a), (b) and/or (c) may in total or in part be assisted by automation, e.g., by a suitable robotic and sensory equipment for the determination in step (a) or a computer-implemented calculation in step (b).

In accordance with the present invention, atrial fibrillation shall be assessed. The term "assessing atrial fibrillation" as used herein preferably refers to the diagnosis of atrial fibrillation, the diagnosis of the recent episode of AF, the differentiation between paroxysmal and persistent atrial fibrillation, the prediction of a risk of an adverse event associated with atrial fibrillation (such as stroke and/or the recurrence of atrial fibrillation, such as the recurrence of AF after intervention), to the identification of a subject who shall be subjected to electrocardiography (ECG), or to the assessment of a therapy for atrial fibrillation.

As will be understood by those skilled in the art, the assessment of the present invention is usually not intended to be correct for 100% of the subjects to be tested. The term, preferably, requires that a correct assessment (such as the diagnosis, differentiation, prediction, identification or assessment of a therapy as referred to herein) can be made for a statistically significant portion of subjects. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.4, 0.1, 0.05, 0.01, 0.005, or 0.0001.

In accordance with the present invention, the expression "assessment of atrial fibrillation" is understood as an aid in the assessment of atrial fibrillation, and thus as an aid in diagnosing atrial fibrillation, an aid in differentiating between paroxysmal and persistent atrial fibrillation, an aid in the prediction of a risk of an adverse event associated with atrial fibrillation, an aid in the identification of a subject who shall be subjected to electrocardiography (ECG), or as an aid in the assessment of a therapy for atrial fibrillation. The final diagnosis, in principle, will be carried out by physician.

In a preferred embodiment of the present invention, the assessment of atrial fibrillation is the diagnosis of atrial fibrillation. Accordingly, it is diagnosed, whether a subject suffers from atrial fibrillation, or not.

Accordingly, the present invention envisages a method for diagnosing atrial fibrillation in a subject, comprising the steps of

a) determining the amount of one or more BMP10-type peptides in a sample from the subject, and

b) comparing the amount of the one or more BMP10-type peptides to a reference amount, whereby atrial fibrillation is to be diagnosed.

In an embodiment, the aforementioned method comprises the steps of:

(a) determining, in at least one sample from the subject, the amount of one or more BMP10-type peptides (Bone Morphogenetic Protein 10 peptides) and, optionally, the amount of at least one further biomarker selected from the group consisting of a natriuretic peptide, ESM-1 (Endocan), Ang2 (Angiopoietin 2) and FABP-3 (Fatty acid binding protein 3), and
(b) comparing the amount of the one or more BMP10-type peptides to a reference amount for the one or more BMP10-type peptides and, optionally, comparing the amount of the at least one further biomarker to a reference amount for said at least one further biomarker, whereby atrial fibrillation is to be diagnosed.

Preferably, the subject to be tested in connection with method for diagnosing of atrial fibrillation is a subject who is suspected to suffer from atrial fibrillation. However, it is also contemplated that the subject already has been diagnosed previously to suffer from AF and that the previous diagnosis is confirmed by carrying out the method of the present invention.
In another preferred embodiment of the present invention, the assessment of atrial fibrillation is the differentiation between paroxysmal and persistent atrial fibrillation. Accordingly, it is determined whether a subject suffers from the paroxysmal or persistent atrial fibrillation.
Accordingly, the present invention envisages a method for differentiating between paroxysmal and persistent atrial fibrillation in a subject, comprising the steps of

a) determining the amount of one or more BMP10-type peptides in a sample from the subject, and
b) comparing the amount of the one or more BMP10-type peptides to a reference amount, whereby it is differentiated between paroxysmal and persistent atrial fibrillation.

In an embodiment, the aforementioned method comprises the steps of:

a) determining, in at least one sample from the subject, the amount of one or more BMP10-type peptides (Bone Morphogenetic Protein 10 peptides) and, optionally, the amount of at least one further biomarker selected from the group consisting of a natriuretic peptide, ESM-1 (Endocan), Ang2 (Angiopoietin 2) and FABP-3 (Fatty acid binding protein 3), and
b) comparing the amount of the one or more BMP10-type peptides to a reference amount for the one or more BMP10-type peptides and, optionally, comparing the amount of the at least one further biomarker to a reference amount for said at least one further biomarker, whereby it is differentiated between paroxysmal and persistent atrial fibrillation.

In another preferred embodiment of the present invention, the assessment of atrial fibrillation is the prediction of the risk of an adverse event associated with atrial fibrillation (such as stroke). Accordingly, it is predicted whether a subject is at risk and/or not as risk of said adverse event.
Thus, the present invention envisages a method for predicting the risk of an adverse event associated with atrial fibrillation in a subject, comprising the steps of

a) determining the amount of one or more BMP10-type peptides in a sample from the subject, and
b) comparing the amount of the one or more BMP10-type peptides to a reference amount, whereby the risk of the adverse event associated with atrial fibrillation is to be predicted.

In an embodiment, the aforementioned method comprises the steps of:

a) determining, in at least one sample from the subject, the amount of one or more BMP10-type peptides (Bone Morphogenetic Protein 10 peptides) and, optionally, the amount of at least one further biomarker selected from the group consisting of a natriuretic peptide, ESM-1 (Endocan), Ang2 (Angiopoietin 2) and FABP-3 (Fatty acid binding protein 3), and
b) comparing the amount of the one or more BMP10-type peptides to a reference amount for the one or more BMP10-type peptides and, optionally, comparing the amount of the at least one further biomarker to a reference amount for said at least one further biomarker, whereby the risk of the adverse event associated with atrial fibrillation is to be predicted.

It is envisaged that various adverse events can be predicted. A preferred adverse event to be predicted is stroke.

Accordingly, the present invention, in particular, contemplates a method for predicting the risk of stroke in a subject, comprising the steps of

a) determining the amount of one or more BMP10-type peptides in a sample from the subject, and
b) comparing the amount of the one or more BMP10-type peptides to a reference amount, whereby the risk of stroke is to be predicted.

The aforementioned method may further comprise step c) of predicting stroke based on the comparison results of step b). Thus, steps a), b), c) are preferably as follows:

a) determining the amount of one or more BMP10-type peptides in a sample from the subject, and
b) comparing the amount of the one or more BMP10-type peptides to a reference amount, and
c) predicting stroke based on the comparison results of step b)

In another preferred embodiment of the present invention, the assessment of atrial fibrillation is the assessment of a therapy for atrial fibrillation.
Accordingly, the present invention envisages a method for the assessment of a therapy for atrial fibrillation in a subject, comprising the steps of

a) determining the amount of one or more BMP10-type peptides in a sample from the subject, and
b) comparing the amount of the one or more BMP10-type peptides to a reference amount, whereby the therapy for atrial fibrillation is to be assessed.

In an embodiment, the aforementioned method comprises the steps of:

a) determining, in at least one sample from the subject, the amount of one or more BMP10-type peptides (Bone Morphogenetic Protein 10) and, optionally, the amount of at least one further biomarker selected from the group consisting of a natriuretic peptide, ESM-1 (Endocan), Ang2 (Angiopoietin 2) and FABP-3 (Fatty acid binding protein 3), and
b) comparing the amount of the one or more BMP10-type peptides to a reference amount for the one or more BMP10-type peptides and, optionally, comparing the amount of the at least one further biomarker to a reference amount for said at least one further biomarker, whereby the therapy for atrial fibrillation is to be assessed.

Preferably, the subject in connection with the aforementioned differentiation, the aforementioned prediction, and the assessment of a therapy for atrial fibrillation is a subject who suffers from atrial fibrillation, in particular who is known to suffer from atrial fibrillation (and thus to have a known history of atrial fibrillation). However, with respect to the aforementioned prediction method, it is also envisaged that the subject has no known history of atrial fibrillation.
In another preferred embodiment of the present invention, the assessment of atrial fibrillation is the identification of a subject who shall be subjected to electrocardiography (ECG). Accordingly, a subject is identified who shall be subjected to electrocardiography, or not.
The method may comprise the steps of

a) determining, in at least one sample from the subject, the amount of one or more BMP10-type peptides (Bone Morphogenetic Protein 10) and, optionally, the amount of at least one further biomarker selected from the group consisting of a natriuretic peptide, ESM-1 (Endocan), Ang2 (Angiopoietin 2) and FABP-3 (Fatty acid binding protein 3), and
b) comparing the amount of the one more more BMP10-type peptides to a reference amount for the one or more BMP10-type peptides and, optionally, comparing the amount of the at least one further biomarker to a reference amount for said at least one further biomarker, whereby a subject is identified who shall be subjected to electrocardiography.

Preferably, the subject in connection with the aforementioned method of identifying a subject who shall be subjected to electrocardiography is a subject who has no known history of atrial fibrillation. The expression "no known history of atrial fibrillation" is defined elsewhere herein.
In another preferred embodiment of the present invention, the assessment of atrial fibrillation is the assessment of efficacy of an anticoagulation therapy of a subject. Accordingly, the efficacy of said therapy is assessed.
In another preferred embodiment of the present invention, the assessment of atrial fibrillation is the prediction of the risk of stroke in a subject. Accordingly, it is predicted whether a subject as referred to herein is at risk of stroke, or not.

In another preferred embodiment of the present invention, the assessment of atrial fibrillation is the identification a subject being eligible to the administration of at least one anticoagulation medicament or being eligible for increasing the dosage of at least one anticoagulation medicament. Accordingly, it is assessed whether a subject is eligible to said administration and/or said increase of the dosage.

In another preferred embodiment of the present invention, the assessment of atrial fibrillation is the monitoring of anticoagulation therapy. Accordingly, it is assessed whether a subject responds to said therapy, or not.

The term "atrial fibrillation" ("abbreviated" AF or AFib) is well known in the art. As used herein, the term preferably refers to a supraventricular tachyarrhythmia characterized by uncoordinated atrial activation with consequent deterioration of atrial mechanical function. In particular, the term refers to an abnormal heart rhythm characterized by rapid and irregular beating. It involves the two upper chambers of the heart. In a normal heart rhythm, the impulse generated by the sino-atrial node spreads through the heart and causes contraction of the heart muscle and pumping of blood. In atrial fibrillation, the regular electrical impulses of the sino-atrial node are replaced by disorganized, rapid electrical impulses which result in irregular heart beats. Symptoms of atrial fibrillation are heart palpitations, fainting, shortness of breath, or chest pain. However, most episodes have no symptoms. On the electrocardiogram, Atrial Fibrillation is characterized by the replacement of consistent P waves by rapid oscillations or fibrillatory waves that vary in amplitude, shape, and timing, associated with an irregular, frequently rapid ventricular response when atrioventricular conduction is intact.

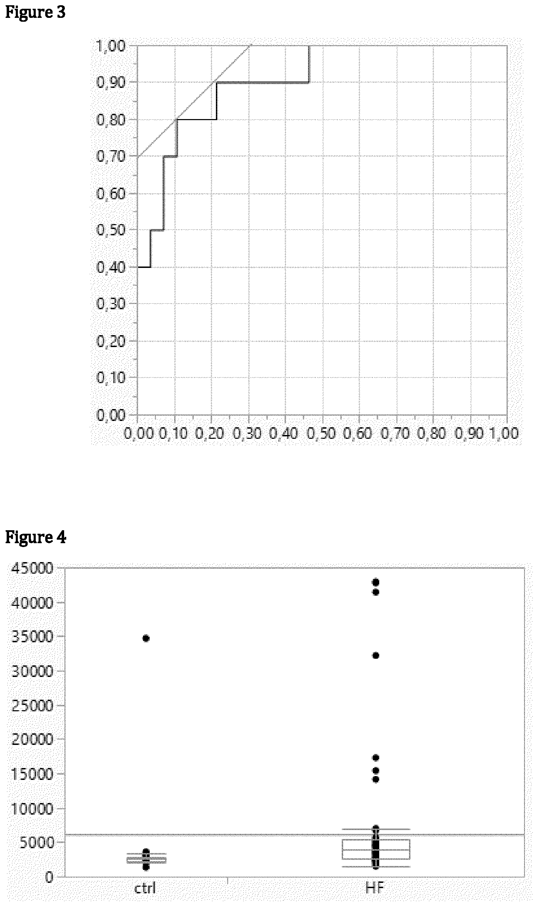

The American College of Cardiology (ACC), American Heart Association (AHA), and the European Society of Cardiology (ESC) propose the following classification system (see Fuster V. et al., Circulation 2006;114 (7): e257-354 which herewith is incorporated by reference in its entirety, see e.g. Figure 3 in the document): First detected AF, paroxysmal AF, persistent AF, and permanent AF.

All people with AF are initially in the category called first detected AF. However, the subject may or may not have had previous undetected episodes. A subject suffers from permanent AF, if the AF has persisted for more than one year, and in particular, conversion back to sinus rhythm does not occur (or only with medical intervention). A subject suffers from persistent AF, if the AF lasts more than 7 days. The subject may require either pharmacologic or electrical intervention to terminate Atrial Fibrillation. Preferably, persistent AF occurs in episodes, but the arrhythmia does not convert back to sinus rhythm spontaneously (i.e. without medical intervention). Paroxysmal Atrial Fibrillation, preferably, refers to an intermittent episode of Atrial Fibrillation which lasts up to 7 days. In most cases of paroxysmal AF, the episodes last less than 24 hours. The episode of Atrial Fibrillation terminates spontaneously, i.e. without medical intervention. Thus, whereas the episode(s) of paroxysmal atrial fibrillation preferably terminate spontaneously, persistent atrial fibrillation preferably does not end spontaneously. Preferably, persistent atrial fibrillation requires electrical or pharmacological cardioversion for termination, or other procedures, such as ablation procedures (Fuster V. et al., Circulation 2006;114 (7): e257-354). Both persistent and paroxysmal AF may be recurrent, whereby distinction of paroxysmal and persistent AF is provided by ECG recordings: When a patient has had 2 or more episodes, AF is considered recurrent. If the arrhythmia terminates spontaneously, AF, in particular recurrent AF, is designated paroxysmal. AF is designated persistent if it lasts more than 7 days.

In a preferred embodiment of the present invention, the term "paroxysmal atrial fibrillation" is defined as episodes of AF that terminate spontaneously, wherein said episodes last less than 24 hours. In an alternative embodiment, the episodes which terminate spontaneously last up to seven days.

The "subject" as referred to herein is, preferably, a mammal. Mammals include, but are not limited to, domesticated animals (e.g., cows, sheep, cats, dogs, and horses), primates (e.g., humans and non-human primates such as monkeys), rabbits, and rodents (e.g., mice and rats). Preferably, the subject is a human subject.

Preferably, the subject to be tested is of any age, more preferably, the subject to be tested is 50 years of age or older, more preferably 60 years of age or older, and most preferably 65 years of age or older. Further, it is envisaged that the subject to be tested is 70 years of age or older.

Moreover, it is envisaged that the subject to be tested is 75 years of age or older. Also, the subject may be between 50 and 90 years.

In a preferred embodiment of the method of assessing atrial fibrillation, the subject to be tested shall suffer from atrial fibrillation. Accordingly, the subject shall have a known history of atrial fibrillation. Thus, the subject shall have experienced episodes of Atrial Fibrillation prior to obtaining the test sample, and at least one of the previous episodes of atrial fibrillation shall have been diagnosed, e.g. by ECG. For example, it is envisaged that the subject suffers from atrial fibrillation, if the assessment of atrial fibrillation is the differentiation between paroxysmal and persistent atrial fibrillation, or if the assessment of atrial fibrillation is the prediction of a risk of an adverse event associated with atrial fibrillation, or if the assessment of atrial fibrillation is the assessment of a therapy for atrial fibrillation.

In another preferred embodiment of the method of assessing atrial fibrillation, the subject to be tested is suspected to suffer from atrial fibrillation, e.g. if the assessment of atrial fibrillation is the diagnosis of atrial fibrillation or the identification of a subject who shall be subjected to electrocardiography (ECG).

Preferably, a subject who is suspected to suffer from atrial fibrillation is a subject who has shown at least one symptom of atrial fibrillation prior to carrying out the method for assessing atrial fibrillation. Said symptoms are usually transient and may arise in a few seconds and may disappear just as quickly. Symptoms of atrial fibrillation include dizziness, fainting, shortness of breath and, in particular, heart palpitations. Preferably, the subject has shown at least one symptom of atrial fibrillation within six months prior to obtaining the sample.

Alternatively or additionally, a subject who is suspected to suffer from atrial fibrillation shall be a subject who is 70 years of age or older.

Preferably, the subject who is suspected to suffer from atrial fibrillation shall have no known history of atrial fibrillation.

In accordance with the present invention, a subject having no known history of atrial fibrillation is, preferably, a subject who has not been diagnosed to suffer from atrial fibrillation previously, i.e. before carrying out the method of the present invention (in particular before obtaining the sample from the subject). However, the subject may or may not have had previous undiagnosed episodes of atrial fibrillation.

Preferably, the term "atrial fibrillation" refers to all types of atrial fibrillation. Accordingly, the term preferably encompasses paroxysmal, persistent or permanent atrial fibrillation.

In an embodiment of the present invention, however, the subject to be tested does not suffer from permanent atrial fibrillation. In this embodiment, the term "atrial fibrillation" only refers to paroxysmal and persistent atrial fibrillation.

In another embodiment of the present invention, however, the subject to be tested does not suffer from paroxysmal and permanent atrial fibrillation. In this embodiment, the term "atrial fibrillation" only refers to persistent atrial fibrillation.

The subject to be tested may or may not experience episodes of atrial fibrillation when the sample is obtained. Thus, in a preferred embodiment of the assessment of atrial fibrillation (such as in the diagnosis of atrial fibrillation), the subject does not experience episodes of Atrial Fibrillation when the sample is obtained. In this embodiment, the subject shall have a normal sinus rhythm when the sample is obtained (and shall be accordingly in sinus rhythm). Thus, the diagnosis of atrial fibrillation with the biomarker is possible even in the (temporary) absence of atrial fibrillation in the ECG. In accordance with the method of the present invention, the elevation of the biomarkers as referred to herein should be preserved after the episode of Atrial Fibrillation and, thus, provide a diagnosis of a subject who has suffered from Atrial Fibrillation ("Memory Effect"). Preferably, the diagnosis of AF within about three days, within about one week, within about one month, within about three months, or within about 6 months after carrying out the method of the present invention (or to be more precise after the sample has been obtained). In a preferred embodiment, the diagnosis of Atrial Fibrillation within about six months after the episode is feasible. In a preferred embodiment, the diagnosis of Atrial Fibrillation within about six months after the episode is feasible. Accordingly, the assessment of atrial fibrillation as referred to herein, in particular the diagnosis, the prediction of the risk or the differentiation as referred to herein in connection with the assessment of atrial fibrillation is preferably carried out after about three days, more preferably after about one month, even more preferably after about three month, and most preferably after about six months after the last episode of atrial fibrillation. Consequently, is envisaged that is sample to be tested is preferably obtained after about three days, more preferably after about one month, even more preferably after about three month, and most preferably after about six months after the last episode of atrial fibrillation. Accordingly, the diagnosis of atrial fibrillation preferably also encompasses the diagnosis of episodes of atrial fibrillation that occurred preferably within about three days, more preferably within about one week, even more preferably within about three months, and most preferably within about six months before the sample was obtained. Thus, the present invention allows the diagnosis of a recent episode of AF, such as an episode which occurred within about three days or, more preferably, within about one week prior to carrying out the method of the present invention (or to be more precise before the sample to be tested has been obtained). Further, a recent episode of AF may have occurred about two weeks prior to carrying out the method of the present invention.

Accordingly the present invention allows for aiding in the diagnosis of a recent episode of atrial fibrillation in a subject who is in sinus rhythm, comprising the determination of the amount of one or more BMP10-peptide (and optionally of at least one further biomarker as described elsewhere herein), and comparing the thus determined amount (or amounts) to a reference amount (or reference amounts), thereby aiding in the diagnosis of recent episode of AF. In an embodiment, said subject shall be suspected to have suffered from an episode of AF recently, for example the subject may be a subject who has shown symptoms of AF recently (such as within three days, one week or two weeks prior to carrying out the method of the present invention). Symptoms of atrial fibrillation are heart palpitations, fainting, shortness of breath, or chest pain.

However, it is also envisaged that the subject experiences episodes of atrial fibrillation when the sample is obtained (e.g. with respect to the prediction of stroke).

The term "sample" refers to a sample of a body fluid, to a sample of separated cells or to a sample from a tissue or an organ. Samples of body fluids can be obtained by well-known techniques and include, samples of blood, plasma, serum, urine, lymphatic fluid, sputum, ascites, or any other bodily secretion or derivative thereof. Tissue or organ samples may be obtained from any tissue or organ by, e.g., biopsy. Separated cells may be obtained from the body fluids or the tissues or organs by separating techniques such as centrifugation or cell sorting. E.g., cell-, tissue- or organ samples may be obtained from those cells, tissues or organs which express or produce the biomarker. The sample may be frozen, fresh, fixed (e.g. formalin fixed), centrifuged, and/or embedded (e.g. paraffin embedded), etc. The cell sample can, of course, be subjected to a variety of well-known post-collection preparative and storage techniques (e.g., nucleic acid and/or protein extraction, fixation, storage, freezing, ultrafiltration, concentration, evaporation, centrifugation, etc.) prior to assessing the amount of the biomarker(s) in the sample.

In a preferred embodiment of the present invention, the sample is a blood (i.e. whole blood), serum or plasma sample. Serum is the liquid fraction of whole blood that is obtained after the blood is allowed to clot. For obtaining the serum, the clot is removed by centrifugation and the supernatant is collected. Plasma is the acellular fluid portion of blood. For obtaining a plasma sample, whole blood is collected in anticoagulant-treated tubes (e.g. citrate-treated or EDTA-treated tubes). Cells are removed from the sample by centrifugation and the supernatant (i.e. the plasma sample) is obtained.

As set forth above, the subject may be in sinus rhythm or may suffer from an episode of AF rhythm at the time at which the sample is obtained.

BMP10-type peptides are well known in the art. Preferred BMP10-types peptide are e.g. disclosed in Susan-Resiga et al. (J Biol Chem. 2011 Jul 1;286(26):22785-94) which herewith is incorporated by reference in its entirety (see e.g. Figure 3A of Susan-Resiga et al., or US 2012/0213782).

In an embodiment, the BMP10-type peptide is unprocessed preproBMP10 (see SEQ ID NO: 1 below). In another embodiment, the BMP10-type peptide is the propeptide proBMP10. This marker comprises the N-terminal prosegement and BMP10. In another embodiment, the BMP10-type peptide is the N-terminal prosegment of BMP10 (N-terminal proBMP 10, or NT-proBMP 10).

In an embodiment, the BMP10-type peptide is part of a homo- or heterodimeric complex.

Human preproBMP10 (i.e. unprocessed preproBMP10) has a length of 424 amino acids. The amino acid sequence of human preproBMP10 is e.g. shown in SEQ ID NO: 1 or in Fig. 3 of US 2012/0213782 which herewith is incorporated by reference in its entirety. Further, the amino acid sequence of preproBMP10 can be assessed via Uniprot (see sequence under accession number O95393-1).

Moreover, the amino acid sequence of human preproBMP10 is shown herein below (SEQ ID NO: 1):

*MGSLVLTLCALFCLAAYLVSG*SPIMNLEQSPLEEDMSLFGDVFSEQDGVDFNTLLQSMK

DEFLKTLNLSDIPTQDSAKVDPPEYMLELYNKFATDRTSMPSANIIRSFKNEDLFSQPV

SFNGLRKYPLLFNVSIPHHEEVIMAELRLYTLVQRDRMIYDGVDRKITIFEVLESKGDN

EGERNMLVLVSGEIYGTNSEWETFDVTDAIRRWQKSGSSTHQLEVHIESKHDEAEDASS

GRLEIDTSAQNKHNPLLIVFSDDQSSDKERKEELNEMISHEQLPELDNLGLDSFSSGPGE

EALLQMRSNIIYDSTARIRR**NAKGNYCKRTPLYIDFKEIGWDSWIIAPPGYEAYECRGVC**

**NYPLAEHLTPTKHAIIQALVHLKNSQKASKACCVPTKLEPISILYLDKGVVTYKFKYEGM**

**AVSECGCR**

Human preproBMP10 comprises a short signal peptide (amino acids 1 to 21) which is enzymatically cleaved off to release proBMP10 (in the above sequence, the signal peptide is indicated in *italics)*. Accordingly, human proBMP10 comprises amino acids 22 to 424 of human preproBMP10 (i.e. of the polypeptide having a sequence shown in SEQ ID NO 1). Human proBMP10 is further cleaved into an N-terminal prosegment of BMP10 and (non-glycosylated) BMP10 which is the active form. The N-terminal prosegment of BMP10 comprises amino acids 22 to 316 of the polypeptide having a sequence shown in SEQ ID NO 1 (i.e of human preproBMP10). The sequence of NT-proBMP10 is underlined in the above sequence. Accordingly, NT-proBMP10 has a sequence as shown as follows (SEQ ID: 6):

SPIMNLEQSPLEEDMSLFGDVFSEQDGVDFNTLLQSMK

DEFLKTLNLSDIPTQDSAKVDPPEYMLELYNKFATDRTSMPSANIIRSFKNEDLFSQPV

SFNGLRKYPLLFNVSIPHHEEVIMAELRLYTLVQRDRMIYDGVDRKITIFEVLESKGDN

EGERNMLVLVSGEIYGTNSEWETFDVTDAIRRWQKSGSSTHQLEVHIESKHDEAEDASS

GRLEIDTSAQNKHNPLLIVFSDDQSSDKERKEELNEMISHEQLPELDNLGLDSFSSGPGE

EALLQMRSNIIYDSTARIRR

BMP10 comprises amino acids 317 to 424 of the polypeptide having a sequence shown in SEQ ID NO 1 (indicated in **bold** in above sequence).

The preferred BMP10-type peptides are proBMP10 and N-terminal proBMP10. After cleavage of proBMP10, BMP10 and N-terminal proBMP10 remain in structural proximity forming homo- or hetero-dimers of BMP10 or in combination with other BMP-family proteins (Yadin et al., CYTOGFR 2016, 27 (2016) 13-34). The dimerization occurs by formation of Cys-Cys bridge or strong adhesion in the C-terminal peptides of both binding partners. Thus, an architecture consisting of two subunits is formed.

Preferably, the amount of the BMP10-type peptide is determined by using at least one agent which specifically binds to the BMP10-type peptide, such as one or more antibodies (or antigen-binding fragments thereof) which specifically bind to the BMP10-type peptide.

As set forth above, it was found in the studies underlying the present invention that the detection of BMP10-type peptides is better, when using antibodies against amino acid region 22 to 316 of human preproBMP10, as compared antibodies against the mature BMP10 hormone itself.

Therefore, it is in particular contemplated to determine the amount of the one or more BMP10-type peptides by using at least one agent which binds the N-terminal prosegment of BMP10, and thus binds amino acid region 22 to 316 of SEQ ID NO: 1, i.e. which bind within amino acid region from amino acid 22 to amino acid 316 of the polypeptide having an amino acid sequence as shown in SEQ ID NO: 1. Thus, the agent shall bind to an epitope contained in this region, such as an epitope described elsewhere herein or shown in the table of Example 12.

SEQ ID NO: 1 is the sequence of human preproBMP10. The sequence is used as reference sequence herein. It is stated herein, that the agents shall bind certain regions within the polypeptide having a sequence as shown in SEQ ID NO: 1, for example to amino acid region 37 to 299, or region 171 to 185 of SEQ ID NO: 5. This means that the agent shall bind to a region in the BMP10-type which corresponds to these regions. For example, proBMP10 and NT-proBMP10 lack the first 21 amino acids of the human preproBMP10. Thus, the agent binds to amino acid region 16 to 278 and region 150 to 164 of NT-proBMP10 or proBMP10

Subregions within amino acid region 22 to 316 of SEQ ID NO: 1 were identified which are particularly suitable for the detection of BMP 10-types peptides.

In a preferred embodiment, the at least one agent (such as the antibody or fragment thereof) which binds the BMP10-type peptide binds within amino acid region 37 to 299 of SEQ ID NO: 1, i.e. said at least agent binds to an epitope contained in the region which starts at the amino acid at position 37 and ends with the amino acid at position 299 of SEQ ID NO: 1.

In another preferred embodiment, at least one agent binds within amino acid region 110 to 200 of SEQ ID NO: 1, i.e. said at least agent binds to an epitope contained in this region.

In another preferred embodiment, said at least one agent binds within amino acid region 37 to 195, such as within amino acid region 37 to 185 of SEQ ID NO: 1, i.e. said at least agent binds to an epitope contained in this region.

In another preferred embodiment, said at least one agent binds within amino acid region 160 to 299, such as within amino acid region 171 to 299 of SEQ ID NO: 1, i.e. said at least agent binds to an epitope contained in this region.

In another preferred embodiment, said at least one agent binds within amino acid region 160 to 195, such as within amino acid region 171 to 185 of SEQ ID NO: 1.

In a further preferred embodiment, said at least one agent binds to an epitope contained in amino acid region 37 to 47 of SEQ ID NO: 1 (SLFGDVFSEQD, SEQ ID NO 2). In an embodiment, the epitope of said agent essentially consists of SLFGDVFSEQD (SEQ ID NO:2).

In a further preferred embodiment, said at least one agent binds to an epitope contained in amino acid region 171 to 185 of SEQ ID NO: 1 (LESKGDNEGERNMLV, SEQ ID NO: 3). For example, said at least one agent binds to an epitope contained in amino acid region 173 to 181 of SEQ ID NO: 1 (SKGDNEGER, SEQ ID NO: 4). In an embodiment, the epitope of said agent essentially consists of SKGDNEGER (SEQ ID NO: 4).

In a further preferred embodiment, said at least one agent binds to an epitope contained in amino acid region 291 to 299 of SEQ ID NO: 1 (SSGPGEEAL, SEQ ID NO: 5). In an embodiment, the epitope of said agent essentially consists of SSGPGEEAL (SEQ ID NO: 5).

In a preferred embodiment, one or more antibodies, or antigen-binding fragments thereof, which specifically bind to the N-terminal prosegment of BMP10 could be used. Since such antibodies (or fragments) would also bind to proBMP10 and preproBMP10, the sum of the amounts of the N-terminal prosegment of BMP10, proBMP10 and preproBMP10 can be determined in step a) of the methods of the present invention. Accordingly, the expression "determining the amount of the N-terminal prosegment of BMP10" also shall mean "determining the sum of the amounts of the N-terminal prosegment of BMP10, proBMP10 and preproBMP10". As preproBMP10 is essentially not present in many samples, the expression may also mean "determining the sum of the amounts of the N-terminal prosegment of BMP 10 and preproBMP10".

For example, one or more antibodies which specifically bind to BMP10 could be used. Since such antibodies (or fragments) would also bind to proBMP10 and preproBMP10, the sum of the amounts of the BMP10, proBMP10 and preproBMP10 is determined in step a) of the methods of the present invention. Accordingly, the expression "determining the amount of BMP10" also shall mean "determining the sum of the amounts of BMP10, proBMP10 and preproBMP10".

Further, it is envisaged to determine the sum of the amounts of all four BMP10-type peptides as referred to above, i.e. of BMP10, the N-terminal prosegment of BMP10, proBMP 10 and preproBMP 10.

Accordingly, the following amounts of BMP10-type peptides can be determined in accordance with the present invention:

- the amount of BMP 10
- the amount of the N-terminal prosegment of BMP10
- the amount of proBMP 10
- the amount of preproBMP 10
- the sum of the amounts of BMP10, proBMP 10 and preproBMP 10
- the sum of the amounts of the N-terminal prosegment of BMP 10, proBMP 10 and preproBMP 10, or
- the sum of the amounts of BMP10, the N-terminal prosegment of BMP10, proBMP 10 and preproBMP 10

In particular, the following amounts of BMP10-type peptides can be determined in accordance with the present invention:

- the amount of the N-terminal prosegment of BMP10
- the amount of proBMP 10
- the amount of preproBMP 10
- the sum of the amounts of the N-terminal prosegment of BMP 10, proBMP 10 and preproBMP 10, or
- the sum of the amounts of the N-terminal prosegment of BMP10 and proBMP10 and preproBMP 10.

The term "natriuretic peptide" comprises atrial natriuretic peptide (ANP)-type and brain natriuretic peptide (BNP)-type peptides. Thus, natriuretic peptides according to the present invention comprise ANP-type and BNP-type peptides and variants thereof (see, e.g., Bonow RO. et al., Circulation 1996;93: 1946-1950).

ANP-type peptides comprise pre-proANP, proANP, NT-proANP, and ANP.

BNP-type peptides comprise pre-proBNP, proBNP, NT-proBNP, and BNP.

The pre-pro peptide (134 amino acids in the case of pre-proBNP) comprises a short signal peptide, which is enzymatically cleaved off to release the pro peptide (108 amino acids in the case of proBNP). The pro peptide is further cleaved into an N-terminal pro peptide (NT-pro peptide, 76 amino acids in case of NT-proBNP) and the active hormone (32 amino acids in the case of BNP, 28 amino acids in the case of ANP).

Preferred natriuretic peptides according to the present invention are NT-proANP, ANP, NT-proBNP, BNP. ANP and BNP are the active hormones and have a shorter half-life than their respective inactive counterparts, NT-proANP and NT-proBNP. BNP is metabolized in the blood, whereas NT-proBNP circulates in the blood as an intact molecule and as such is eliminated renally.

The most preferred natriuretic peptides according to the present invention are NT-proBNP and BNP, in particular NT-proBNP. As briefly discussed above, the human NT-proBNP as referred to in accordance with the present invention is a polypeptide comprising, preferably, 76 amino acids in length corresponding to the N-terminal portion of the human NT-proBNP molecule. The structure of the human BNP and NT-proBNP has been described already in detail in the prior art, e.g., WO 02/089657, WO 02/083913, and Bonow RO. Et al., New Insights into the cardiac natriuretic peptides. Circulation 1996;93: 1946-1950. Preferably, human NT-proBNP as used herein is human NT-proBNP as disclosed in EP 0 648 228 B1.

The term "FABP-3" as used herein refers to the fatty acid binding protein 3. FABP-3 is also known as heart fatty acid binding protein or heart type fatty acid binding protein (abbreviated H-FABP). Preferably, the term also includes variants of FABP-3. FABP-3 as used herein, preferably, relates to human FABP-3. The DNA sequence of the polypeptide encoding the human FABP-3 polypeptide as well the protein sequence of human FABP-3 is well known in the art and was first described by Peeters et al. (Biochem. J. 276 (Pt 1), 203-207 (1991)). Moreover, the sequence of human H-FABP can be found, preferably, in Genbank entry U57623.1 (cDNA sequence) and AAB02555.1 (protein sequence). The major physiological function of FABP is thought to be the transport of free fatty acids, see e.g. Storch et al., Biochem. Biophys. Acta. 1486 (2000), 28-44. Other names for FABP-3 and H-FABP are: FABP-11 (fatty acid binding protein 11), M-FABP (muscle fatty acid-binding protein), MDGI (mammary-derived growth inhibitor), and O-FABP.

The biomarker endothelial cell specific molecule 1 (abbreviated ESM-1) is well known in the art. The biomarker is frequently also referred to as endocan. ESM-1 is a secreted protein which is mainly expressed in the endothelial cells in human lung and kidney tissues. Public domain data suggest expression also in thyroid, lung and kidney, but also in heart tissue, see. e.g. the entry for ESM-1 in the Protein Atlas database (Uhlén M. et al., Science 2015;347(6220): 1260419). The expression of this gene is regulated by cytokines. ESM-1 is a proteoglycan composed of a 20 kDa mature polypeptide and a 30 kDa O-linked glycan chain (Bechard D et al., J Biol Chem 2001;276(51):48341-48349). In a preferred embodiment of the present invention, the amount of the human ESM-1 polypeptide is determined in a sample from the subject. The sequence of the human ESM-1 polypeptide is well known in the art (see e.g. Lassale P. et al., J. Biol. Chem. 1996;271:20458-20464 and can be e.g. assessed via Uniprot database, see entry Q9NQ30 (ESM1_HUMAN). Two isoforms of ESM-1 are produced by alternative splicing, isoform 1 (having the Uniprot identifier Q9NQ30-1) and isoform 2 (having the Uniprot identifier Q9NQ30-2). Isoform 1 has length of 184 amino acids. In isoform 2, amino acids 101 to 150 of isoform 1 are missing. Amino acids 1 to 19 form the signal peptide (which might be cleaved off).

In a preferred embodiment, the amount of isoform 1 of the ESM-1 polypeptide is determined, i.e. isoform 1 having a sequence as shown under UniProt accession number Q9NQ30-1.

In another preferred embodiment, the amount of isoform 2 of the ESM-1 polypeptide is determined, i.e. isoform 2 having a sequence as shown under UniProt accession number Q9NQ30-2.

In another preferred embodiment, the amount of isoform-1 and isoform 2 of the ESM-1 polypeptide is determined, i.e. total ESM-1.

For example, the amount of ESM-1 could be determined with a monoclonal antibody (such as a mouse antibody) against amino acids 85 to 184 of the ESM-1 polypeptide and/or with a goat polyclonal antibody.

The biomarker Angiopoietin-2 (abbreviated "Ang-2", frequently also referred to as ANGPT2) is well known in the art. It is a naturally occurring antagonist for both Ang-1 and TIE2 (see e.g. Maisonpierre et al., Science 277 (1997) 55-60). The protein can induce tyrosine phosphorylation of TEK/TIE2 in the absence of ANG-1. In the absence of angiogenic inducers, such as VEGF, ANG2-mediated loosening of cell-matrix contacts may induce endothelial cell apoptosis with consequent vascular regression. In concert with VEGF, it may facilitate endothelial cell migration and proliferation, thus serving as a permissive angiogenic signal. The sequence of human Angiopoietin is well known in the art.

Uniprot lists three isoforms of Angiopoietin-2: Isoform 1 (Uniprot identifier: 015123-1), Isoform 2 (identifier: 015123-2) and Isoform 3 (015123-3). In a preferred embodiment, the total amount of Angiopoietin-2 is determined. The total amount is preferably the sum of the amounts of complexed and free Angiopoietin-2.

## Determination of the amount of a biomarker

The term "determining" the amount of a biomarker as referred to herein (such as the one or more BMP10-type peptides or the natriuretic peptide) refers to the quantification of the biomarker, e.g. to measuring the level of the biomarker in the sample, employing appropriate methods of detection described elsewhere herein. The terms "measuring" and "determining" are used herein interchangeably.

In an embodiment, the determination of the amount of a biomarker comprises by contacting the sample with an agent that specifically binds to the biomarker (such as an antibody of the present invention), thereby forming a complex between the agent and said biomarker, detecting the amount of complex formed, and thereby measuring the amount of said biomarker. The determination may further comprise steps, such as contacting the sample with a second agent.

The biomarkers as referred to herein (such as the one or more BMP10-type peptides) can be detected using methods generally known in the art. Methods of detection generally encompass methods to quantify the amount of a biomarker in the sample (quantitative method). It is generally known to the skilled artisan which of the following methods are suitable for qualitative and/or for quantitative detection of a biomarker. Samples can be conveniently assayed for, e.g., proteins using Westerns and immunoassays, like ELISAs, RIAs, fluorescence- and luminescence-based immunoassays and proximity extension assays, which are commercially available. Further suitable methods to detect biomarkers include measuring a physical or chemical property specific for the peptide or polypeptide such as its precise molecular mass or NMR spectrum. Said methods comprise, e.g., biosensors, optical devices coupled to immunoassays, biochips, analytical devices such as mass-spectrometers, NMR- analyzers, or chromatography devices. Further, methods include microplate ELISA-based methods, fully-automated or robotic immunoassays (available for example on Elecsys™ analyzers), CBA (an enzymatic Cobalt Binding Assay, available for example on Roche-Hitachi™ analyzers), and latex agglutination assays (available for example on Roche-Hitachi™ analyzers).

For the detection of biomarker proteins as referred to herein a wide range of immunoassay techniques using such an assay format are available, see, e.g., U.S. Pat. Nos. 4,016,043, 4,424,279, and 4,018,653. These include both single-site and two-site or "sandwich" assays of the non-competitive types, as well as in the traditional competitive binding assays. These assays also include direct binding of a labeled antibody to a target biomarker.

Methods employing electrochemiluminescent labels are well-known. Such methods make use of the ability of special metal complexes to achieve, by means of oxidation, an excited state from which they decay to ground state, emitting electrochemiluminescence. For review see Richter, M.M., Chem. Rev. 2004;104: 3003-3036.

In an embodiment, the antibody (or an antigen-binding fragment thereof) to be used for measuring the amount of a biomarker is ruthenylated or iridinylated. Accordingly, the antibody (or an antigen-binding fragment thereof) shall comprise a ruthenium label. In an embodiment, said ruthenium label is a bipyridine-ruthenium(II) complex. Alternatively the antibody (or an antigen-binding fragment thereof) shall comprise an iridium label. In an embodiment, said iridium label is a complex as disclosed in WO 2012/107419.

In an embodiment of the sandwich assay for the determination of the one or more BMP 10-type peptides, the assay comprises a biotinylated first monoclonal antibody (or fragment thereof) and a ruthenylated second monoclonal antibody (or fragment thereof) that specifically binds one or more BMP10-type peptide.. The two antibodies form sandwich immunoassay complexes with the one or more BMP10-type peptides in the sample.

Measuring the amount of a polypeptide (such as a BMP10-type peptide or the natriuretic peptide) may, preferably, comprise the steps of (a) contacting the polypeptide with an agent that specifically binds said polypeptide, (b) (optionally) removing non-bound agent, (c) measuring the amount of bound binding agent, i.e. the complex of the agent formed in step (a). According to a preferred embodiment, said steps of contacting, removing and measuring may be performed by an analyzer unit. According to some embodiments, said steps may be performed by a single analyzer unit of said system or by more than one analyzer unit in operable communication with each other. For example, according to a specific embodiment, said system disclosed herein may include a first analyzer unit for performing said steps of contacting and removing and a second analyzer unit, operably connected to said first analyzer unit by a transport unit (for example, a robotic arm), which performs said step of measuring.

The agent which specifically binds the biomarker (herein also referred to as "binding agent") may be coupled covalently or non-covalently to a label allowing detection and measurement of the bound agent. Labeling may be done by direct or indirect methods. Direct labeling involves coupling of the label directly (covalently or non-covalently) to the binding agent. Indirect labeling involves binding (covalently or non-covalently) of a secondary binding agent to the first binding agent. The secondary binding agent should specifically bind to the first binding agent. Said secondary binding agent may be coupled with a suitable label and/or be the target (receptor) of a tertiary binding agent binding to the secondary binding agent. Suitable secondary and higher order binding agents may include antibodies, secondary antibodies, and the well-known streptavidin-biotin system (Vector Laboratories, Inc.). The binding agent or substrate may also be "tagged" with one or more tags as known in the art. Such tags may then be targets for higher order binding agents. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium complexes, iridium complexes, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be determined according to methods known in the art (e.g. using a light-sensitive film or a suit-able camera system). As for measuring the enzymatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. A radioactive label can be detected by any method known and appropriate, e.g. a light-sensitive film or a phosphor imager.

The amount of a polypeptide may be, also preferably, determined as follows: (a) contacting a solid support comprising a binding agent for the polypeptide as described elsewhere herein with a sample comprising the peptide or polypeptide and (b) measuring the amount of peptide or poly-peptide which is bound to the support. Materials for manufacturing supports are well-known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc.

In yet an aspect the sample is removed from the complex formed between the binding agent and the at least one marker prior to the measurement of the amount of formed complex. Accordingly, in an aspect, the binding agent may be immobilized on a solid support. In yet an aspect, the sample can be removed from the formed complex on the solid support by applying a washing solution.

"Sandwich assays" are among the most useful and commonly used assays encompassing a number of variations of the sandwich assay technique. Briefly, in a typical assay, an unlabeled (capture) binding agent is immobilized or can be immobilized on a solid substrate, and the sample to be tested is brought into contact with the capture binding agent. After a suitable period of incubation, for a period of time sufficient to allow formation of a binding agent-biomarker complex, a second (detection) binding agent labeled with a reporter molecule capable of producing a detectable signal is then added and incubated, allowing time sufficient for the formation of another complex of binding agent-biomarker-labeled binding agent. Any unreacted material may be washed away, and the presence of the biomarker is determined by observation of a signal produced by the reporter molecule bound to the detection binding agent. The results may either be qualitative, by simple observation of a visible signal, or may be quantitated by comparison with a control sample containing known amounts of biomarker.

The incubation steps of a typical sandwich assays can be varied as required and appropriate. Such variations include for example simultaneous incubations, in which two or more of binding agent and biomarker are co-incubated. For example, both, the sample to be analyzed and a labeled binding agent are added simultaneously to an immobilized capture binding agent. It is also possible to first incubate the sample to be analyzed and a labeled binding agent and to thereafter add an antibody bound to a solid phase or capable of binding to a solid phase.

The formed complex between a specific binding agent and the biomarker shall be proportional to the amount of the biomarker present in the sample. It will be understood that the specificity and/or sensitivity of the binding agent to be applied defines the degree of proportion of at least one marker comprised in the sample which is capable of being specifically bound. Further details on how the measurement can be carried out are also found elsewhere herein. The amount of formed complex shall be transformed into an amount of the biomarker reflecting the amount indeed present in the sample.

The terms "binding agent", "specific binding agent", "analyte-specific binding agent", "detection agent" and "agent that specifically binds to a biomarker" are used interchangeably herein. Preferably it relates to an agent that comprises a binding moiety which specifically binds the corresponding biomarker. Examples of "binding agents", "detection agents", "agents" are a nucleic acid probe, nucleic acid primer, DNA molecule, RNA molecule, aptamer, antibody, antibody fragment, peptide, peptide nucleic acid (PNA) or chemical compound. A preferred agent is an antibody which specifically binds to the biomarker to be determined. The term "antibody" herein is used in the broadest sense and encompasses various antibody structures, including but not limited to monoclonal antibodies, polyclonal antibodies, multispecific antibodies (e.g., bispecific antibodies), and antibody fragments so long as they exhibit the desired antigen-binding activity (i.e. antigen-binding fragments thereof). Preferably, the antibody is a polyclonal antibody (or an antigen-binding fragment therefrom). More preferably, the antibody is a monoclonal antibody (or an antigen binding fragment therefore Moreover, as described elsewhere herein, it is envisaged that two monoclonal antibodies are used that bind at different positions of the one or more BMP10-type peptides (in a sandwich immunoassay). Thus, at least one antibody is used for the determination of the amount of the BMP10-type peptide.

In an embodiment, the at least one antibody is a mouse monoclonal antibody. In another embodiment, the at least one antibody is a rabbit monoclonal antibody. In a further embodiment, the antibody is goat polyclonal antibody. In an even further embodiment, the antibody is a sheep polyclonal antibody.

In some embodiments, the at least one antibody, or fragment thereof, which specifically binds to the BMP10-type peptide is at least one antibody or fragment thereof described in the next section (with the title "Antibodies of the present invention").

The term "amount" as used herein encompasses the absolute amount of a biomarker as referred to herein (such as one or more BMP10-type peptides or the natriuretic peptide), the relative amount or concentration of the said biomarker as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response amounts determined from biological read out systems in response to the peptides or intensity signals obtained from specifically bound ligands. It is to be understood that values correlating to the aforementioned amounts or parameters can also be obtained by all standard mathematical operations.

The term "comparing" as used herein refers to comparing the amount of the biomarker (such as the one or more BMP10-type peptides and the natriuretic peptide such as NT-proBNP or BNP) in the sample from the subject with the reference amount of the biomarker specified elsewhere in this description. It is to be understood that comparing as used herein usually refers to a comparison of corresponding parameters or values, e.g., an absolute amount is compared to an absolute reference amount while a concentration is compared to a reference concentration or an intensity signal obtained from the biomarker in a sample is compared to the same type of intensity signal obtained from a first sample. The comparison may be carried out manually or computer-assisted. Thus, the comparison may be carried out by a computing device. The value of the determined or detected amount of the biomarker in the sample from the subject and the reference amount can be, e.g., compared to each other and the said comparison can be automatically carried out by a computer program executing an algorithm for the comparison. The computer program carrying out the said evaluation will provide the desired assessment in a suitable output format. For a computer-assisted comparison, the value of the determined amount may be compared to values corresponding to suitable references which are stored in a database by a computer program. The computer program may further evaluate the result of the comparison, i.e. automatically provide the desired assessment in a suitable output format. For a computer-assisted comparison, the value of the determined amount may be compared to values corresponding to suitable references which are stored in a database by a computer program. The computer program may further evaluate the result of the comparison, i.e. automatically provides the desired assessment in a suitable output format.

In accordance with the present invention, the amount of one or more BMP10-type peptides and optionally the amount of the at least one further biomarker (such as the natriuretic peptide) shall be compared to a reference. The reference is preferably a reference amount. The term "reference amount" is well understood by the skilled person. It is to be understood that the reference amount shall allow for the herein described assessment of atrial fibrillation. E.g., in connection with the method for diagnosing atrial fibrillation, the reference amount preferably refers to an amount which allows for allocation of a subject into either (i) the group of subjects suffering from atrial fibrillation or (ii) the group of subjects not suffering from atrial fibrillation. A suitable reference amount may be determined from a first sample to be analyzed together, i.e. simultaneously or subsequently, with the test sample.

It is to be understood that the amount of the one or more BMP10-type peptides is compared to a reference amount for the one or more BMP10-type peptides, whereas the amount of the at least one further biomarker (such as the natriuretic peptide) is compared to a reference amount for said at least one at least one further biomarker (such as the natriuretic peptide). If the amounts of two markers or more are determined, it is also envisaged that a combined score is calculated based on the amounts the two or more marker (such as the amount of the one or more BMP10-type peptides and the amount of the natriuretic peptide). In a subsequent step, the score is compared to a reference score.

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig MH. et al., Clin. Chem. 1993;39:561-577). The ROC graph is a plot of all the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1 - specificity for the complete range of thresholds suitable for making a distinction. On the y-axis is sensitivity, or the true-positive fraction, which is defined as the ratio of number of true-positive test results to the product of number of true-positive and number of false-negative test results. It is calculated solely from the affected subgroup. On the x-axis is the false-positive fraction, or 1 - specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/1 - specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis for a given event with a proper balance of sensitivity and specificity, respectively. Accordingly, the reference to be used for the method of the present invention, i.e. a threshold which allows to assess atrial fibrillation can be generated, preferably, by establishing a ROC for said cohort as described above and deriving a threshold amount therefrom. Dependent on a desired sensitivity and specificity for a diagnostic method, the ROC plot allows deriving a suitable threshold. It will be understood that an optimal sensitivity is desired for e.g. excluding a subject from suffering from atrial fibrillation (i.e. a rule out) whereas an optimal specificity is envisaged for a subject to be assessed as suffering from atrial fibrillation (i.e. a rule in). In an embodiment, the method of the present invention allows for the prediction that a subject is at risk of an adverse event associated with atrial fibrillation such as the occurrence or recurrence of Atrial Fibrillation and/or stroke.

In a preferred embodiment, the term "reference amount" herein refers to a predetermined value. Said predetermined value shall allow for assessing atrial fibrillation, and thus for diagnosing atrial fibrillation, for differentiating between paroxysmal and persistent atrial fibrillation, for prediction the risk of an adverse event associated with atrial fibrillation, for identifying a subject who shall be subjected to electrocardiography (ECG), or for the assessment of a therapy for atrial fibrillation. It is to be understood that the reference amount may differ based on the type of assessment. E.g., the reference amount for the one or more BMP10-type peptides for the differentiation of AF will be usually higher than the reference amount for the diagnosis of AF. However, this will be taken into account by the skilled person.

As set forth above, the term "assessing atrial fibrillation" preferably refers to the diagnosis of atrial fibrillation, the differentiation between paroxysmal and persistent atrial fibrillation, the prediction of a risk of an adverse event associated with atrial fibrillation, to the identification of a subject who shall be subjected to electrocardiography (ECG), or the assessment of a therapy for atrial fibrillation. In the following, these embodiments of the method of the present invention will be described in more detail. The definitions above apply accordingly.

Method for diagnosing atrial fibrillation

The term "diagnosing" as used herein means assessing whether a subject as referred to in accordance with the method of the present invention suffers from atrial fibrillation (AF), or not. In a preferred embodiment, it is diagnosed that a subject suffers from paroxysmal AF. In an alternative embodiment, it is diagnosed that a subject does not suffer from AF.

In accordance with the present invention, all types of AF can be diagnosed. Thus, the atrial fibrillation may be paroxysmal, persistent or permanent AF. Preferably, paroxysmal or persistent atrial fibrillation is diagnosed, in particular in a subject not suffering from permanent AF.

The actual diagnosis whether a subject suffers from AF, or not may comprise further steps such as the confirmation of a diagnosis (e.g. by ECG such as Holter-ECG). Thus, the present invention allows for assessing the likelihood that a patient suffers from atrial fibrillation. A subject who has an amount of the one or more BMP10-type peptides above the reference amount is likely to suffer from atrial fibrillation, whereas a subject who has an amount of the one or more BMP10-type peptides below the reference amount is not likely to suffer from atrial fibrillation. Accordingly, the term "diagnosing" in the context of the present invention also encompasses aiding the physician to assess whether a subject suffers from atrial fibrillation, or not.

Preferably, an amount of the one or more BMP10-type peptides (and optionally an amount of the at least one further biomarker such as ESM-1, Ang-2, FABP-3 and/or the natriuretic peptide) in the sample from a test subject which is (are) increased as compared to the reference amount (or to the reference amounts) is indicative for a subject suffering from atrial fibrillation, and/or an amount of the one or more BMP10-type peptides (and optionally an amount of the at least one further biomarker such as ESM-1, Ang-2, FABP-3 and/or the natriuretic peptide) in the sample from a subject which is (are) decreased as compared to the reference amount (or the reference amounts) is indicative for a subject not suffering from atrial fibrillation.

In a preferred embodiment, the reference amount, i.e. the reference amount for the one or more BMP10-type peptides and, if determined, the reference amount for the at least one further biomarker, shall allow for differentiating between a subject suffering from atrial fibrillation and a subject not suffering from atrial fibrillation. Preferably, said reference amount is a predetermined value.

In an embodiment, the method of the present invention allows for the diagnosis of a subject suffering from atrial fibrillation. Preferably, the subject is suffering from AF, if the amount of the one or more BMP10-type peptides (and optionally an amount of the at least one further biomarker such as ESM-1, Ang-2, FABP-3 and/or the natriuretic peptide) is (are) above the reference amount. In an embodiment, the subject is suffering from AF, if the amount of the one or more BMP10-type peptides is above a certain percentile (e.g. 99th percentile) upper reference limit (URL) of a reference amount.

In an embodiment of the method of diagnosing atrial fibrillation, said method further comprises a step of recommending and/or initiating a therapy for atrial fibrillation based on the results of the diagnosis. Preferably, a therapy is recommended or initiated if it is diagnosed that the subject suffers from AF. Preferred therapies for atrial fibrillation are disclosed elsewhere herein (such as anticoagulation therapies).

Method for differentiating between paroxysmal and persistent atrial fibrillation

The term "differentiating" as used herein means to distinguish between paroxysmal and persistent atrial fibrillation in a subject. The term as used herein, preferably, includes differentially diagnosing paroxysmal and persistent atrial fibrillation in a subject. Thus, the method of the present invention allows for assessing whether a subject with atrial fibrillation suffers from paroxysmal atrial fibrillation or persistent atrial fibrillation. The actual differentiation may comprise further steps such as the confirmation of the differentiation. Thus, the term "differentiation" in the context of the present invention also encompasses aiding the physician to differentiate between paroxysmal and persistent AF.

Preferably, an amount of the one or more BMP10-type peptides (and optionally an amount of the at least one further biomarker such as ESM-1, Ang-2, FABP-3 and/or the natriuretic peptide) in the sample from a subject which is (are) increased as compared to the reference amount (or to the reference amounts) is indicative for a subject suffering from persistent atrial fibrillation and/or an amount of the one or more BMP10-type peptides (and optionally an amount of the at least one further biomarker such as ESM-1, Ang-2, FABP-3 and/or the natriuretic peptide) in the sample from a subject which is (are) decreased as compared to a reference amount (or to the reference amounts) is indicative for a subject suffering from paroxysmal atrial fibrillation. In both AF types (paroxysmal and persistent), the amount of the BMP10-type peptide is increased as compared to the reference amount of non-AF subjects.

In a preferred embodiment, the reference amount(s) shall allow for differentiating between a subject suffering from paroxysmal atrial fibrillation and a subject suffering from persistent atrial fibrillation. Preferably, said reference amount is a predetermined value.

In an embodiment of the above method of differentiating between paroxysmal and persistent atrial fibrillation, the subject does not suffer from permanent atrial fibrillation.

Method for predicting the risk a risk of an adverse event associated with atrial fibrillation

The method of the present invention also contemplates a method for predicting the risk of an adverse event.

In an embodiment, the risk of an adverse event as set forth herein can be the prediction of any adverse event associated with atrial fibrillation. Preferably, said adverse event is selected from recurrence of atrial fibrillation (such as the recurrence of atrial fibrillation after cardioversion) and stroke. Accordingly, the risk of a subject (who suffers from atrial fibrillation) to suffer in the future from an adverse event (such as stroke or recurrence of atrial fibrillation) shall be predicted.

Further, it is envisaged that said adverse event associated with atrial fibrillation is the occurrence of atrial fibrillation in a subject has no known history of atrial fibrillation.

In a particularly preferred embodiment, the risk of stroke is predicted.

Accordingly, the present invention method for predicting the risk of stroke in a subject, comprising the steps of

a) determining the amount of one or more BMP10-type peptides in a sample from the subject, and

b) comparing the amount of the one or more BMP10-type peptides to a reference amount, whereby the risk of stroke is to be predicted.

In particular, the present invention relates to a method for predicting the risk of stroke in a subject, comprising the steps of

(a) determining, in at least one sample from the subject, the amount of one or more BMP10-type peptides (Bone Morphogenetic Protein 10) and, optionally, the amount of at least one further biomarker selected from the group consisting of a natriuretic peptide, ESM-1 (Endocan), Ang2 (Angiopoietin 2) and FABP-3 (Fatty acid binding protein 3), and

(b) comparing the amount of the one or more BMP10-type peptides to a reference amount for the BMP10-type peptide and, optionally, comparing the amount of the at least one further biomarker to a reference amount for said at least one further biomarker, whereby the risk of stroke is to be predicted.

Further, it is envisaged that the risk of a subject of recurrence of atrial fibrillation after cardioversion is predicted, for example the risk of recurrence of atrial fibrillation after pulmonary vein isolation or ablation therapy is predicted. Accordingly, the biomarkers as set forth herein are used as predictors of atrial fibrillation recurrence after therapeutic interventions such as pulmonary vein isolation and ablation therapies. Therapeutic success rates in such procedures depend on ablation strategies and patient characteristics and are reported between 50% to 60% for persistent AF (J Am Heart Assoc. 2013;2:e004549). In this embodiment, it is envisaged that the subject shall be analyzed prior to therapeutic intervention, i.e. the subject to be tested suffers from an episode of AF at the time of the testing, i.e. when the sample is obtained. In accordance with this embodiment, the amount of the biomarker(s) shall be determined in a sample that has been obtained before the (planned) intervention, preferably, one month before the planned intervention, more preferably one week before the (planned) intervention, most preferably, one day before the (planned) intervention. Thus, the prediction of AF recurrence risk with the biomarker is possible before the therapy. In accordance with the method of the present invention, the elevation of the biomarkers as referred to herein should help to stratify subjects with high risk from low risk for recurrent AFib after a therapeutic intervention. Depending on the risk detected or predicted, some patients with low probability of success might be treated differently than patients with high probability of success depending on a blood biomarker measured prior to the procedure as predictors for AF recurrence after a PVI or ablation.

Preferably, term "predicting the risk" as used herein refers to assessing the probability according to which the subject will suffer from an adverse event as referred to herein (e.g. of stroke). Typically, it is predicted whether a subject is at risk (and thus at elevated risk) or not at risk (and thus at reduced risk) of suffering from said adverse event. Accordingly, the method of the present invention allows for differentiating between a subject at risk and a subject not at risk of suffering from said adverse event. Further, it is envisaged that the method of the present invention allows for differentiating between a subject who is a reduced, average, or elevated risk.

As set forth above, the risk (and probability) of suffering from said adverse event within a certain time window shall be predicted. In a preferred embodiment of the present invention, the predictive window is a period of about three months, about six months, or, in particular, about one year. Thus, the short-term risk is predicted.

In another preferred embodiment, the predictive window is a period of about five years (e.g. for the prediction of stroke). Further, the predictive window might be a period of about six years (e.g. for the prediction of stroke). Alternatively, the predictive window may be about 10 years. Also, it is envisaged that the predictive window a period of 1 to 3 years. Thus, the risk to suffer from stroke within 1 to 3 year is predicted. Also, it is envisaged that the predictive window a period of 1 to 10 years. Thus, the risk to suffer from stroke within 1 to 10 years is predicted.

Preferably, said predictive window is calculated from the completion of the method of the present invention. More preferably, said predictive window is calculated from the time point at which the sample to be tested has been obtained. As will be understood by those skilled in the art, the prediction of a risk is usually not intended to be correct for 100% of the subjects. The term, however, requires that prediction can be made for a statistically significant portion of subjects in a proper and correct manner. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.1, 0.05, 0.01, 0.005, or 0.0001.

In a preferred embodiment, the expression "predicting the risk of suffering from said adverse event" means that the subject to be analyzed by the method of the present invention is allocated either into the group of subjects being at risk of suffering from said adverse event, or into the group of subjects not being at risk of suffering from said adverse event (such as stroke). Thus, it is predicted whether the subject is at risk or not at risk of suffering from said adverse event. As used herein "a subject who is at risk of suffering from said adverse event", preferably has an elevated risk of suffering from said adverse event (preferably within the predictive window). Preferably, said risk is elevated as compared to the average risk in a cohort of subjects. As used herein, "a subject who is not at risk of suffering from said adverse event", preferably, has a reduced risk of suffering from said adverse event (preferably within the predictive window). Preferably, said risk is reduced as compared to the average risk in a cohort of subjects. A subject who is at risk of suffering from said adverse event preferably has a risk of suffering from said adverse event such as recurrence or occurrence of atrial fibrillation of at least 20% or more preferably of at least 30%, preferably, within a predictive window of about one year. A subject who is not at risk of suffering from said adverse event preferably has a risk of lower than 12%, more preferably of lower than 10% of suffering from said adverse event, preferably within a predictive window of one year.

With respect to the prediction of stroke, a subject who is at risk of suffering from said adverse event preferably has a risk of suffering from said adverse event of at least 10% or more preferably of at least 13%, preferably, within a predictive window of about five years, or in particular of about six years. A subject who is not at risk of suffering from said adverse event preferably has a risk of lower than 10%, more preferably of lower than 8%, or most preferably of lower than 5% of suffering from said adverse event, preferably within a predictive window of about five years, or in particular of about six years. The risk may be higher, if the subject does not receive anticoagulation therapy. This will be taken into account by the skilled person.

Preferably, an amount of the one or more BMP10-type peptides (and optionally an amount of the at least one further biomarker such as ESM-1, Ang-2, FABP-3 and/or the natriuretic peptide) in the sample from a subject which is (are) increased as compared to the reference amount (or to the reference amounts) is indicative for a subject who is at risk of the adverse event associated with atrial fibrillation, and/or an amount of the one or more BMP10-type peptides (and optionally an amount of the at least one further biomarker such as ESM-1, Ang-2, FABP-3 and/or the natriuretic peptide) in the sample from a subject which is decreased as compared to the reference amount (or to the reference amounts) is indicative for a subject who is not at risk the adverse event associated with atrial fibrillation.

In a preferred embodiment, the reference amount (or reference amounts) shall allow for differentiating between a subject who is at risk of an adverse event as referred to herein and a subject who is not at risk of said adverse event. Preferably, said reference amount is a predetermined value.

The adverse event to be predicted is preferably stroke. The term "stroke" is well known in the art. As used herein, the term, preferably, refers to ischemic stroke, in particular to cerebral ischemic stroke. A stroke which is predicted by the method of the present invention shall be caused by reduced blood flow to the brain or parts thereof which leads to an undersupply of oxygen to brain cells. In particular, the stroke leads to irreversible tissue damage due to brain cell death. Symptoms of stroke are well known in the art. E.g., stroke symptoms include sudden numbness or weakness of face, arm or leg, especially on one side of the body, sudden confusion, trouble speaking or understanding, sudden trouble seeing in one or both eyes, and sudden trouble walking, dizziness, loss of balance or coordination. Ischemic stroke may be caused by atherothrombosis or embolism of a major cerebral artery, by coagulation disorders or nonatheromatous vascular disease, or by cardiac ischemia which leads to a reduced overall blood flow. The ischemic stroke is preferably selected from the group consisting of atherothrombotic stroke, cardioembolic stroke and lacunar stroke. Preferably, the stroke to be predicted is an acute ischemic stroke, in particular cardioembolic stroke. A cardioembolic stroke (frequently also referred to as embolic or thromboembolic stroke) can be caused by atrial fibrillation.

Preferably, said stroke shall be associated with atrial fibrillation. More preferably, the stroke shall be caused by atrial fibrillation. However, it is also envisaged that the subject has no history of atrial fibrillation.

Preferably, a stroke is associated with atrial fibrillation, if there is a temporal relationship between the stroke and an episode of atrial fibrillation. More preferably, a stroke is associated with atrial fibrillation, if the stroke is caused by atrial fibrillation. Most preferably, a stroke is associated with atrial fibrillation, if the stroke can be caused by atrial fibrillation. For example, a cardioembolic stroke (frequently also referred to as embolic or thromboembolic stroke) can be caused by atrial fibrillation. Preferably, a stroke associated with AF can be prevented by oral anticoagulation. Also preferably, the stroke is considered as associated with atrial fibrillation, if the subject to be tested suffers from atrial fibrillation and/or has a known history thereof. Also, in an embodiment, the stroke may be considered as being associated with atrial fibrillation, if the subject is suspected to suffer from atrial fibrillation.

The term "stroke" does, preferably, not include hemorrhagic stroke.

In a preferred embodiment of the aforementioned method of predicting an adverse event (such as stroke), the subject to be tested suffers from atrial fibrillation. More preferably, the subject has a known history of atrial fibrillation. In accordance with the method for predicting an adverse event, the subject preferably suffers from permanent atrial fibrillation, more preferably from persistent atrial fibrillation and most preferably from paroxysmal atrial fibrillation.

In an embodiment of the method of predicting an adverse event, the subject suffering from atrial fibrillation experiences episodes of atrial fibrillation when the sample is obtained. In another embodiment of the method of predicting an adverse event, the subject suffering from atrial fibrillation does not experiences episode of atrial fibrillation when the sample is obtained (and thus shall have a normal sinus rhythm). Further, the subject whose risk is to be predicted may be on anticoagulation therapy.

In another embodiment of the method of predicting an adverse event, the subject to be tested has no known history of atrial fibrillation. In particular, it is envisaged that the subject does not suffer from atrial fibrillation.

The method of the present invention may aid personalized medicine. In a preferred embodiment, the method for predicting the risk of stroke in a subject further comprises i) the step of recommending anticoagulation therapy, or ii) of recommending an intensification of anticoagulation therapy, if the subject has been identified to be at risk to suffer from stroke. In a preferred embodiment, the method for predicting the risk of stroke in a subject further comprises i) the step of initiating anticoagulation therapy, or ii) of intensifying anticoagulation therapy, if the subject has been identified to be at risk to suffer from stroke (by the method of the present invention).

If the test subject is on anticoagulation therapy, and if the subject has been identified not to be at risk to suffer from stroke (by the method of the present invention) the dosage of anticoagulation therapy may be reduced. Accordingly, a reduction of the dosage may be recommended. Be reducing the dosage, the risk to suffer from side effects (such as bleeding) may be reduced.

The term "recommending" as used herein means establishing a proposal for a therapy which could be applied to the subject. However, it is to be understood that applying the actual therapy whatsoever is not comprised by the term. The therapy to be recommended depends on the outcome of the provided by the method of the present invention.

In particular, the following applies:

If the subject to be tested does not receive anticoagulation therapy, the initiation of anticoagulation is recommended, if the subject has been identified to be at risk to suffer from stroke. Thus, anticoagulation therapy shall be initiated.

If the subject to be tested already receives anticoagulation therapy, the intensification of anticoagulation is recommended, if the subject has been identified to be at risk to suffer from stroke. Thus, anticoagulation therapy shall be intensified.

In a preferred embodiment, anticoagulation therapy is intensified by increasing the dosage of the anticoagulant, i.e. the dosage of the currently administered coagulant.

In a particularly preferred embodiment, anticoagulation therapy is intensified by increasing the replacing the currently administered anticoagulant with a more effective anticoagulant. Thus, a replacement of the anticoagulant is recommended.

It has been described that better prevention in high risk patients is achieved with the oral anticoagulant apixaban versus the vitamin K antagonist warfarin as shown in Hijazi at al., The Lancet 2016 387, 2302-2311,(Figure 4).

Thus, it is envisaged that the subject to be tested is a subject who is treated with a vitamin K antagonist such as warfarin or dicumarol. If the subject has been identified to be at risk to suffer from stroke (by the method of the present invention, it the replacement of the vitamin K antagonist with an oral anticoagulant, in particular dabigatran, rivaroxaban or apixaban is recommended. Accordingly, the therapy with the vitamin K antagonist is discontinued and therapy with an oral anticoagulant is initiated.

Method for identifying a subject who shall be subjected to electrocardiography (ECG)

In accordance with this embodiment of method of the present invention, it shall be assessed whether the subject to be tested with the biomarker shall be subjected to electrocardiography (ECG), i.e. to an electrocardiography assessment. Said assessment shall be carried for diagnosing, i.e. to detect the presence of absence of AF, in said subject.

The term "identifying a subject" as used herein preferably refers to using the information or data generated relating to the amount of the one or more BMP10-type peptides (and optionally the amount of the at least one further biomarker) in a sample of a subject to identify subject shall be subjected to ECG. The subject who is identified has an increased likelihood of suffering from AF. The ECG assessment is made as a confirmation.

Electrocardiography (abbreviated ECG) is the process of recording the electrical activity of the heart by suitable ECG. An ECG device records the electrical signals produced by the heart which spread throughout the body to the skin. The recording is of the electrical signal is achieved by contacting the skin of the test subject with electrodes comprised by the ECG device. The process of obtaining the recording is non-invasive and risk-free. The ECG is carried out for the diagnosis of atrial fibrillation, i.e. for the assessment of the presence of absence of atrial fibrillation in the test subject. In embodiment of the method of the present invention, the ECG device is a one-lead device (such as a one-lead handheld ECG-device). In another preferred embodiment the ECG device is a 12-lead ECG device such as a Holter monitor.

Preferably, an amount of the one or more BMP10-type peptides (and optionally an amount of the at least one further biomarker such as ESM-1, Ang-2, FABP-3 and/or the natriuretic peptide) in the sample from a test subject which is (are) increased as compared to the reference amount (or to the reference amounts) is indicative for a subject who shall be subjected to ECG, and/or an amount of the one or more BMP10-type peptides (and optionally an amount of the at least one further biomarker such as ESM-1, Ang-2, FABP-3 and/or the natriuretic peptide) in the sample from a subject which is (are) decreased as compared to the reference amount (or to the reference amounts) is indicative for a subject who shall not be subjected to ECG.

In a preferred embodiment, the reference amount shall allow for differentiating between a subject who shall be subjected to ECG and a subject who shall not be subjected to ECG. Preferably, said reference amount is a predetermined value.

In an embodiment of the aforementioned method, the method comprises identifying a subject who shall be subjected to electrocardiography, in particular, when the amount of the one or more BMP10-type peptides (and optionally an amount of the at least one further biomarker such as ESM-1, Ang-2, FABP-3 and/or the natriuretic peptide) in the sample from the test subject is (are) increased as compared to the reference amount (or to the reference amounts), and subjecting the identified subject to electrocardiography.

Method for the assessment of a therapy for atrial fibrillation

As used herein, the term "assessing a therapy for atrial fibrillation", preferably refers to the assessment of a therapy that aims to treat atrial fibrillation. In particular, the efficacy of a therapy shall be assessed.

The therapy to be assessed can be any therapy that aims to treat atrial fibrillation. Preferably, said therapy is selected from the group consisting of administration of at least one anticoagulant, rhythm control, rate control, cardioversion and ablation. Said therapies are well known in the art and are e.g. reviewed in Fuster V et al. Circulation 2011;123:e269-e367 which herewith is incorporated by reference in its entirety.

In an embodiment, the therapy is the administration of at least one anticoagulant, i.e. anticoagulation therapy. anticoagulation therapy is preferably a therapy which aims to reduce the risk of anticoagulation in said subject. Administration of at least one anticoagulant shall aim to reduce or prevent coagulation of blood and related stroke. In a preferred embodiment, at least one anticoagulant is selected from the group consisting of heparin, a coumarin derivative (i.e. a vitamin K antagonist), in particular warfarin or dicumarol, oral anticoagulants, in particular dabigatran, rivaroxaban or apixaban, tissue factor pathway inhibitor (TFPI), antithrombin III, factor IXa inhibitors, factor Xa inhibitors, inhibitors of factors Va and Villa and thrombin inhibitors (anti-IIa type). Accordingly, it is envisaged that the subject takes at least one of the aforementioned medicaments.

In preferred embodiment, the anticoagulant is a vitamin K antagonist such as warfarin or dicumarol. Vitamin K antagonists, such as warfarin or dicumarol are less expensive, but need better patient compliance, because of the inconvenient, cumbersome and often unreliable treatment with fluctuating time in therapeutic range. NOAC (new oral anticoagulants) comprise direct factor Xa inhibitors (apixaban, rivaroxaban, darexaban, edoxaban), direct thrombin inhibitors (dabigatran) and PAR-1 antagonists (vorapaxar, atopaxar).

In another preferred embodiment the anticoagulant and oral anticoagulant, in particular apixaban, rivaroxaban, darexaban, edoxaban, dabigatran, vorapaxar, or atopaxar.

Thus, the subject to be tested may be on therapy with an oral anticoagulant or a vitamin K antagonist at the time of the testing (i.e. at the time at which the sample is received.

In a preferred embodiment, the assessment of a therapy for atrial fibrillation is the monitoring of said therapy. In this embodiment, the reference amount is preferably the amount for the or more BMP10-type peptides in an earlier obtained sample (i.e. in a sample that has been obtained prior to the test sample in step a).

Optionally, the amount of the at least one further biomarker as referred to herein is determined in addition to the amount of the one or more BMP10-type peptides.

Accordingly, the present invention relates to a method for monitoring a therapy for atrial fibrillation in subject, said subject preferably suffering from atrial fibrillation, wherein said method comprises the steps of

(a) determining, in at first sample from the subject, the amount of one or more BMP10-type peptides (Bone Morphogenetic Protein 10-type peptides) and, optionally, the amount of at least one further biomarker selected from the group consisting of a natriuretic peptide, ESM-1 (Endocan), Ang2 (Angiopoietin 2) and FABP-3 (Fatty acid binding protein 3),

(b) determining, in a second sample from the subject, the amount of the one or more BMP10-type peptides and, optionally, the amount of at least one further biomarker selected from the group consisting of a natriuretic peptide, ESM-1 (Endocan), Ang2 (Angiopoietin 2) and FABP-3 (Fatty acid binding protein 3), wherein said second sample has been obtained after said first sample,

(c) comparing the amount of the one or more BMP10-type peptides in the first sample to the amount of the one or more BMP10-type peptides in said second sample, and optionally comparing the amount of said at least one further biomarker in the first sample to the amount of said at least one further biomarker in said second sample, thereby monitoring anticoagulation therapy.

The term "monitoring" as used herein, preferably, relates to assessing the effects a therapy as referred to herein elsewhere. Thus, the efficacy of a therapy (such as anticoagulation therapy) is monitored.

The aforementioned method may comprise the further step of monitoring the therapy based on the results of the comparison step carried out in step c). As will be understood by those skilled in the art, the prediction of a risk is usually not intended to be correct for 100% of the subjects. The term, however, requires that prediction can be made for a statistically significant portion of subjects in a proper and correct manner. Thus, the actual monitoring may comprise further steps such as the confirmation.

Preferably, by carrying out the method of the present invention it can be assessed whether the subject responds to said therapy or not. A subject responds to a therapy if the condition the subject improves between obtaining the first and the second sample. Preferably, a subject does not respond to the therapy if the condition worsened between obtaining the first and the second sample.

Preferably, the first sample is obtained prior to the initiation of said therapy. More preferably, the sample is obtained within one week in particular within two weeks prior to the initiation of said therapy. However, it is also contemplated that the first sample may is obtained after initiation of said therapy (but before the second sample is obtained). In this case an ongoing therapy is monitored.

Thus, the second sample shall be obtained after the first sample. It is to be understood that the second sample shall be obtained after the initiation of said therapy.

Moreover, it is particularly contemplated that the second sample is obtained after a reasonable period of time after obtaining the first sample. It is to be understood, that the amounts of biomarkers referred herein, do not instantly change (e.g. within 1 minute or 1 hour) Therefore, "reasonable" in this context refers to intervals between obtaining the first and second sample which intervals allow the biomarker(s) to adjust. Therefore, the second sample, preferably, is obtained at least one month after said first sample, at least three months, or, in particular, at least six month after said first sample.

Preferably, a decrease and, more preferably, a significant decrease, and, most preferably, a statistically significant decrease of the amount(s) of the biomarker(s), i.e. of the one or more BMP10-types peptide and optionally of the natriuretic peptide in the second sample as compared to the amount(s) of the biomarker(s) in the first sample is indicative for a subject who responds to the therapy. Thus, the therapy is efficient. Also preferably, no change of the concentration of the one or more BMP10-type peptides or an increase, more preferably, a significant increase, most preferably, a statistically significant increase of the amount(s) of the biomarker(s) in the second sample as compared to the amount(s) of the biomarker(s) in the first sample is indicative for a subject who does not respond to the therapy. Thus, the therapy is not efficient.

The terms "significant" and "statistically significant" are known by the person skilled in the art. Thus, whether an increase or decrease is significant or statistically significant can be determined without further ado by the person skilled in the art using various well known statistic evaluation tools. For example, a significant increase or decrease is an increase or decrease of at least 10%, in particular of at least 20%.

A subject is considered to respond to the therapy, if the therapy reduces the risk of the subject of recurrence of atrial fibrillation. A subject is considered as not to respond to the therapy, if the therapy does not the risk of the subject of recurrence of atrial fibrillation.

In an embodiment, the intensity of the therapy is increased if the subject does not respond to the therapy. Moreover, it is envisaged that the intensity of the therapy is decreased, if a subject responds to the therapy. For example, the intensity of a therapy can be increased by increasing the dosage of the administered medicament. For example, the intensity of a therapy can be decreased by decreasing the dosage of the administered medicament. Thereby, it might be possible to avoid unwanted adverse side effects such as bleeding.

In another preferred embodiment, the assessment of a therapy for atrial fibrillation is the guidance of a therapy for atrial fibrillation. The term "guidance" as used herein, preferably, relates to adjusting the intensity of a therapy, such as increasing or decreasing the dose of oral anti coagulation, based on the determination of the biomarker, i.e. the BMP10-type peptide, during therapy.

In a further preferred embodiment, the assessment of a therapy for atrial fibrillation is the stratification of a therapy for atrial fibrillation. Thus, a subject shall be identified who is eligible to a certain therapy for atrial fibrillation. The term "stratification" as used herein, preferably, relates to selecting an adequate therapy based on the given or particular particular risk, molecular path identified and/or expected efficacy of the particular drug or procedure. Depending on the risk detected or predicted, particularly patients with minimal or no symptoms related to the arrhythmia will become eligible to control of the ventricular rate, cardioversion or ablation, who otherwise would receive only antithrombotic therapy. Depending on the risk detected or predicted, some patients with low probability of success might be treated differently than patients with high probability of success depending on a blood biomarker measured prior to the procedure as predictors for AF recurrence after a PVI or ablation.

The definitions and explanations given herein above apply *mutatis mutandis* to the following methods of the present invention (except if stated otherwise). For example, the terms "subject", "sample", "determining" have been defined above. Further, the determination preferably comprises contacting the sample with at least one agent that is capable of binding within amino acid region 37 to 299 of the polypeptide shown in SEQ ID NO: 1.

Interestingly, it was shown in the studies underlying the present invention that BMP10-type peptides can be used for estimating the risk, presence and/or severity of cerebrovascular injury as cause of dementia and cognitive dysfunction in patients, such as in atrial fibrillation patients. Specifically, it was shown that biomarker correlates with existence of white matter lesions (WML) in patients. The higher the amount of the biomarker, the higher the extent of white matter lesions (and *vice versa*). Therefore, the can be used as a marker for assessing the extent of white matter lesions.

Furthermore, the present invention relates to method for assessing the extent of white matter lesions in a subject, said method comprising

> a) determining the amount of one or more BMP10-type peptides in a sample from the subject, and
> b) assessing the extent of white matter lesions in a subject based on the amount determined in step a).

The term "white matter lesions" is well-known in the art. White matter refers to areas of the central nervous system (CNS) that are predominantly made up of myelinated axons. White matter lesions (also refered to as "White matter disease") is commonly detected on brain MRI of aging individuals as white matter hyperintensities (WMH), or 'leukoaraiosis". It has been described that the presence and extent of WMH is a radiographic marker of small cerebral vessel disease and an important predictor of the life-long risk of stroke, cognitive impairment, and functional disability (Chutinet A, Rost NS. White matter disease as a biomarker for long-term cerebrovascular disease and dementia. Curr Treat Options Cardiovasc Med. 2014;16(3):292. doi:10.1007/s11936-013-0292-z). The determination of RET allows for assessing the extent of WMI,s, i.e. the burden of WMLs. Accordingly, the biomarker allows for the quantification of WMLs in a subject, i.e. it is a marker for loss of volume of functional brain tissue.

The extent of white matter lesions can be expressed by the Fazekas score (Fazekas, JB Chawluk, A Alavi, HI Hurtig, and RA Zimmerman American Journal of Roentgenology 1987 149:2, 351-356). The Fazekas score is ranging from 0 to 3. 0 indicates no WML, 1 mild WML, 2 moderate WMI, and 3 severe WML.

WMI, extent can be caused by clinically silent strokes. Therefore, the biomarker RET could be further used for aiding in the assessment whether a subject has experienced one or more silent strokes in past, i.e. before the sample has been obtained.

Silent strokes, i.e. silent cerebral strokes, are known in the art and are, for example, described in Conen et al. (Conen et al., J Am Coll Cardiol 2019;73:989-99) which herewith is incorporated by reference with respect to its entire disclosure content. Silent strokes are clinically silent strokes in patients without a clinical history of stroke or transient ischemic attack. Accordingly, the subject to be tested shall have no known history of stroke and/or TIA (transient ischemic attack).

Moreover, the present invention relates to a method for the assessment whether a subject has experienced one or more silent strokes, said method comprising

> a) determining the amount one or more BMP10-type peptides in a sample from the subject,
> b) comparing the amount determined in step a) to a reference, and
> c) assessing whether a subject has experienced one or more silent strokes.

The following preferably applies as diagnostic algorithm: An amount of biomarker larger than the reference is indicative for a subject who has experienced one or more silent strokes, and/or an amount of lower than the reference is indicative for a subject who has not experienced silent strokes.

Accordingly, the present invention relates to a method for predicting dementia, such as vascular dementia and/or Alzheimer's disease in a subject, said method comprising

> a) determining the amount of one or more BMP10-type peptides in a sample from the subject,
> b) comparing the amount determined in step a) to a reference, and
> c) predicting the risk of dementia in said subject.

The term "predicting dementia" as used herein, preferably, refers to assessing the probability according to which the subject will suffer from dementia. Typically, it is predicted whether a subject is at risk (and thus at elevated risk) or not at risk (and thus at reduced risk) of suffering from dementia.

In an embodiment, the predictive window a period of 1 to 3 years. Thus, the risk to suffer from dementia within 1 to 3 year is predicted. In a preferred embodiment, the predictive window a period of 1 to 10 years. Thus, the risk of the subject to suffer from dementia within 1 to 10 years is predicted. Preferably, said predictive window is calculated from the completion of the method of the present invention. More preferably, said predictive window is calculated from the time point at which the sample to be tested has been obtained.

The term "dementia" as used herein, preferably, refers to a condition which can be characterized as a loss, usually progressive, of cognitive and intellectual functions, without impairment of perception or consciousness caused by a variety of disorders, but most commonly associated with structural brain disease. The most common type of dementia is Alzheimer's disease, which makes up 50% to 70% of cases. Other common types include vascular dementia (25%), dementia with Lewy bodies, and frontotemporal dementia. The term "dementia" includes, but is not restricted to AIDS dementia, Alzheimer dementia, presenile dementia, senile dementia, catatonic dementia, Lewy body dementia (diffuse Lewy body disease), multi-infarct dementia (vascular dementia), paralytic dementia, posttraumatic dementia, dementia praecox, vascular dementia.

In an embodiment, the term dementia refers to vascular dementia, Alzheimer's disease, dementia with Lewy bodies, and/or frontotemporal dementia. Thus, the risk to suffer from vascular dementia, Alzheimer's disease, dementia with Lewy bodies, and/or frontotemporal dementia is predicted.

In an embodiment, the risk to suffer from "Alzheimer's disease" is predicted. The term "Alzheimer's disease" is well known in the art. Alzheimer's disease is a chronic neurodegenerative disease that usually starts slowly and gradually worsens over time. As the disease advances, symptoms can include problems with language, disorientation, mood swings, loss of motivation, not managing self-care, and behavioural issues.

In an embodiment, the risk to suffer from "vascular dementia" is predicted. The term "vascular dementia" preferably refers to progressive loss of memory and other cognitive functions caused by vascular injury or disease within the brain. Thus, the term shall refer to the symptoms of dementia caused by problems of circulation of blood to the brain. It may occur after a stroke or build up over time.

In connection with the prediction of the risk of dementia, the diagnostic algorithm is preferably as follows: Preferably, an amount of the biomarker larger than the reference is indicative for a subject who is at risk of dementia, and/or an amount of the biomarker lower than the reference is indicative for a subject who is not at risk of dementia.

The definitions and explanations given herein above apply *mutatis mutandis* to the following methods of the present invention (except if stated otherwise). For example, the terms "subject", "sample", "determining" have been defined above. Further, the determination preferably comprises contacting the sample with at least one agent that is capable of binding within amino acid region 37 to 299 of the polypeptide shown in SEQ ID NO: 1.

The present invention further concerns a method of aiding in the assessment of atrial fibrillation, said method comprising the steps of:

a) providing at least one sample from a subject,
b) determining, in the at least one sample provided in step a), the amount of one or more BMP10-type peptides and, optionally, the amount of at least one further biomarker selected from the group consisting of a natriuretic peptide, ESM-1 (Endocan), Ang2 and FABP-3 (Fatty acid binding protein 3), and
c) providing information on the determined amount of the one or more BMP10-type peptides and optionally on the determined amount of the at least one further biomarker to a physician, thereby aiding in the assessment of atrial fibrillation.

The physician shall be the attending physician, i.e. the physician who requested the determination of the biomarker(s). The aforementioned method shall aid the attending physician in the assessment of atrial fibrillation. Thus, the method does not encompass the diagnosis, prediction, monitoring, differentiation, identification as referred to above in connection with the method of assessing atrial fibrillation.

Step a) of the aforementioned method of obtaining the sample does not encompass the drawing of the sample from the subject. Preferably, the sample is obtained by receiving a sample from said subject. Thus, the sample can have been delivered.

In an embodiment, the method above is a method of aiding in the prediction of stroke, said method comprising the steps of:

a) providing at least one sample from a subject as referred to herein in connection with the method of assessing atrial fibrillation, in particular in connection with the method of predicting atrial fibrillation,
b) determining the amount of one or more BMP10-type peptides and the amount of at least one further biomarker selected from the group consisting of a natriuretic peptide, ESM-1 (Endocan), Ang2 and FABP-3 (Fatty acid binding protein 3), and
c) providing information on the determined amount of the one or more BMP10-type peptides and optionally on the determined amount of the at least one further biomarker to a physician, thereby aiding in the prediction of stroke.

The present invention further relates to a method, comprising:

a) providing an assay for one or more BMP10-type peptides and, optionally, at least one further assay for a further biomarker selected from the group consisting of a natriuretic peptide, ESM-1 (Endocan), Ang2 and FABP-3 (Fatty acid binding protein 3), and

b) providing instructions for use of assay results obtained or obtainable by said assay(s) in the assessment of atrial fibrillation.

The purpose of the aforementioned method is, preferably, the aid in the assessment of atrial fibrillation.

The instructions shall contain a protocol for carrying out the method of assessing atrial fibrillation as described herein above. Further, the instructions shall contain at least one value for a reference amount for the one or more BMP10-type peptides and optionally at least one value for a reference amount for a natriuretic peptide.

The "assay" is preferably a kit adapted for determining the amount of the biomarker. The term "kit" is explained herein below. E.g. said kit shall comprise at least one detection agent for one or more BMP10-type peptides and optionally and at least one further agent selected from the group consisting of an agent which specifically binds to a natriuretic peptide, an agent which specifically binds to ESM-1, an agent which specifically binds Ang2 and an agent which specifically binds to FABP-3. Thus, one to four detection agents may be present. The detection agents for the one to four biomarkers can be provided in a single kit or in separate kits.

The test result obtained or obtainable by said test, is the value for the amount of the biomarker(s).

In an embodiment, step b) comprises providing instructions for using of test results obtained or obtainable by said test(s) in prediction of stroke (as described herein elsewhere).

The present invention further pertains to computer-implemented method for assessing atrial fibrillation, comprising

a) receiving, at a processing unit, a value for the amount of one or more BMP10-type peptides, and, optionally at least one further value for the amount of at least one further biomarker selected from the group consisting of a natriuretic peptide, ESM-1 (Endocan), Ang2 and FABP-3 (Fatty acid binding protein 3), wherein said amount of the one or more BMP10-type peptides and, optionally, the amount of the at least one further biomarker have been determined in a sample from a subject,

b) comparing, by said processing unit, the value or values received in step (a) to a reference or to references, and

c) assessing atrial fibrillation based in the comparison step b).

The above-mentioned method is a computer-implemented method. Preferably, all steps of the computer-implemented method are performed by one or more processing units of a computer (or computer network). Thus, the assessment in step (c) is carried out by a processing unit. Preferably, said assessment is based on the results of step (b).

The value or values received in step (a) shall be derived from the determination of the amount of the biomarker from a subject as described elsewhere herein. Preferably, the value is a value for the concentration of the biomarker. The value will be typically received by the processing unit by uploading or sending the value to the processing unit. Alternatively, the value can be received by the processing unit by inputting the value via an user interface.

In an embodiment of the aforementioned method, the reference (or references) set forth in step (b) is (are) established from a memory. Preferably, a value for the reference is established from the memory.

In an embodiment of the aforementioned computer-implemented method of the present invention, the result of the assessment made in step c) is provided via a display, configured for presenting result.

In an embodiment of the aforementioned computer-implemented method of the present invention, the method may comprise the further step of transferring the information on the assessment made in step c) to the subject's electronic medical records.

Method of for the diagnosis of heart failure

Further, is has been shown in the studies of the present invention that the determination of the amount of one or more BMP10-type peptides in a sample from a subject allows for the diagnosis of heart failure. Accordingly, the present invention also contemplates a method for diagnosing heart failure based on the amount of the one or more BMP10-type peptides (and optionally further based on a natriuretic peptide, ESM-1, Ang2 and/or FABP3.

The definitions given herein above in connection with the assessment of atrial fibrillation apply *mutatis mutandis* to the following (except if stated otherwise).

Accordingly, the present invention further relates to a method for diagnosing heart failure in a subject, said method comprising the steps of

a) determining the amount of one or more BMP10-type peptides in a sample from the subject, and

b) comparing the amount of the one or BMP10-type peptides to a reference amount, whereby heart failure is to be diagnosed.

The method for diagnosing heart failure may further comprise the determination the amount of at least one further biomarker selected from the group consisting of a natriuretic peptide, ESM-1 (Endocan), Ang2 (Angiopoietin 2) and FABP-3 (Fatty acid binding protein 3) and the comparison with a suitable reference amount.

Thus, the method for diagnosing heart failure may comprise the steps of:

a) determining, in at least one sample from the subject, the amount of one or more BMP10-type peptides (Bone Morphogenetic Protein 10-type peptides) and, optionally, the amount of at least one further biomarker selected from the group consisting of a natriuretic peptide, ESM-1 (Endocan), Ang2 (Angiopoietin 2) and FABP-3 (Fatty acid binding protein 3), and

b) comparing the amount of the one or more BMP10-type peptides to a reference amount for the one or more BMP10-type peptides and, optionally, comparing the amount of the at least one further biomarker to a reference amount for said at least one further biomarker, whereby heart failure is to be diagnosed.

The term "diagnosing" as used herein means assessing whether a subject as referred to in accordance with the method of the present invention suffers from heart failure, or not. The actual diagnosis whether a subject suffers from heart failure, or not, may comprise further steps such as the confirmation of a diagnosis. Thus, the diagnosis of heart failure is understood as an aid in the diagnosis of heart failure. Accordingly, the term "diagnosing" in the context of the present invention also encompasses aiding the physician to assess whether a subject suffers from heart failure, or not.

The term "heart failure" (abbreviated "HF") is well known by the skilled person. As used herein, the term preferably relates to an impaired systolic and/or diastolic function of the heart being accompanied by overt signs of heart failure as known to the person skilled in the art. Preferably, heart failure referred to herein is also chronic heart failure. Heart failure according to the present invention includes overt and/or advanced heart failure. In overt heart failure, the subject shows symptoms of heart failure as known to the person skilled in the art.

In an embodiment of present invention, the term "heart failure" refers to heart failure with reduced left ventricular ejection fraction (HFrEF). In another embodiment of present invention, the term "heart failure" refers to heart failure with preserved left ventricular ejection fraction (HFpEF).

HF can be classified into various degrees of severity. According to the NYHA (New York Heart Association) classification, heart failure patients are classified as belonging to NY-HA classes I, II, III and IV. A patient having heart failure has already experienced structural and functional changes to his pericardium, myocardium, coronary circulation or cardiac valves. He will not be able to fully restore his health, and is in need of a treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001; 38; 2101-2113, updated in 2005, see J. Am. Coll. Cardiol. 2005; 46; e1-e82). 4 stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy and/or impaired LV function would be designated as stage B. Stage C then denotes patients with current or past symptoms of HF associated with underlying structural heart disease (the bulk of patients with HF), and stage D designates patients with truly refractory HF.

As used herein, the term "heart failure", preferably, includes stages A, B, C and D of the ACC/AHA classification referred to above. Also, the term includes NYHA class I, II, III and IV. Thus, the subject may or may not show typical symptoms of heart failure.

In a preferred embodiment, the term "heart failure" refers the heart failure stage A or, in particular, heart failure stage B according to the ACC/AHA classification referred to above. The identification of these early stages, in particular of stage A, is advantageous because treatment could be initiated before irreversible damage occurs.

The subject to be tested in accordance with the method of diagnosing heart failure preferably does not suffer from atrial fibrillation. However, it is also envisaged the subject suffers from atrial fibrillation. The term "atrial fibrillation" is defined in connection with the method of assessing heart failure.

Preferably, the subject to be tested in connection with the method of diagnosing heart failure is suspected to suffer heart failure.

The term "reference amount" has been defined in connection with the method of assessing atrial fibrillation. The reference amount that is applied in the method for diagnosing heart failure, in principle, can be determined as described above.

Preferably, an amount of the one or more BMP10-type peptides (and optionally an amount of the at least one further biomarker such as ESM-1, Ang-2, FABP-3 and/or the natriuretic peptide) in the sample from a subject which is increased as compared to the reference amount is indicative for a subject suffering from heart failure and/or wherein an amount of the one or more BMP10-type peptides (and optionally an amount of the at least one further biomarker such as ESM-1, Ang-2, FABP-3 and/or the natriuretic peptide) in the sample from a subject which is decreased as compared to the reference amount is indicative for a subject not suffering from heart failure.

In an embodiment of the method of diagnosing heart failure, said method further comprises a step of recommending and/or initiating a therapy for heart failure based on the results of the diagnosis. Preferably, a therapy is recommended or initiated if it is diagnosed that the subject suffers from heart failure. Preferably, the heart failure therapy comprises administration of at least one medicament selected from the group consisting of angiotensin converting enzyme (ACE) inhibitors, angiotensin II receptor blockers, beta blockers and aldosterone antagonists. Examples for angiotensin converting enzyme (ACE) inhibitors, angiotensin II receptor blockers, beta blockers and aldosterone antagonists are described in the next section.

Method for predicting the risk of a subject of hospitalization

It is known that some subjects progress more rapidly to heart failure, and thus are at elevated risk of hospitalization due to heart failure. It is important to identify these subjects as early as possible since this would allow for therapeutic measures that prevent or delay the progression to heart failure.

Advantageously, it has been found in the studies underlying the present invention that the amount of one or more BMP10-type peptides in a sample of a subject allows for identifying subjects who are at risk of heart failure hospitalization. For example, subjects in the fourth quartile of BMP 10 of the analyzed cohort (Example 4) had an about fourfold risk of heart failure hospitalization within a period of three years as compared to subjects in the first quartile.

Accordingly, the present invention further relates to a method for predicting the risk of a subject of hospitalization due to heart failure, said method comprising the steps of

(a) determining, in at least one sample from the subject, the amount of one or more BMP10-type peptides (Bone Morphogenetic Protein 10-type peptides), and, optionally, the amount of at least one further biomarker selected from the group consisting of a natriuretic peptide, ESM-1 (Endocan), Ang2 (Angiopoietin 2) and FABP-3 (Fatty acid binding protein 3), and
(b) comparing the amount of the one or more BMP10-type peptides to a reference amount for the one or more BMP10-type peptides, and, optionally, comparing the amount of the at least one further biomarker to a reference amount for said at least one further biomarker

The definitions and explanations made in connection the method of assessing atrial fibrillation and the method of diagnosing heart failure, preferably, apply to the method for predicting the risk of a subject of hospitalization due to heart failure

The above method may further comprise step (c) of predicting the risk of a subject of hospitalization due to heart failure. Thus, steps (a), (b), (c) are preferably as follows:

(a) determining, in at least one sample from the subject, the amount of one or more BMP10-type peptides (Bone Morphogenetic Protein 10-type peptides) and, optionally, the amount of at least one further biomarker selected from the group consisting of a natriuretic peptide, ESM-1 (Endocan), Ang2 (Angiopoietin 2) and FABP-3 (Fatty acid binding protein 3), and
(b) comparing the amount of the BMP10-type peptide to a reference amount, and, optionally, comparing the amount of the at least one further biomarker to a reference amount for said at least one further biomarker, and
(c) predicting the risk of a subject of hospitalization due to heart failure.

Preferably, the prediction is based on the results of the comparison in step (b).

The expression "hospitalization" is well understood by the skilled person and, preferably means that the subject is admitted to a hospital, in particular on an in-patient basis. The hospitalization should be due to heart failure. Thus, heart failure shall be the cause for the hospitalization. Preferably, the hospitalization is hospitalization due to acute or chronic heart failure. Thus, the heart failure includes both acute and chronic heart failure. More preferably, the hospitalization is hospitalization due to acute heart failure. Thus, the risk of a subject of hospitalization due to heart failure is predicted.

The term "heart failure" has been defined above. The definition applies accordingly. In some embodiments, the hospitalization is due heart failure classified as stage C or D according to according to the ACC/AHA classification. The ACC/AHA classification is well known in the art and described e.g. in Hunt et. al. (Journal of the American College of Cardiology, Volume 46, Issue 6, 20 September 2005, Pages e1-e82, ACC/AHA Practice Guidelines) which is herewith incorporated by reference in its entirety.

In accordance with the aforementioned method, the risk of a subject of hospitalization due to heart failure shall be predicted. Thus, a subject can be identified who is at risk of hospitalization due to heart failure, or who is not at risk hospitalization due to heart failure. Accordingly, the term "predicting the risk" as used herein in accordance with the aforementioned method, preferably, refers to assessing the probability of hospitalization due to heart failure. In some embodiments, the above method of the present invention allows for differentiating between a subject at risk and a subject not at risk of hospitalization due to heart failure.

In accordance with the present invention, the term "predicting the risk" is understood as an aid in the prediction of the risk of hospitalization due to heart failure. The final prediction, in principle, will be carried out by physician and may include further diagnostic results.

As will be understood by those skilled in the art, the prediction of a risk is usually not intended to be correct for 100% of the subjects. The term, preferably, means that the prediction can be made for a statistically significant portion of subjects in a proper and correct manner. Whether a portion is statistically significant can be determined without further ado by the person skilled in the art using various well known statistic evaluation tools, e.g., determination of confidence intervals, p-value determination, Student's t-test, Mann-Whitney test etc. Details are found in Dowdy and Wearden, Statistics for Research, John Wiley & Sons, New York 1983. Preferred confidence intervals are at least 90%, at least 95%, at least 97%, at least 98%, or at least 99%. The p-values are, preferably, 0.1, 0.05, 0.01, 0.005, or 0.0001.

Preferably, the risk/probability within a certain time window is predicted. In some embodiments, said predictive window is calculated from the completion of the method of the present invention. In particular, said predictive window is calculated from the time point at which the sample to be tested has been obtained.

In a preferred embodiment of the present invention, the predictive window, preferably, is an interval of at least 1 year, at least 2 years, at least 3 years, at least 4 years, at least 5 years, or at least 10 years, or any intermitting time range. In another preferred embodiment of the present invention, the predictive window, preferably, is a period of up to 5 years, more preferably of up to 4 years, or most preferably, of up 3 years. Thus, the risk within a period of up to three, up to four or up to five years is predicted. Also, it is envisaged that the predictive window a period of 1 to 5 years. Alternatively, the predictive window may be a period of 1 to 3 years.

In a preferred embodiment, the risk of hospitalization due to heart failure within three years is predicted.

Preferably, the subject to be analyzed by the above method of the present invention is allocated either into the group of subjects being at risk of hospitalization due to heart failure, or into the group of subjects being not at risk of hospitalization due to heart failure. At subject who is at risk, preferably, is a subject who is at elevated risk of hospitalization due to heart failure (in particular within the predictive window). Preferably, said risk is elevated as compared to the risk in a cohort of subjects (i.e. a group of subjects). At subject who is not at risk, preferably, is a subject who is at reduced risk of hospitalization due to heart failure (in particular within the predictive window). Preferably, said risk is reduced as compared to the average risk in a cohort of subjects (i.e. a group of subjects). Accordingly, the method of the present invention allows for differentiating between an elevated risk and a reduced risk. A subject who is at risk of preferably has a risk of 12% or larger, or, more preferably of 15% or larger, or most preferably of 20% or larger of hospitalization due to heart failure, preferably, within a predictive window of 3 years. A subject who is not at risk preferably has a risk of lower than 10%, more preferably of lower than, 8%, or most preferably of lower than 7% of hospitalization due to heart failure, preferably, within a predictive window of 3 years.

The term "reference amount" has been defined elsewhere herein. The definition applies accordingly. The reference amount to be applied in the above method shall allow for predicting the risk of hospitalization due to heart failure. In some embodiments, the reference amount shall allow for differentiating between a subject who is at risk of hospitalization due to heart failure and a subject who is not at risk of hospitalization due to heart failure. In some embodiments, said reference amount is a predetermined value.

Preferably, an amount of the one or more BMP10-type peptides in the sample from a subject which is increased as compared to the reference amount is indicative for a subject being at risk of hospitalization due to heart failure. Also preferably, an amount of the one or more BMP10-type peptides in the sample from a subject which is decreased as compared to the reference amount is indicative for a subject who is not at risk of hospitalization due to heart failure.

If more than one biomarker is determined, the following applies:
Preferably, an amount of the one or more BMP10-type peptides and an amount (or amounts) of the at least one further biomarker in the sample from a subject which is increased as compared to the respective reference amount is indicative for a subject being at risk of hospitalization due to heart failure. Also preferably, an amount of the one or more BMP10-type peptides and an amount (or amounts) of the at least one further biomarker in the sample from a subject which is decreased as compared to the respective reference amount is indicative for a subject who is not at risk of hospitalization due to heart failure.

The term "sample" has been defined elsewhere herein. The definition applies accordingly. In some embodiments, the sample is a blood, serum or plasma sample.

The term "subject" has been defined elsewhere herein. The definition applies accordingly. In some embodiments, the subject is a human subject. Preferably, the subject to be tested is 50 years of age or older, more preferably 60 years of age or older, and most preferably 65 years of age or older. Further, it is envisaged that the subject to be tested is 70 years of age or older. Moreover, it is envisaged that the subject to be tested is 75 years of age or older. Also, the subject may be between 50 and 90 years.

In an embodiment, the subject to be tested a history of heart failure. In another embodiment, the subject to be tested has no history of heart failure.

The method of the present invention may aid personalized medicine. In a preferred embodiment, the above method for predicting the risk of a subject of hospitalization due to heart failure further comprises the step of recommending and/or initiating at least one suitable therapy, if it is predicted that the subject is at risk of hospitalization due to heart failure. Accordingly, the present invention also pertains to a method of treatment.

Preferably, term "therapy" as used in the context of the method for predicting the risk of a subject of hospitalization due to heart failure encompasses life style changes, diet regimen, interventions on the body as well as medicinal treatment, i.e. treatment with a medicament (or with medicaments). Preferably, the said therapy aims to reduce the risk of hospitalization due to heart failure. In an embodiment, the therapy is the administration of a medicament (or medicaments). Preferably, the medicament is selected from the group consisting of an angiotensin-converting enzyme (ACE) inhibitor, an angiotensin receptor antagonist (ARB), an aldosterone antagonist and a beta blocker.

In some embodiments, the medicament is a beta blocker, such as are proprenolol, metopro-lol, bisoprolol, carvedilol, bucindolol, and nebivolol. In some embodiments, the medicament is an ACE inhibitor, such as Enalapril, Captopril, Ramipril and Trandolapril. In some embodiments, the medicament is an angiotensin II receptor blocker, such as Losartan, Valsartan, Irbesartan, Candesartan, Telmisartan and Eprosartan. In some embodiments, the medicament is an aldosterone antagonist such as Eplerone, Spironolactone, Canrenone, Mexrenone and Prorenone.

Life style changes include smoking cessation, moderation of alcohol consumption, increased physical activity, weight loss, sodium (salt) restriction, weight management and healthy eating, daily fish oil, salt restriction.

Moreover, the present invention relates to the use (in particular, the in vitro use, e.g. in a sample from a subject) of

i) one of more BMP10-type peptides and optionally of at least one further biomarker selected from the group consisting of a natriuretic peptide, ESM-1 (Endocan), Ang2 and FABP-3 (Fatty acid binding protein 3), and/or

ii) at least one agent that specifically binds to one or more BMP10-type peptides, and, optionally, at least one further agent selected from the group consisting of an agent which specifically binds to a natriuretic peptide, an agent which specifically binds to ESM-1, an agent which specifically binds to Ang2 and an agent which specifically binds to FABP-3, for a) assessing atrial fibrillation b) predicting the risk of stroke in a subject, and/or c) diagnosing heart failure.

The terms mentioned in connection with the aforementioned use such as "sample", "subject", "detection agent", "specifically binding", "atrial fibrillation", and "assessing atrial fibrillation" have been defined in connection with the method for assessing atrial fibrillation. The definitions and explanations apply accordingly.

The present invention further relates to the *in vitro* use of a BMP10-type peptide, and/or of at least one agent that specifically binds to a BMP10-type peptide for predicting dementia in a subject.

The present invention further relates to the *in vitro* use of a BMP10-type peptide, and/or of at least one agent that specifically binds to a BMP10-type peptide for assessing the extent of white matter lesions in a subject.

The present invention further relates to the *in vitro* use of a BMP10-type peptide, and/or of at least one agent that specifically binds to a BMP10-type peptide for assessing whether a subject has experienced one or more silent strokes.

The present invention further concerns the use (in particular, the in vitro use, e.g. in a sample from a subject) of a BMP10-type peptide, and/or of at least one agent that specifically binds to a BMP10-type peptide for predicting the risk of a subject of hospitalization due to heart failure.

Preferably, the aforementioned uses are an in vitro uses. Moreover, the detection agent is preferably and antibody such as a monoclonal antibody (or an antigen binding fragment thereof).

The present invention also relates to a kit. In an embodiment, the kit of the present invention comprises at least one agent which specifically binds to one or more BMP10-type pep-tides and at least one further agent selected from the group consisting of an agent which specifically binds to a natriuretic peptide, an agent which specifically binds to ESM-1, an agent which specifically binds Ang2 and an agent which specifically binds to FABP-3.

Preferably, said kit is adapted for carrying out the method of the present invention, i.e. the method for assessing atrial fibrillation, or the method of diagnosing heart failure, or the method of predicting the risk of a subject of hospitalization due to heart failure. Optionally, said kit comprises instructions for carrying out the said method.

In some embodiments, the at least one agent which specifically binds to one or BMP10-type peptides is at least one antibody or fragment thereof described in the next section (with the title "Antibodies of the present invention").

The term "kit" as used herein refers to a collection of the aforementioned components, preferably, provided separately or within a single container. The container also comprises instructions for carrying out the method of the present invention. These instructions may be in the form of a manual or may be provided by a computer program code which is capable of carrying out the calculations and comparisons referred to in the methods of the present invention and to establish the assessment or diagnosis accordingly when implemented on a computer or a data processing device. The computer program code may be provided on a data storage medium or device such as an optical storage medium (e.g., a Compact Disc) or directly on a computer or data processing device. Moreover, the kit may, preferably, comprise standard amounts for a BMP10-type peptide for calibration purposes. In a preferred embodiment, the kit further comprises standard amounts for the at least one further biomarker as referred to herein (such as the natriuretic peptide, or ESM-1) for calibration purposes

In an embodiment, said kit is used for assessing atrial fibrillation *in vitro.* In an alternative embodiment, said kit is used for diagnosing heart failure *in vitro.* In an alternative embodiment, said kit is used for predicting the risk of hospitalization due to heart failure *in vitro.* In the methods and uses of the present invention described herein above and in the claims, antibodies (of fragments thereof) can be used that are described in the next section.

The definitions and explanations made herein above apply *mutatis mutandis* to the following.

Antibodies of the present invention

The present invention also relates to an antibody (such as monoclonal antibody) or fragment thereof which binds one or more BMP10-type peptides. In particular, it shall bind NT-proBMP10.

The antibody (or fragment thereof) shall be capable of binding to at least one of amino acid region of the polypeptide having a sequence shown in SEQ ID NO: 1 as disclosed in the previous section.

Accordingly, the antibody (or fragment thereof) of the present invention shall be capable of binding NT-proBMP10, i.e. it shall be capable of binding within amino acid region 22 to 316 of the polypeptide shown in SEQ ID NO: 1. Since region is contained in preproBMP10 and proBMP10, the antibody (or fragment thereof) shall be capable of binding preproBMP10, proBMP10 and NT-proBMP10. However, the antibody (or fragment) shall not bind BMP10, i.e. mature BMP10.

In a preferred embodiment, the antibody or fragment thereof which binds one or more BMP10-type peptides, such as NT-proBMP10, shall be capable of binding within amino acid region 37 to 299 of the polypeptide shown in SEQ ID NO: 1, i.e. it is capable of binding to an epitope contained in the region which starts at the amino acid at position 37 and ends with the amino acid at position 299 of the polypeptide shown in SEQ ID NO: 1.

For example, the antibody or fragment thereof which binds one or more BMP10-type pep-tides, such as NT-proBMP10, is capable of binding within amino acid region 110 to 200 of the polypeptide shown SEQ ID NO: 1, i.e. said at least agent binds to an epitope contained in this region.

For example, the antibody or fragment thereof which binds one or more BMP10-type pep-tides, such as NT-proBMP10, is capable of binding within amino acid region 37 to 195, such as within amino acid region 37 to 185 of the polypeptide shown in SEQ ID NO: 1, i.e. said at least agent binds to an epitope contained in this region.

For example, the antibody or fragment thereof which binds to one or more BMP10-type pep-tides, such as NT-proBMP10, is capable of binding within amino acid region 160 to 299, such as within amino acid region 171 to 299 of SEQ ID NO: 1, i.e. said at least agent binds to an epitope contained in this region.

For example, the antibody or fragment thereof which binds one or more BMP10-type pep-tides, such as NT-proBMP10, is capable of binding within amino acid region 160 to 195, such as within amino acid region 171 to 185 of SEQ ID NO: 1.

For example, the antibody or fragment thereof which binds one or more BMP10-type pep-tides, such as NT-proBMP10, is capable of binding binds to an epitope contained in amino acid region 37 to 47 of SEQ ID NO: 1 (SLFGDVFSEQD, SEQ ID NO 2).

For example, the antibody or fragment thereof which binds one or more BMP10-type pep-tides, such as NT-proBMP10, is capable of binding to an epitope contained in amino acid region 171 to 185 of SEQ ID NO: 1 (LESKGDNEGERNMLV, SEQ ID NO: 3). For example, said at least one agent binds to an epitope contained in amino acid region 173 to 181 of SEQ ID NO: 1 (SKGDNEGER, SEQ ID NO: 4).

For example, the antibody or fragment thereof which binds one or more BMP10-type pep-tides, such as NT-proBMP10, is capable of binding to an epitope contained in amino acid region 291 to 299 of SEQ ID NO: 1 (SSGPGEEAL, SEQ ID NO: 5).

In the studies underlying the present invention, the inventors have generated monoclonal antibodies (in rabbits) against human NT-proBMP10. 280 antibodies were subjected to a detailed kinetic screening. In this screening, antibodies were identified which show favorable kinetic properties which makes them particularly useful for the detection of BMP10-type peptides, such as of NT-proBMP10 and/or proBMP10. For example, antibodies were identified which show a fast velocity of the formation of the complex of NT-proBMP10-antibody complex. Further, the antibodies show a slow dissociation of the complex, resulting in long half-life of the complex. Clones 11A5, 11C10, 13G6, 14C8, 2H8 and 9E7 showed comparable fast complex formation velocity and complex half lifes t/2 diss > 15 minutes. Clone 13G6 showed the

EP 4 528 281 A2

slowest dissociation ($k_d$ < 1.0E-04 s$^{-1}$), resulting in the complex-half-life t/2-diss > 115 minutes.

The identified antibodies were as follows: 11A2, 11A5, 11C10, 13G6, 14C8, 2H8, 3H8, 8G5 and 9E7. The sequences of the antibodies are provided in the following Tables A to D.

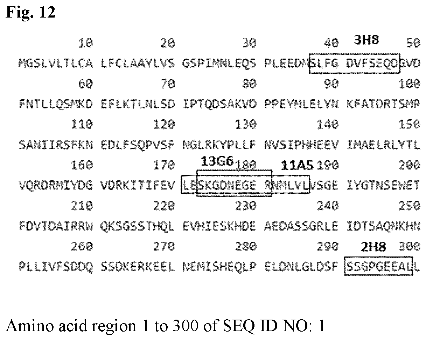

For four of the aforementioned antibodies, linear epitopes could be detected (see Examples section and Figure 12). Two of the antibodies bound to the same region.

The epitope of 3H8 comprises a sequence shown in SEQ ID NO: 2 (SLFGDVFSEQD). The epitope of 11A5 comprises a sequence shown in SEQ ID NO: 3 (LESKGDNEGERNMLV).

The epitope of 13G6 comprises a sequence shown in SEQ ID NO: 4 (SKGDNEGER).

The epitope of 2H8 comprises a sequence shown in SEQ ID NO: 5 (SSGPGEEAL).

The antibody (or fragment thereof) or the agent that is used for determining the amount of BMP10-type peptide may thus bind to the above regions.

Table A summarizes the amino acid sequences of the variable heavy (VH) chains of the antibodies of the present invention

Table A: Variable heavy (VH) chain domains of the present invention

| Clone | Designation | Amino Acid Sequence | SEQ ID NO |
|---|---|---|---|
| 11A2 | VH-1 | QSLEESGGRLVTPGTPLTLTCTVSGIDLSRNVMSWVRQAPGE-GLEWIGSINTGGSTWYASWAEGRLTISKSSTTVDLKISSPT-TEDTATYFCARGVYGHSNGYYSLWGQGTLVTVSS | 7 |
| 11A5 | VH-2 | QSVEESGGRLVTPGTPLTLTCTASGFSLSSYAMSWVRQAPGK-GLQFIGYISGGGSTYYASWVKGRFTISKTSTTVDLKLTSPT-TEDTATYFCARGYPGYISGTWAVGQGTLVTVSS | 8 |
| 11C10 | VH-3 | QSLEESGGRLVTPGTALTVTCTVSGIDLSRNLMSWVRQAPGE-GLEWIGSINFRNITWYASWAKGRFTISKTSTTVDLRISSPT-TEDTATYFCARGVYVNSNGYYSLWGQGTPVTVSS | 9 |
| 13G6 | VH-4 | QSLEESGGRLVTPGTPLTLTCTVSGFSLSNYA-MSWVRQAPGMGLEYIGYISASGNTYYASWVKGRFTISKTSTT-VDLKMTSPTTEDTATYFCARGYSGWISGTWAVGQGTLVTVSS | 10 |
| 14C8 | VH-5 | QSVEESGGRLVTPGTPLTLTCTASGIDLSRYAMTWVRQAPGK-GLEWIGIIGASSGTWYASWAKGRFTIAKTSTTVDLRITSPT-TEDTATYFCARDNGDSKDYAFDPWGPGTLVTVSS | 11 |
| 2H8 | VH-6 | QSVEESGGRLVTPGTPLTLTCTASAFSLSRYVMSWVRQAPGK-GLEFIGFINASGSTSYASWAKGRFTISKTSTTVDLKMTSLT-TEDTATYFCATGNLWGPGTLVTVSS | 12 |
| 3H8 | VH-7 | QSVEESGGRLVTPGTPLTLTCTASGFSLSKYDMSWVRQAPGK-GLEWIGAIGGRGVTDYTTWAKGRFTISKTATTVDLKMTSPT-TEDTATYFCVRALAGDTLWGPGTLVTVSS | 13 |
| 8G5 | VH-8 | QSLEESGGDLVKPGASLTLTCTASGFSFSS-SYWICWVRQAPGKGLEWIACIYAGSSDITYYASWAKGRFT-VSKTSSPTVTLQMTSLTAADTATYFCARGRYYVDGYA-DYYPGDFNLWGPGTLVTVSS | 14 |
| 9E7 | VH-9 | QSLEESGGGLVQPEGSLTLTCIASAFSFSS-GYYMCWVRQAPGKGLEWIACIYAGSSGTTYYASWAKGRF-TISKASSTTVTLLMTSLTAADTATYFCARGPGAS-GYRLGLWGPGTLVTVSS | 15 |

Table B summarizes the amino acid sequences of the variable light chains of the antibodies of the present invention (see below)

Table B: Variable light (VL) chain domains of the present invention

| Clone | Designation | Amino Acid Sequence | SEQ ID NO |
|---|---|---|---|
| 11A2 | VL-1 | ALVMTQTPASVEAAVGGTVTINCQASQSIS-NLLAWYQQKPGQPPKVLIYAASPLASGVSPRFKGSGSGTHFTLT-VSDLECADAATYYCQSYWGGSGERYLNTFGGGTEVVVK | 16 |
| 11A5 | VL-2 | AVLTQTPSPVSAAVGGTVSISCQSSQSVVNN-NRCSWFQQKPGQPPKQLIYSASTLASGVPSRFKGSGSGTQFTL-TISDVQCDDAATYYCLGDYVSYGDSAFGGGTEVVVK | 17 |
| 11C10 | VL-3 | DIVMTQTPAS-VEAAVGGTVTINCQASQSVSNLLAWYQQKPGQPPKLLIYGASK-LESGVPSRFKGSGSGTQFTLTINDLECDDAATYYCQTYWGGDGT-SYLNPFGGGTEVVVK | 18 |
| 13G6 | VL-4 | AVLTQTPSPVSAAVGGTVNIGCQSSQSVVNNNRLS-WFQQRPGQPPKQLIYRASTLASGVPSRFKGSGSGTQFTL-TISDVQCDDAATYYCLGDYVSYSEAAFGGGTEVVVK | 19 |
| 14C8 | VL-5 | IDMTQTPSPVSAAVGDTVTINCQASENIYS-FLAWYQQKPGHSPNLLIYFASKLVSGVPSRFKGSGSGTQFTL-TISDVQCDDAATYYCQQTYTYSNGDNPFGGGTEVVVK | 20 |
| 2H8 | VL-6 | DIVMTQTPSS-VSGPVGGTVTINCQASQNIYSNLAWYQQKPGQPPKLLIYYAST-LVSGVPSRFKGSGSGTQFTLTISDLECANAATYYCQSKDGPTS-STYGAFGGGTEVVVR | 21 |
| 3H8 | VL-7 | AQVLTQTASPVSAAVGSTVTINCQASQNIYKYNN-LAWYQQKPGQPPKRLIYEASKLASGVPSRFSGSGSGTQFTLTIS-GVQCEDAATYYCQGTFECSSADCFGFGGGTEVVVK | 22 |
| 8G5 | VL-8 | DIVMTQTPASVEAAVGGTVTIKCQASQSISSYLAWYQQKSGQPP-KLLIYLASTLESGVPSRFKGSGSGTEFTLTISDLE-CADAATYYCQSYYYSSSSSYGSAFGGGTEVVVK | 23 |
| 9E7 | VL-9 | DVVMTQTPASVSEPVGGTVTIKCQASEDIYRLLAWYQQKPGQPP-KLLIYDASDLASGVPSRLSGSGSGTEYTLSISDLE-CADAATYYCQQGYYISGGDSFGGGTEVVVK | 24 |

The heavy chain CDR sequences are shown in Table C. The light chain CDR sequences are shown in Table D below. Complementarity determining regions (CDRs) of an antibody can be identified, for example, by using the system described in Kabat et al. in Sequences of Proteins of Immunological Interest, 5th Ed., US Dept. of Health and Human Services, PHS, NIH, NIH Publication no. 91-3242, 1991.

Table C: CDRH Sequences

| Clone | Designation | SEQ ID | CDR1 sequence | Designation | SEQ ID | CDR2 sequence | Designation | SEQ ID NO | CDR3 sequence |
|---|---|---|---|---|---|---|---|---|---|
| 11A2 | CDRH1-1 | 25 | RNVMS | CDRH2-1 | 43 | SINTGGSTWYASWAEG | CDRH3-1 | 61 | GVYGHSNGYYSL |
| 11A5 | CDRH1-2 | 26 | SYAMS | CDRH2-2 | 44 | Y!SGGGSTYYASWVKG | CDRH3-2 | 62 | GYPGYISGTWA |
| 11C10 | CDRH1-3 | 27 | RNLMS | CDRH2-3 | 45 | S!NFRN!TWYASWAKG | CDRH3-3 | 63 | GVYVNSNGYYSL |
| 13G6 | CDRH1-4 | 28 | NYAMS | CDRH2-4 | 46 | YISASGNTYYASWVKG | CDRH3-4 | 64 | GYSGWISGTWA |
| 14C8 | CDRH1-5 | 29 | RYAMT | CDRH2-5 | 47 | IIGASSGTWYASWAKG | CDRH3-5 | 65 | DNGDSKDYAFDP |
| 2H8 | CDRH1-6 | 30 | RYVMS | CDRH2-6 | 48 | FINASGSTSYASWAKG | CDRH3-6 | 66 | GNL |
| 3H8 | CDRH1-7 | 31 | KYDMS | CDRH2-7 | 49 | AIGGRGVTDYTTWAKG | CDRH3-7 | 67 | ALAGDTL |
| 8G5 | CDRH1-8 | 32 | SSSYWIC | CDRH2-8 | 50 | C!YAGSSD!TYYASWAKG | CDRH3-8 | 68 | GRYYVDGYADYYPGDFNL |
| 9E7 | CDRH1-9 | 33 | SSGYYMC | CDRH2-9 | 51 | CIYAGSSGTTYYASWAKG | CDRH3-9 | 69 | GPGASGYRLGL |

Table D: CDRL Sequences

| Clone | Designation | SEQ ID | CDR1 | Designation | SEQ ID N | CDR2 | Designation | SEQ ID | CDR3 |
|---|---|---|---|---|---|---|---|---|---|
| 11A2 | CDRL1-1 | 34 | QASQSISNLLA | CDRL2-1 | 52 | AASPLAS | CDRL3-1 | 70 | QSYWGGSGERYLNT |
| 11A5 | CDRL1-2 | 35 | QSSQSVVNNNRCS | CDRL2-2 | 53 | SASTLAS | CDRL3-2 | 71 | LG DYVSYG DSA |
| 11C10 | CDRL1-3 | 36 | QASQSVSNLLA | CDRL2-3 | 54 | GASKLES | CDRL3-3 | 72 | QTYWGGDGTSYLNP |
| 13G6 | CDRL1-4 | 37 | QSSQSVVNNNRLS | CDRL2-4 | 55 | RASTLAS | CDRL3-4 | 73 | LGDYVSYSEAA |
| 14C8 | CDRL1-5 | 38 | QASENIYSFLA | CDRL2-5 | 56 | FASKLVS | CDRL3-5 | 74 | QQTYTYSNGDNP |
| 2H8 | CDRL1-6 | 39 | QASQNIYSNLA | CDRL2-6 | 57 | YASTLVS | CDRL3-6 | 75 | QSKDGPTSSTYGA |
| 3H8 | CDRL1-7 | 40 | QASQNIYKYNNLA | CDRL2-7 | 58 | EASKLAS | CDRL3-7 | 76 | QGTFECSSADCFG |
| 8G5 | CDRL1-8 | 41 | QASQSISSYLA | CDRL2-8 | 59 | LASTLES | CDRL3-8 | 77 | QSYYYSSSSSYGSA |
| 9E7 | CDRL1-9 | 42 | QASEDIYRLLA | CDRL2-9 | 60 | DASDLAS | CDRL3-9 | 78 | QQGYYISGGDS |

The antibody (or fragment thereof) of the present invention shall specifically bind the preproBMP, the proBMP10 and the NT-proBMP10 polypeptide. Accordingly, the antigen binding protein shall bind to preproBMP, the proBMP10 and NT-proBMP10 within the amino acid region 22 to 316 of SEQ ID NO: 1 as described elsewhere herein in more detail. It is to be understood that it does not bind to mature BMP10. However, it may be used in combination with an antigen-binding protein which binds mature BMP10.

The present invention is not limited to the identified antibodies, i.e. 11A2, 11A5, 11C10, 13G6, 14C8, 2H8, 3H8, 8G5 and 9E7, but also encompass variants thereof which bind one or more BMP10-types, preferably within the same region. For example, a variant of 13G6 shall bind the same or essentially the same epitope as 13G6.

Preferably, the antibody of the present invention, or fragment thereof, shall comprise a light chain variable domain (or variant thereof) and a heavy chain variable domain (or variant thereof) of the antibody 11A2, 11A5, 11C10, 13G6, 14C8, 2H8, 3H8, 8G5 or 9E7. For example, it may comprise a light chain variable domain (or variant thereof) and a heavy chain variable domain (or variant thereof) of the 13G6, For example, it may comprise a light chain variable domain (or variant thereof) and a heavy chain variable domain (or variant thereof) of the antibody 11C10.

In a preferred embodiment, the antibody of the present invention, or antigen-binding fragment thereof, comprises a heavy chain variable domain selected from VH1, VH2, VH3, VH4, VH5, VH6, VH7, VH8 and VH9 as shown in Table A (or a variant thereof) and/or a light chain variable domain selected from VL1, VL2, VL3, VL4, VL5, VL6, VL7, VL8 and VL9 as shown in Table B (or a variant thereof). Each heavy chain variable domain shown in Table A above can be combined with each light chain variable domain shown in Table B. In a preferred embodiment, the heavy chain variable domain in Table A is combined with the corresponding light chain variable domain in Table B, for example VH1 with VL1, VH2 with VL2, VH3 with VL3 etc.

In some embodiments, a variant of polypeptide as referred to in accordance with the present invention shall have an amino acid sequence which differs due to at least one amino acid substitution, deletion and/or addition wherein the amino acid sequence of the variant is still, preferably, at least 80%, 85%, 90%, 95%, 98%, 99% identical with the amino sequence of the polypeptide.

In some embodiments, a variant of an antibody(or fragment antibody )comprises a heavy chain variable domain that is at least 80%, 85%, 90%, 95%, 98%, or 99% identical to the heavy chain variable domain comprising a sequence shown in SEQ ID NO: 7, 8, 9, 10, 11, 12, 13, 14 or 15 (see Table A) and/or a light chain variable domain that is, in increasing order of preference at least 80%, 85%, 90%, 95%, 98%, or 99% identical to the light chain variable domain comprising a sequence as shown in SEQ ID NO: 16, 17, 18, 19, 20, 21, 22, 23 or 24 (see Table B).

Alternatively or additionally, the variant of an antibody, or fragment thereof, comprises antibody, or fragment thereof, of the present invention comprises the six CDRs of the parent antibody, wherein said six CDRs differ by not more than a total of three, two, or in particular one amino acid additions, substitutions, and/or deletions from the respective parent CDR (see Table C and D).

Thus, the antibody of the present invention, or fragment thereof may comprise, in some embodiments, a heavy chain variable domain that is at least 80%, 85%, 90%, 95%, 98%, 99%, or 100% identical to the heavy chain variable domain comprising a sequence shown in SEQ ID NO: 7, 8, 9, 10, 11, 12, 13, 14 or 15 (see Table A) and/or a light chain variable domain that is, in increasing order of preference at least 80%, 85%, 90%, 95%, 98%, 99% or 100% identical to the light chain variable domain comprising a sequence as shown in SEQ ID NO: 16, 17, 18, 19, 20, 21, 22, 23 or 24 (see Table B). Each heavy chain variable domain shown in can be combined with each light chain variable domain. However, it is preferred that corresponding sequences are combined (e.g. SEQ ID NO: 7 with SEQ ID NO: 16).

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity. Preferably, standard parameters are applied for determining the degree of sequence identity of two sequences. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad. Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. In an embodiment, the percent identity between two amino acid sequences is determined using the Needleman and Wunsch algorithm (Needleman 1970, J. Mol. Biol. (48):444-453) which has been incorporated into the needle program in the EMBOSS software package (EMBOSS: The European Molecular Biology Open Software Suite, Rice, P., Longden, I., and Bleasby, A., Trends in Genetics 16(6), 276-277, 2000), a BLOSUM62 scoring matrix, and a gap opening penalty of 10 and a gap entension pentalty of 0.5. A preferred, non-limiting example of parameters to be used for aligning two amino acid sequences using the needle program are the default parameters, including the EBLOSUM62 scoring matrix, a gap opening penalty of 10 and a gap extension penalty of 0.5.

In an aspect, the antibody of the present invention, or fragment thereof, comprises a heavy chain variable domain having a sequence as shown in SEQ ID NO: 7, and a light chain variable domain having a sequence as shown in SEQ ID NO: 16.

In an aspect, the antibody of the present invention, or fragment thereof, comprises a heavy chain variable domain having a sequence as shown in SEQ ID NO: 8, and a light chain variable domain having a sequence as shown in SEQ ID NO: 17.

In an aspect, the antibody of the present invention, or fragment thereof, comprises a heavy chain variable domain having a sequence as shown in SEQ ID NO: 9, and a light chain variable domain having a sequence as shown in SEQ ID NO: 18.

In an aspect, the antibody of the present invention, or fragment thereof, comprises a heavy chain variable domain having a sequence as shown in SEQ ID NO: 10, and a light chain variable domain having a sequence as shown in SEQ ID NO: 19.

In an aspect, the antibody of the present invention, or fragment thereof, comprises a heavy chain variable domain having a sequence as shown in SEQ ID NO: 11, and a light chain variable domain having a sequence as shown in SEQ ID NO: 20.

In an aspect, the antibody of the present invention, or fragment thereof, comprises a heavy chain variable domain having a sequence as shown in SEQ ID NO: 12, and a light chain variable domain having a sequence as shown in SEQ ID NO: 21.

In an aspect, the antibody of the present invention, or fragment thereof, comprises a heavy chain variable domain having a sequence as shown in SEQ ID NO: 13, and a light chain variable domain having a sequence as shown in SEQ ID NO: 22.

In an aspect, the antibody of the present invention, or fragment thereof, comprises a heavy chain variable domain having a sequence as shown in SEQ ID NO: 14, and a light chain variable domain having a sequence as shown in SEQ ID NO: 23.

In an aspect, the antibody of the present invention, or fragment thereof, comprises a heavy chain variable domain having a sequence as shown in SEQ ID NO: 15, and a light chain variable domain having a sequence as shown in SEQ ID NO: 24.

Alternatively or additionally, the antibody, or fragment thereof, of the present invention comprises

(a) a light chain variable domain comprising

(a1) a light chain CDR1 that differs by not more than a total of three, two, or in particular one amino acid additions, substitutions, and/or deletions from a light chain CDR1 shown in Table D,
(a2) a light chain CDR2 that differs by not more than a total of three, two, or in particular one amino acid additions, substitutions, and/or deletions from a light chain CDR2 shown in Table D,
(a3) a light chain CDR3 that differs by not more than a total of three, two, or in particular one amino acid additions, substitutions, and/or deletions from a light chain CDR3 shown in Table D, and

(b) a heavy chain variable domain comprising

(b1) a heavy chain CDR1 that differs by not more than a total of three, two, or in particular one amino acid additions, substitutions, and/or deletions from a heavy chain CDR1 shown in Table C,
(b2) a heavy chain CDR2 that differs by not more than a total of three, two, or in particular one amino acid additions, substitutions, and/or deletions from a heavy chain CDR2 shown in Table C, and
(b3) a heavy chain CDR3 that differs by not more than a total of three, two, or in particular one amino acid additions, substitutions, and/or deletions from a heavy chain CDR3 shown in Table C.

For example, the antibody, or fragment thereof, of the present invention comprises

(a) a light chain variable domain comprising

(a1) a light chain CDR1 that differs by not more than a total of three, two, or in particular one amino acid additions, substitutions, and/or deletions from a light chain CDRL1-4 shown in Table D,
(a2) a light chain CDR2 that differs by not more than a total of three, two, or in particular one amino acid additions, substitutions, and/or deletions from a light chain CDRL2-4 shown in Table D,
(a3) a light chain CDR3 that differs by not more than a total of three, two, or in particular one amino acid additions, substitutions, and/or deletions from a light chain CDRL3-4 shown in Table D, and

(b) a heavy chain variable domain comprising

(b 1) a heavy chain CDR1 that differs by not more than a total of three, two, or in particular one amino acid additions, substitutions, and/or deletions from a heavy chain CDRH1-1 shown in Table C,
(b2) a heavy chain CDR2 that differs by not more than a total of three, two, or in particular one amino acid additions, substitutions, and/or deletions from a heavy chain CDRH2-4 shown in Table C, and
(b3) a heavy chain CDR3 that differs by not more than a total of three, two, or in particular one amino acid additions, substitutions, and/or deletions from a heavy chain CDRH3-4 shown in Table C.

Preferably, the antibody, or fragment thereof, of the present invention comprises

(c) a light chain variable domain comprising

(a1) a light chain CDR1 shown in Table D (and thus a light chain CDR1 sequence selected from SEQ ID NOs:34-42),
(a2) a light chain CDR2 shown in Table D (and thus a light chain CDR2 sequence selected from SEQ ID NOs:52-60), and
(a3) a light chain CDR3 shown in Table D (and thus a light chain CDR3 sequence selected from SEQ ID NOs:70-78),
and

(d) a heavy chain variable domain comprising

(b1) a heavy chain CDR1 shown in Table C (and thus a heavy chain CDR1 sequence selected from SEQ ID NOs:25-33),
(b2) a heavy chain CDR2 shown in Table C (and thus a heavy chain CDR2 sequence selected from SEQ ID NOs:43-51), and
(b3) a heavy chain CDR3 shown in Table C (and thus a heavy chain CDR3 sequence selected from SEQ ID NOs:61-69).

More preferably, the antibody of the present invention, or antigen-binding fragment thereof, comprises the six CDRs of 11A2, the six CDRs of 11A5, the six CDRs of 11C10, the six CDRs of 13G6, the six CDRs of 14C8, the six CDRs of 2H8, the six CDRs of 3H8, the six CDRs of 8G5 or the six CDRs of 9E7 (or six CDRs that differ from said six CDRs by not more than a total of three, two, or in particular one amino acid additions, substitutions, and/or deletions from a heavy chain).

In a particularly preferred embodiment, the antibody of the present invention, or antigen-binding fragment thereof, comprises the six CDRs of 13G6. Accordingly, the antibody or fragment thereof comprises the heavy chain CDRH1 of SEQ ID NO: 28 (NYAMS), the heavy chain CDRH2 of SEQ ID NO: 46 (YISASGNTYYASWVKG) and the heavy chain CDRH3 of SEQ ID NO: 64 (GYSGWISGTWA), the light chain CDRL1 of SEQ ID NO: 37 (QSSQSVVNNNRLS), the light chain CDRL2 of SEQ ID NO: 55 (RASTLAS) and the light chain CDRL3 of SEQ ID NO: 73 (LGDYVSYSEAA).

In another particularly preferred embodiment, the antibody of the present invention, or antigen-binding fragment thereof, comprises the six CDRs of 11C10. Accordingly, the antibody or fragment thereof comprises the heavy chain CDRH1 of SEQ ID NO: 27 (RNLMS), the heavy chain CDRH2 of SEQ ID NO: 45 (SINFRNITWYASWAKG), the heavy chain CDRH3 of SEQ ID NO: 63 (GVYVNSNGYYSL), the light chain CDRL1 of SEQ ID NO: 36 (QASQSVSNLLA), the light chain CDRL2 of SEQ ID NO: 54 (GASKLES), the light chain CDRL3 of SEQ ID NO: 72 (QTYWGGDGTSYLNP).

In some embodiments, the antibody, or fragment thereof, comprises the six CDRs of 11A2. In some embodiments, the antibody, or fragment of the present invention comprises the six CDRs of 11A5. In some embodiments, the antibody, or fragment of the present invention comprises the six CDRs of 14C8. In some embodiments, the antibody, or fragment of the present invention comprises the six CDRs of 2H8. In some embodiments, the antibody, or fragment of the present invention comprises the six CDRs of 3H8. In some embodiments, the antibody, or fragment of the present invention comprises the six CDRs of 8G5. In some embodiments, the antibody, or fragment of the present invention comprises the six CDRs of 9E7.

It is to be understood that the light chain CDRs shall be encompassed by the light chain variable domain, wherein the heave chain CDRs shall be encompassed by the heavy chain variable domain.

Further, it is envisaged that the antibody, or fragment thereof, of the present invention comprises

(a) a light chain variable domain that is, in increasing order of preference at least 80%, 85%, 90%, 95%, 98%, 99% or 100% identical to the light chain variable domain comprising a sequence as shown in SEQ ID NO: 16, 17, 18, 19, 20, 21, 22, 23 or 24 (see Table B), wherein the light chain variable domain comprises

(a1) a light chain CDR1 that differs by not more than a total of three, two, or in particular one amino acid additions, substitutions, and/or deletions from a light chain CDR1 shown in Table D,
(a2) a light chain CDR2 that differs by not more than a total of three, two, or in particular one amino acid additions, substitutions, and/or deletions from a light chain CDR2 shown in Table D,
(a3) a light chain CDR3 that differs by not more than a total of three, two, or in particular one amino acid additions, substitutions, and/or deletions from a light chain CDR3 shown in Table D, and

(b) and a heavy chain variable domain that is at least 80%, 85%, 90%, 95%, 98%, 99%, or 100% identical to the heavy chain variable domain comprising a sequence shown in SEQ ID NO: 7, 8, 9, 10, 11, 12, 13, 14 or 15 (see Table A), wherein the heavy chain variable domain comprises

(b1) a heavy chain CDR1 that differs by not more than a total of three, two, or in particular one amino acid additions, substitutions, and/or deletions from a heavy chain CDR1 shown in Table C,
(b2) a heavy chain CDR2 that differs by not more than a total of three, two, or in particular one amino acid additions, substitutions, and/or deletions from a heavy chain CDR2 shown in Table C, and
(b3) a heavy chain CDR3 that differs by not more than a total of three, two, or in particular one amino acid additions, substitutions, and/or deletions from a heavy chain CDR3 shown in Table C.

In an embodiment, the antibody, or fragment thereof, of the present invention comprises

(a) a light chain variable domain that is, in increasing order of preference at least 80%, 85%, 90%, 95%, 98%, 99% or 100% identical to the light chain variable domain comprising a sequence as shown in SEQ ID NO: 16, 17, 18, 19, 20, 21, 22, 23 or 24 (see Table B), wherein the light chain variable domain comprises

(a1) a light chain CDR1 shown in Table D,
(a2) a light chain CDR2 shown in Table D, and
(a3) a light chain CDR3 shown in Table D,

(b) and a heavy chain variable domain that is at least 80%, 85%, 90%, 95%, 98%, 99%, or 100% identical to the heavy chain variable domain comprising a sequence shown in SEQ ID NO: 7, 8, 9, 10, 11, 12, 13, 14 or 15 (see Table A), wherein the heavy chain variable domain comprises

(b1) a heavy chain CDR1 shown in Table C,
(b2) a heavy chain CDR2 shown in Table C, and
(b3) a heavy chain CDR3 shown in Table C.

The term "antibody" refers to immunoglobulins or immunoglobulin-like molecules including by way of example and without limitation, IgA, IgD, IgE, IgG and IgM, combinations thereof, and similar molecules produced during an immune response in any vertebrate, for example, in mammals such as goats, rabbits and mice, as well as non-mammalian species, such as shark immunoglobulins. In some embodiments, the antibody may be a rabbit antibody. The term "antibody" includes intact immunoglobulins and "antibody fragments" or "antigen binding fragments" that specifically bind to a molecule of interest. In a preferred embodiment, the antibody is an IgG antibody.

In particular, the term "antibody" refers to a polypeptide ligand comprising at least a light chain and heavy chain immunoglobulin variable region which specifically recognizes and binds an epitope of an antigen. Antibodies are composed of a heavy and a light chain, each of which has a variable region, termed the variable heavy (VH) region and the variable light (VL) region. Together, the VH region and the VL region are responsible for binding the antigen recognized by the antibody. Typically, the antibody of the present invention has heavy (H) chains and light (L) chains interconnected by disulfide bonds. The term "light chain" as used herein includes a full-length light chain and fragments thereof having a sufficient variable region sequence to confer binding specificity. A full-length light chain includes a variable region domain, VL, and a constant region domain, CL. The variable region domain of the light chain is at the amino-terminus of the polypeptide. The term "heavy chain" includes a full-length heavy chain and fragments thereof having sufficient variable region sequence to confer binding specificity. A full-length heavy chain includes a variable region domain, $V_H$, and three constant region domains, $C_H1$, $C_H2$, and $C_H3$, The $V_H$ domain is at the amino-terminus of the polypeptide, and the $C_H$ domains are at the carboxyl-terminus, with the $C_H3$ being closest to the carboxy-terminus of the polypeptide,

There are five main heavy chain classes (or isotypes) which determine the functional activity of an antibody molecule: IgM, IgD, IgG, IgA and IgE. Each heavy and light chain contains a constant region and a variable region (the regions are also known as "domains"). In combination, the heavy and the light chain variable regions specifically bind the antigen. Light and heavy chain variable regions contain a "framework" region interrupted by three hypervariable regions, also called "complementarity-determining regions" or "CDRs". The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a VH CDR3 is located in the variable domain of the heavy chain of the antibody in which it is found, whereas a VL CDR1 is the CDR1 from the variable domain of the light chain of the antibody in which it is found.

The antibody, or fragment thereof, of the present invention may be a single chain antibody, an IgD antibody, an IgE antibody, an IgM antibody, an IgG antibody and fragments thereof. For example, the antibody may be an IgG antibody such as an IgG1 antibody; an IgG2 antibody; an IgG3 antibody; or an IgG4 antibody. In an embodiment, the antibody, or fragment thereof, has been produced recombinantly.

Also encompassed by the present invention are fragments of the monoclonal antibody of the present invention. The fragment shall be an immunologically functional fragment, i.e. an antigen-binding fragment. Accordingly, the fragment of the monoclonal antibody of the present invention shall be capable of the binding one or more BMP10-type peptides Accordingly, the term "immunologically functional fragment" of an antibody, as used herein, refers to a portion of an antibody that lacks at least some of the amino acids present in a full-length chain but which is capable of specifically binding to one or more BMP10-type peptides. Immunologically functional immunoglobulin fragments include Fab, Fab', $F(ab')_2$, and Fv fragments. How to produce antigen-binding fragments is well-known in the art. For example, the fragments can be produced by enzymatic cleavage of an antibody of the present invention. In addition, the fragments can be generated by synthetic or recombinant techniques. Fab fragments are preferably generated by papain digestion of an antibody, Fab' fragments by pepsin digestion and partial reduction, $F(ab')_2$ fragments by pepsin digestion. Fv fragments are preferably produced by molecular biology techniques.

In an embodiment, the fragment of the antibody of the present invention is a $F(ab')_2$ fragment. In another embodiment, the fragment of the antibody of the present invention is a $F(ab')_2$ fragment. In another embodiment, the fragment of the antibody of the present invention is a Fab fragment. In another embodiment, the fragment of the antibody of the present invention is a Fv fragment.

The fragment of an antibody may also be a diabody. Diabodies are small antibody fragments with two antigen-binding sites. Diabodies preferably comprise a heavy chain variable domain connected to a light chain variable domain in the same polypeptide chain.

The antibody of the present invention is preferably a monoclonal antibody. As used herein, the term "monoclonal antibody" refers to an antibody produced by a single clone of B-lymphocytes or by a cell into which the light and heavy chain genes of for the antibody have been transfected.

In an embodiment, the antibody of the present invention is an isolated antibody. Thus, the antibody shall be an antibody which has been purified. Purification of an antibody can be achieved by methods well-known in the art.

The monoclonal antibody of the present invention, or fragment thereof (and the agents referred to in the previous section shall be capable of binding to one or more BMP10-type peptides, such as NT-proBMP10. The terms "binding", "specifically binding", "capable of binding" and "capable of specifically binding" are, preferably, used interchangeably herein. The term "specific binding" or "specifically bind" refers to a binding reaction wherein binding pair molecules exhibit a binding to each other under conditions where they do not significantly bind to other molecules. The term "specific binding" or "specifically binds", when referring to a protein or peptide as biomarker, preferably refers to a binding reaction wherein a binding agent binds to the corresponding biomarker with an affinity ("association constant" $K_a$) of at least $10^7$ M$^{-1}$. The term "specific binding" or "specifically binds" preferably refers to an affinity of at least $10^8$ M$^{-1}$ or even more preferred of at least $10^9$ M$^{-1}$ for its target molecule. The term "specific" or "specifically" is used to indicate that other molecules present in the sample do not significantly bind to the binding agent specific for the target molecule. However, as explained herein above, the agents are referred to herein shall be not only able to bind to NT-proBMP10, but also to preproBMP10 and proBMP10. $K_D$ is the dissociation constant which can be determined with a binding assay, such as surface plasmon resonance techniques (BIAcore®, GE-Healthcare Uppsala, Sweden). Preferably, the antibody (or antigen binding fragment thereof) that binds to one or more BMP10-type peptides, in some embodiments, has a $K_D$ value for the one or more BMP10-type pep-tides (in particular human NT-proBMP10) in the 1-digit nanomolar range or sub-nanomolar range at 37°C.

Another means to describe the kinetic binding properties of an antibody to its antigen is the resolution of the dissociation equilibrium constant into its kinetic rate contributions, as there are the association rate $k_a$ constant and the dissociation rate constant $k_d$. The association rate $k_a$ constant characterizes the velocity of the antibody/antigen complex formation and is time and concentration dependent. In some embodiments, the antibody of the present invention (or antigen binding fragment thereof) that binds to one or more BMP10-type peptides as referred to herein (in particular human NT-proBMP10) has a $k_a$ value (for its antigen) of more than 1.0 E+05 M-1s-1 at 37°C.

The dissociation rate constant ($k_d$) indicates the dissociation rate of an antibody from its antigen. Thus, the dissociation rate constant indicates the probability that the complex will fall apart in a unit of time. The lower the dissociation rate constant, the more tightly bound the antibody is to its antigen. In some embodiments, the antibody of the present invention (or antigen binding fragment thereof) that binds to one or more BMP10-type peptides as referred to herein (in particular human NT-proBMP10) has a $k_d$ value of less than 1.1 E-03 s-1 at 37°C.

In some embodiments, the $K_D$ value, the $k_d$ value and the $k_a$ value are determined as in the Examples section.

The term "epitope" denotes a protein determinant capable of specifically binding to an antibody. Thus, the term preferably refers to the portion of the polypeptide as referred to herein capable of being specifically bound by the antibody of the present invention (or the fragment thereof). Epitopes usually consist of chemically active surface groupings of molecules such as amino acids and usually epitopes have specific three-dimensional structural characteristics, as well as specific charge characteristics. Conformational and nonconformational epitopes are distinguished in that the binding to the former but not the latter is lost in the presence of denaturing solvents.

The antibody of the present invention, or antigen-binding fragment thereof, is useful in methods relating to the determination of the amount of a BMP10-type peptide as referred to herein. For example, the antibody, or fragment thereof, allows for determining the amount of a BMP10-type peptide a sample for use in diagnostic methods.

Accordingly, the present invention is directed to a method for determining the amount of one or more BMP10-type peptides by using the antibodies, or fragments, of the present invention. Preferred methods, such as sandwich assays, for determining the amount of a biomarker is described in the section with the title "Determination of the amount of a biomarker".

For example, the method determining the amount of one or more BMP10-type peptides may comprise the steps of contacting a sample containing one or more BMP10-type pep-tides with at least one agent that binds within amino acid region 22 to 316 of the polypeptide shown in SEQ ID NO: 1, thereby allowing the formation of a complex of the BMP10-type peptide and the at least one agent, and determining the amount of the complex formed.

Further, the antibody, or fragment, thereof may comprise a label, i.e. it may be coupled covalently or non-covalently to a label allowing detection and measurement of the bound agent. Preferred labels are disclosed herein above. For example, the label may be biotin, a radioactive label, a fluorescent label, a chemiluminescent label, an electrochemiluminescent label, a gold label, or a magnetic label as described above in more detail.

In an embodiment, the antibody, or fragment thereof (such as a F(ab')2 fragment), is biotinylated. In another embodiment, the antibody, or fragment thereof, is ruthenylated (as described above).

The present invention is further directed to the *in vitro* use of the antibody, or fragment thereof, of the present invention for the determining the amount of one or more BMP10-type peptides in a sample.

The present invention also relates to a kit comprising at least one monoclonal antibody or fragment thereof, of the invention. The term kit has been defined above.

In addition, antibody pairs were identified which allow for the improved detection of BMP10-type peptides in sandwich assays (Example 13). Specifically, biotinylated and ruthenylated clones 2H8, 3H8, 8G5, 9E7 11A5, 11C10, 14C8, 13G6 were tested in multiple sandwiches for identification of sandwich partners. If was found that combinations of clones 13G6 and 11C10 show a high a grade of performance independent of the orientation. A similar observation was made for the combination of 3H8 and 9E7. Other combinations achieved good ratios. Although in only one direction, other combinations achieved good ratios, too.

Accordingly, the present invention relates to a kit comprising a first antibody, or antigen-binding fragment thereof, which binds one or more BMP10-type peptides and a second antibody, or antigen-binding fragment thereof, which binds one or more BMP10-type pep-tides, preferably, wherein said first and second antibody bind to different epitopes, wherein the first antibody is selected from 2H8, 3H8, 8G5, 9E7 11A5, 11C10, 14C8, 13G6 (or a variant thereof, and wherein the second antibody is selected from 2H8, 3H8, 8G5, 9E7 11A5, 11C10, 14C8, 13G6 (or a variant thereof).

Both antibodies, or fragments thereof, may be labeled. For example, the first antibody, or fragment, thereof may be ruthenylated and the second antibody, or fragment thereof, may be biotinylated (and vice versa).

In some embodiments, the first antibody is 2H8. In some embodiments, the first antibody is 3H8. In some embodiments, the first antibody is 8G5. In some embodiments, the first antibody is 9E7.In some embodiments, the first antibody is 11A5. In some embodiments, the first antibody is 11C10. In some embodiments, the first antibody is 14C8. In some embodiments, the first antibody is 13G6.

Preferred combinations of antibodies are disclosed in Tables 13 and 14 of Example 14.

For example, the first antibody may be 11C10 and the second antibody may be 13G6 (and vice versa).

For example, the first antibody may be 3H8 and the second antibody may be 9E7 (and vice versa).

In an embodiment, the kit comprises a biotinylated first monoclonal antibody that specifically binds one or more BMP10-type peptides, or fragment thereof, wherein the first antibody is 11C10 (or a variant thereof), and a ruthenylated second monoclonal antibody, or fragment thereof, wherein the second antibody is 13G6 (or a variant thereof).

In an embodiment, the kit comprises a biotinylated first monoclonal antibody that specifically binds one or more BMP10-type peptides, or fragment thereof, wherein the first antibody is 13G6 (or a variant thereof), and a ruthenylated second monoclonal antibody, or fragment thereof, wherein the second antibody is 11C10 (or a variant thereof).

The present invention also relates to a host cell producing the antibody of the present invention, or the antigen-binding fragment thereof. In a preferred embodiment, the host producing the antibody of the present invention is a hybridoma cell. Moreover, the host cell may be any kind of cellular system which can be engineered to generate the antibodies according to the current invention. For example, the host cell may be an animal cell, in particular a mammalian cell, such as a HEK cell. In one embodiment HEK293 (human embryonal kidney cells) such as HEK 293-F cells as used in the Examples section, or CHO (Chinese hamster ovary) cells are used as host cells. In another embodiment, the host cell is a non-human animal or mammalian cell.

The host cell preferably comprises at least one polynucleotide encoding for the antibody of the present invention, or fragment thereof. For example, the host cell comprises at least one polynucleotide encoding for the light chain of the antibody of the present invention and at least one polynucleotide encoding the heavy chain of the antibody of the present invention. Said polynucleotide(s) shall be operably linked to a suitable promoter.

The figures show:

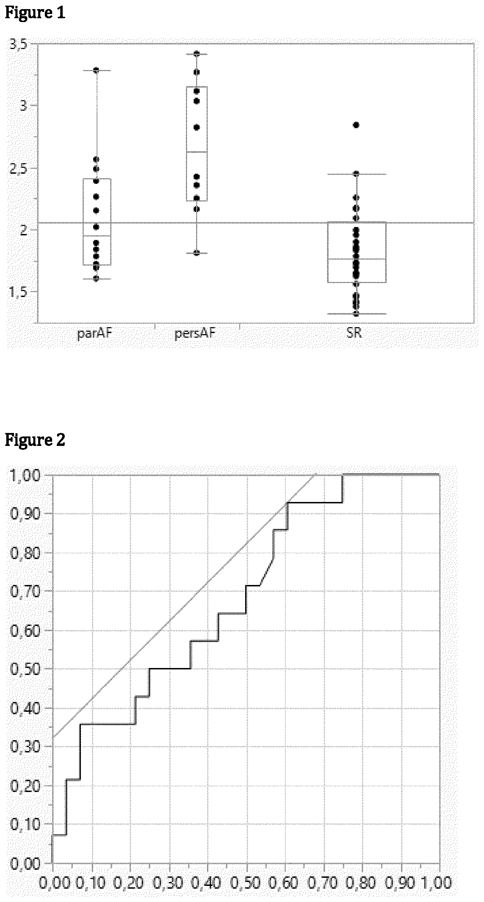

**Figure 1:** Measurement of BMP10 ELISA in three patient groups (paroxysmal atrial fibrillation, persistent atrial fibrillation and patients in sinus rhythm)

**Figure 2:** ROC curve for BMP10 in paroxysmal Afib; AUC = 0.68

**Figure 3:** ROC curve for BMP10 in persistent Afib; AUC = 0.90 (Exploratory AFib panel: Patients with a history of atrial fibrillation covering 14 cases of paroxysmal AFib, 16 cases of persistent Afib and 30 Controls)

**Figure 4:** BMP10 in differentiation of patients with Heart Failure and patients without heart failure [unit: ng/ml]

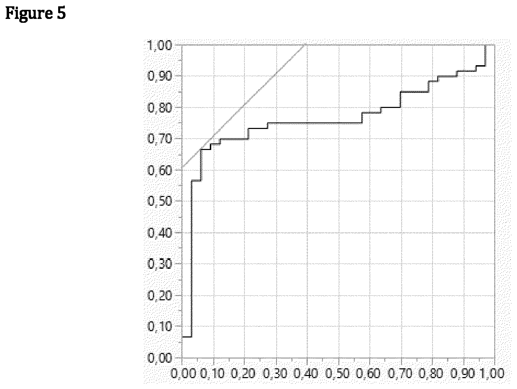

**Figure 5.** BMP10 in differentiation of Heart Failure; ROC curve for BMP10; AUC = 0.76

**Figure 6.** Kaplan-Meier curve showing the risk for a HF hospitalization by quartiles of BMP-10 in patients with a prior history of heart failure.

**Figure 7.** Kaplan-Meier curve showing the risk for a HF hospitalization by quartiles of BMP-10 in patients without a prior history of heart failure.

**Figure 8.** Kaplan-Meier curve showing the risk for a stroke by dichotomized of BMP-10 (at median).

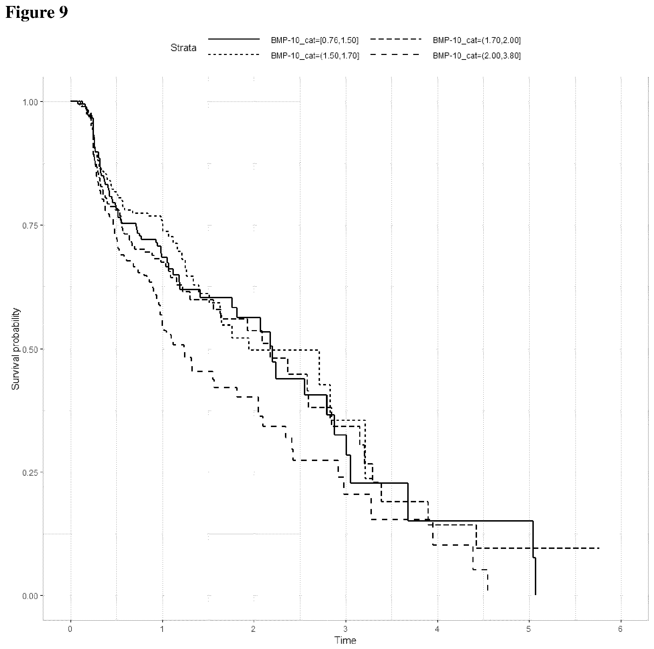

**Figure 9.** Kaplan-Meier curve showing the risk for recurrence of AFib by quartiles of BMP-10 in patients undergoing pulmonary vein isolation (BEAT-PVI)

**Figure 10.** Kinetic signatures for pro-peptide BMP10-binding to a selection of 8 mAbs at 37°C obtained via SPR. Shown are the sensorgram overlays for increasing pro-peptide BMP10 concentrations, ranging between c = 3.7-300 nM;

| A) | 2H8 | B) | 3H8 | C) | 8G5 |
|---|---|---|---|---|---|
| D) | 9E7 | E) | 11A5 | F) | 11C10 |
| G) | 14C8 | H) | 13G6 | | |

**Figure 11.** Overlay of the normalized dissociation phase for 8 mAbs binding pro-peptide BMP10 at 37°C

**Figure 12.** Epitopes of four monoclonal antibodies of the present invention

**Figure 13:** Detection of NT-proBMP10 versus detection of mature BMP10

**Figure 14:** Measurement of circulating BMP-10 in EDTA plasma samples of the SWISS AF study with Fazekas Score < 2 (no) vs Fazekas Score ≥ 2 (yes): Detection of WMI,s/Prediction of the risk of silent stroke: Circulating BMP-10 levels were assessed.

EXAMPLES

The invention will be merely illustrated by the following Examples. The said Examples shall, whatsoever, not be construed in a manner limiting the scope of the invention.

**Example 1: Mapping trial - Diagnose patients with atrial fibrillation as compared to patients based on their different circulating NT-proBMP10 levels**

The MAPPING study related to patients undergoing open chest surgery. Samples were obtained before anesthesia and surgery. Patients were electrophysiologically characterized using high-density epicardial mapping with multi-electrode arrays (high density mapping). The trial comprised 14 patients with paroxysmal atrial fibrillation, 10 patients with persistent atrial fibrillation and 28 controls, matched to best possible (on age, gender, comorbidities). NT-proBMP10 was determined in serum samples of the MAPPING study. Elevated NT-proBMP10 levels were observed in patients with atrial fibrillation versus controls. NT-proBMP10 levels were elevated in patients with paroxysmal atrial fibrillation versus matched controls, as well as in patients with persistent atrial fibrillation versus controls.

In addition, the biomarker ESM-1 was determined in samples from the MAPPING cohort. Interestingly, it was shown that the combined determination of NT-proBMP10 with ESM-1 allowed for an increase of the AUC to 0.92 for the differentiation between persistent AF vs. SR (sinus rhythm).

In addition, the biomarker FABP-3 was determined in samples from the MAPPING cohort. Interestingly, it was shown that the combined determination of NT-proBMP10 with FABP-3 allowed for an increase of the AUC to 0.73 for the differentiation between paroxysmal AF vs. SR (sinus rhythm).

**Example 2: Heart Failure Panel**

The heart failure panel included 60 patients with chronic heart failure. According to the ESC guidelines criteria, heart failure was diagnosed in patients with typical signs and symptoms and objective evidence of a structural or functional abnormality of the heart at rest. Patients between 18 and 80 years with ischemic or dilated cardiomyopathy or significant valvular disease and who were able to sign the consent form were included into the study. Patients with acute myocardial infarction, pulmonary embolism or stroke in the last 6 months, further with severe pulmonary hypertension and end stage renal disease were excluded. The patients suffered predominantly from heart failure stages NYHA II-IV.

The healthy control cohort included 33 subjects. The healthy status was verified by assessing status of ECG and echocardiography results. Participants with any abnormality were excluded.

Elevated NT-proBMP10 levels were observed in serum samples of patients with heart failure versus controls.

**Example 3: Biomarker measurements**

NT-proBMP10 was measured in a research grade ECLIA assay for Bone Morphogenetic Protein 10 (BMP10); ECLIA Assay from Roche Diagnostics, Germany.

For detection of NT-proBMP10 in human serum and plasma samples an antibody sandwich which specifically binds to the N-terminal prosegment of BMP10 was used. Such antibodies also bind to proBMP 10 and preproBMP10. Thus, the sum of the amounts of the N-terminal prosegment of BMP10, proBMP10 and preproBMP10 was determined. Structural prediction based on findings from other BMP-type proteins as e.g. BMP9 show that BMP 10 remains in a complex with proBMP 10, thus detection of N-term prosegement also reflects the amount of prodomain-bound BMP 10. Moreover, the homodimeric form of BMP10 can be detected, as well as heterodimeric structures, as e.g. the combination with BMP9 or other BMP-type proteins.

**Example 4: The SWISS AF study** - **Risk prediction of heart failure hospitalization**

The data from the SWISS-AF study includes 2387 patients from which 617 have a history of heart failure (HF). BMP-10 was measured in these patients to assess its ability to predict the risk of a hospitalization due to heart failure.

As heart failure hospitalization can occur in patients with a history of heart failure and in patients without a known history heart failure the ability was assessed to predict future heart failure hospitalization was assessed in these groups independently. In total for 233 patients a hospitalization due to HF was recorded during follow-up. 125 of the 233 hospitalization occurred in patients with a prior known HF.

Prediction of HF hospitalization in patients with a known history in HF

Table 1 shows the result of a cox proportional hazard model including in patients with a known history of HF. Dependent variable is the time until HF hospitalization and independent variable are log-2 the transformed NT-proBMP10 values.

As visible by the hazard ratio and the low p-value NT-proBMP10 is able to predict the risk for HF hospitalization significantly in patients with a known history of HF. As NT-proBMP10 values were log-2 transformed before they were entered into the model the hazard ratio can interpreted that risk increase by 3.43 for a patient if the value of NT-proBMP10 doubles

Table 1: Summary of cox proportional hazard model for NT-proBMP10 (log-2 transformed) predicting the risk of HF hospitalization in patients with a known history of HF.

| Hazard Ratio | 95% Confidence Interval | P-Value |
|---|---|---|
| 3.43 | 2.23 - 5.27 | < 0.001 |

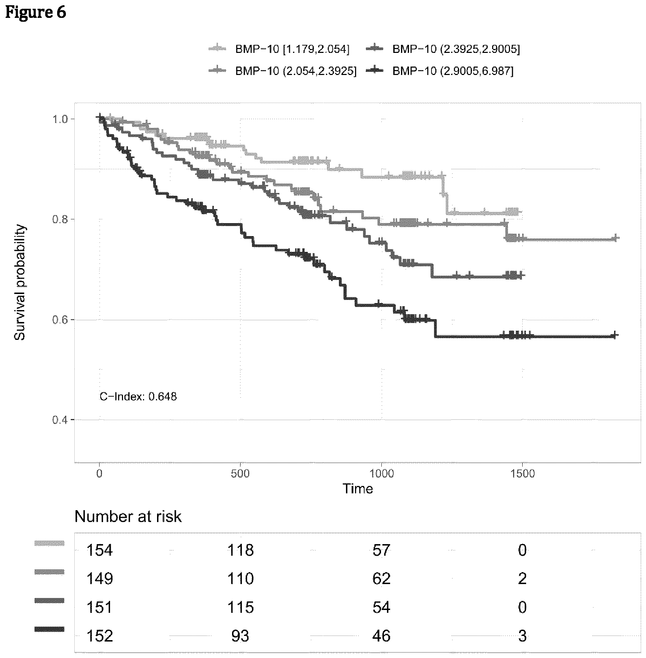

Figure 6 shows a Kaplan-Meier curve which displays the risk of HF hospitalization by quartiles of NT-proBMP10. It is visible that the risk increases constantly with increasing NT-proBMP10 values and the highest risk is observed for patients with NT-proBMP10 levels within the highest quartile.

Prediction of HF hospitalization in patients without a known history in HF

Table 2 shows the result of a cox proportional hazard model including in patients without a known history of HF. Dependent variable is the time until HF hospitalization and independent variable are the log-2 transformed NT-proBMP10 values.

As visible by the hazard ratio and the low p-value NT-proBMP10 is able to predict the risk for HF hospitalization significantly in patients without a known history of HF. As NT-proBMP10 values were log-2 transformed before they were entered into the model the hazard ratio can interpreted that risk increase by 3.43 for a patient if the value of NT-proBMP10 doubles

Table 2: Summary of cox proportional hazard model for NT-proBMP10 (log-2 transformed) predicting the risk of HF hospitalization in patients without a known history of HF.

| Hazard Ratio | 95% Confidence Interval | P-Value |
|---|---|---|
| 4.24 | 2.52 -7.15 | < 0.001 |

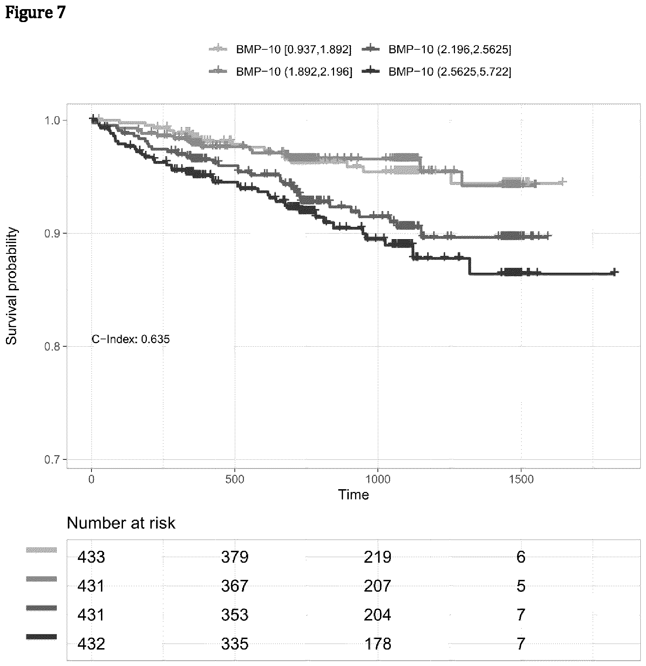

Figure 7 shows a Kaplan-Meier curve which displays the risk of HF hospitalization by quartiles of NT-proBMP10. It is visible that the risk increases with increasing NT-proBMP10 values and the risk is highest for patients with NT-proBMP10 levels within the two highest quartiles.

**Example 5: The SWISS AF study** - **Risk prediction of stroke**

The ability of circulating NT-proBMP10 to predict the risk for the occurrence of stroke was verified (in reference to example 3) in a prospective, multicentric registry of patients with documented atrial fibrillation (Conen D., Swiss Med Wkly. 2017 Jul 10;147:w14467). NT-proBMP10, results were available for 65 patients with an event and 2269 patients without an event.

In order to quantify the univariate prognostic value of NT-proBMP10 proportional hazard models were used with the outcome stroke.

The univariate prognostic performance of NT-proBMP10 was assessed by two different incorporations of the prognostic information given by NT-proBMP10.

The first proportional hazard model included NT-proBMP10 binarized at the median (2.2 ng/mL) and therefore comparing the risk of patients with NT-proBMP10 below or equal to the median versus patient with NT-proBMP10 above the median.

The second proportional hazard model included the original NT-proBMP10 levels but transformed to a log2 scale. The log2 transformation was performed in order to enable a better model calibration.

In order to get estimates for the absolute survival rates in the two groups based on the dichotomized baseline NT-proBMP10 measurement (<=2.2 ng/mL vs > 2.2 ng/mL) a Kaplan-Meier plot was created.

In order to assess if the prognostic value of NT-proBMP10 is independent from known clinical and demographic risk factors a weighted proportional cox model including in addition the variables age, and history of Stroke/TIA/Thromboembolism was calculated. These were the only significant clinical risk predictors on the whole cohort (including all controls).

In order to assess the ability of NT-proBMP10 to improve existing risk scores for the prognosis of stroke the $CHADS_2$ the $CHA_2DS_2$-VASc and the ABC score were extended by NT-proBMP10 (log2 transformed). Extension was done by creating a portioned hazard model including NT-proBMP10 and the respective risk score as independent variables.

The c-indices of the $CHADS_2$, the $CHA_2DS_2$-VASc and ABC score were compared to the c- indices of these extended models.

**Results**

Table 1 shows the results of the two univariate weighted proportional hazard models including the binarized or the log2 transformed NT-proBMP10. The association between the risk for experiencing a stroke with the baseline value of NT-proBMP 10 is not significant in the model using log2-transformed NT -proBMP 1 0 as a risk predictor but close to the significance level of 0.05.

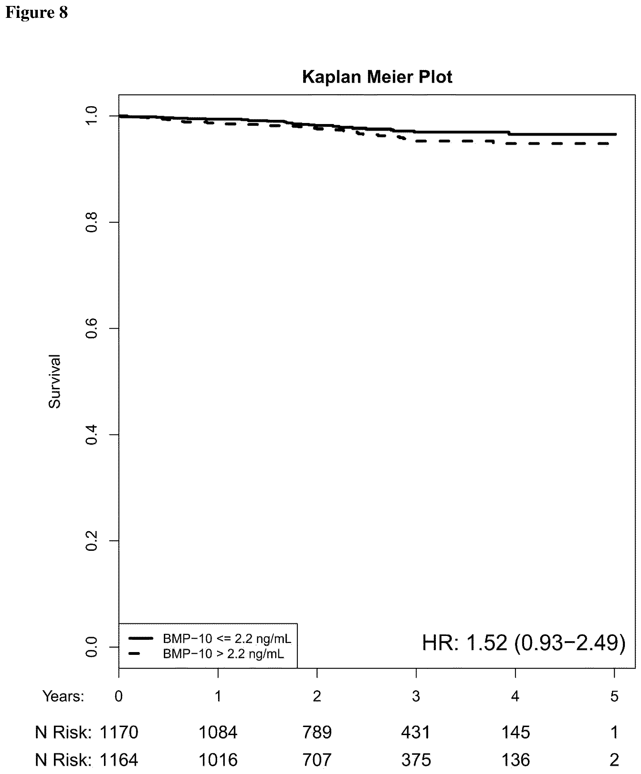

For the model using the binarized NT-proBMP10 the p-value is slighly higher. It could be argued however with a higher number of events the effect could be statistically significant. The hazard ration for the binarized NT-proBMP10 implies a 1.5-fold higher risk for a stroke in the patient group with baseline NT-proBMP10 > 2.2 ng/mL versus the patient group with baseline NT-proBMP10 <= 2.2 ng/mL. This can be seen also in Figure 8 displaying the Kaplan Meier curves for the two groups.

The results of the proportional hazard model including NT-proBMP 10 as log2 transformed linear risk predictor suggest the log2 transformed values NT-proBMP10 are proportional to the risk for experiencing a stroke. The hazard ratio of 2.038 can be interpreted in a way that a 2-fold decrease of NT-proBMP10 is associated with 2.038 increase of risk for a stroke.

Table 1: Results result of the univariate weighted proportional hazard model including the binarized and log2 transformed NT-proBMP10.

|  | Hazard Ratio (HR) | 95%-CI HR | P-Value |
|---|---|---|---|
| NT-proBMP10 log2 | 1.523 | 0.930 - 2.495 | 0.095 |
| Baseline BMP10 <= 2.2 ng/mL vs NT-proBMP10 >2.2 ng/mL | 2.038 | 0.994 4.179 | 0.052 |

Table 2 shows the results of a proportional hazard model including NT-proBMP10 (log2 transformed) in the combination with clinical and demographic variables. It is visible that the prognostic value of NT-proBMP10 diminishes to some extend but this could be partially also being explained by low statistical power of the model.

Table 2: Multivariate proportional hazard model including NT-proBMP10 and relevant clinical and demographic variables.

|  | Hazard Ratio (HR) | 95%-CI HR | P-Value |
|---|---|---|---|
| Age | 1.0615 | 1.0256 - 1.0987 | 0.0007 |
| History Stroke/TIA/embolism | 1.9186 | 1.1451 - 3.2145 | 0.0133 |
| NT-proBMP10 (log2 transformed) | 1.2253 | 0.5545 - 2.7076 | 0.6155 |

Table 3 shows the results of the weighted proportional hazard model combining the $CHADS_2$ score with NT-proBMP10 (log2 transformed). In this model NT-proBMP10 can add prognostic information to the $CHADS_2$ score but with a p-value above 0.05 which can however be tolerated with respect to the low sample size.

Table 3: Weighted proportional hazard model combining the $CHADS_2$ score with NT-proBMP10 (log2 transformed

|  | Hazard Ratio (HR) | 95%-CI HR | P-Value |
|---|---|---|---|
| $CHADS_2$ score | 1.3792 | 1.1590 - 1.6413 | 0.0003 |
| NT-proBMP10 (log2 transformed) | 1.5046 | 0.7094 - 3.1911 | 0.2869 |

Table 4 shows the results of the weighted proportional hazard model combining the $CHA_2DS_2$-VASc score with NT-proBMP10 (log2 transformed). Also in this model NT-proBMP 1 0 can add prognostic information to the $CHA_2DS_2$-VASc score but with a p-value above 0.05 which can however be tolerated with respect to the low sample size.

Table 4: Weighted proportional hazard model combining the $CHA_2DS_2$-VASc score with NT-proBMP10 (log2 transformed)

|  | Hazard Ratio (HR) | 95%-CI HR | P-Value |
|---|---|---|---|
| $CHA_2DS_2$-VASc score | 1.2756 | 1.0992 - 1.4803 | 0.1281 |
| NT-proBMP10 (log2 transformed) | 1.4308 | 0.6645 - 3.0806 | 0.3600 |

Table 5 shows the results of the weighted proportional hazard model combining the ABC score with NT-proBMP10 (log2 transformed). In this model the estimated hazard ratio diminishes and NT-proBMP10 likely can't add any prognostic performance

Table 5: Weighted proportional hazard model combining the ABC score with NT-proBMP 1 0 (log2 transformed)

| | Hazard Ratio (HR) | 95%-CI HR | P-Value |
|---|---|---|---|
| ABC score | 1.1839 | 1.1046 - 1.2688 | < 0.0001 |
| NT-proBMP10 (log2 transformed) | 0.7321 | 0.3123 - 1.7161 | 0.4731 |

Table 6 shows the estimated c-indexes of NT-proBMP10 alone, of the CHADS$_2$, the CHA$_2$DS$_2$-VASc, the ABC score and of the weighted proportional hazard model combining the CHADS$_2$, the CHA$_2$DS$_2$-VASc, the ABC score with NT-proBMP10 (log2) on the case cohort selection. It can be seen that the addition of NT -proBMP 1 0 improves the c-index of the CHADS$_2$, the CHA$_2$DS$_2$-VASc score but not the ABC score.

The differences in c-index are 0.019, 0.015 and -0.002 for the CHADS$_2$, the CHA$_2$DS$_2$-VASc, the ABC score respectively.

Table 6: C-indexes of NT-proBMP10, the CHA$_2$DS$_2$-VASc score and the combination of the CHA$_2$DS$_2$-VASc score with NT-proBMP10 and C-indexes of the CHADS$_2$ and ABC score and their combination with NT-proBMP10.

| | C-Index |
|---|---|
| NT-proBMP10 univariate | 0.577 |
| CHADS$_2$ | 0.629 |
| CHADS$_2$ + NT-proBMP10 | 0.643 |
| CHA$_2$DS$_2$-VASc | 0.616 |
| CHA$_2$DS$_2$-VASc + NT-proBMP10 | 0.627 |
| ABC score | 0.692 |
| ABC score + NT-proBMP10 | 0.690 |

**Example 6: The BEAT-AF-PVI study - prediction of risk of recurrent AFib after pulmonary vein isolation and catheter ablation.**

The ability of NT-proBMP10 to predict the risk of future recurrent atrial fibrillation episodes was assessed in the BEAT-AF-PVI study. The BEAT-AF-PVI study (Knecht S, International Journal of Cardiology, Volume 176, Issue 3, 2014, Pages 645-650) is a prospective cohort study including patients with atrial fibrillation who underwent a pulmonary vein isolation. One of the collected study endpoints was the time until the first recurrence of atrial fibrillation. Thus, the ability of circulating NT -proBMP 1 0 to predict the risk for the reoccurrence of AFib after PVI and catheter ablation was verified in a prospective, multicentric registry of patients with documented atrial fibrillation (Zeljkovic I., Biochem Med. 2019; 29: 020902).

NT-proBMP10 measurements and information about atrial fibrillation recurrence were available in 719 patients. For 310 out of 719 patients an atrial fibrillation recurrence was observed. NT-proBMP10 was measured in a research grade ECLIA assay for Bone Morphogentic Protein 10 (NT-proBMP10), from Roche Diagnostics, Germany.

The ability of NT-proBMP10 to predict the risk of recurrent atrial fibrillation was assessed by a Cox (proportional hazard) regression model. Table 1 shows the results of the proportional hazard model. The results show that with increasing values of NT-proBMP10 the risk of recurrent atrial fibrillation increases significantly. As the NT-proBMP10 was included in the model log2 transformed to improve model calibration one can interpret the hazard ration as 1.91 risk increase if NT-proBMP10 increases 2-fold.

**Table 7: Summary of the Cox regression model predicting the risk of recurrent AFIB by the log2 transformed values of NT-proBMP10.**

| | Hazard Ratio | Hazard Ratio 95% CI | P-Value |
|---|---|---|---|
| NT-proBMP 10 (log2) | 1.91 | 1.24 - 2.93 | 0.003 |

Alternatively NT-proBMP10 can be used also in a binarized (e.g. split at median of 1.7 ng/mL) form for risk prediction of atrial fibrillation. Table 8 indicates that the risk in the patients group with NT-proBMP10 levels above the median is elevated by 32%. This risk difference is again statistically significant.

**Table 8: Summary of the Cox regression model predicting the risk of recurrent AFIB by binarized NT-proBMP10 at the observed median value.**

|  | Hazard Ratio | Hazard Ratio 95% CI | P-Value |
|---|---|---|---|
| NT-proBMP10 > 1.7 ng/mL | 1.32 | 1.05 -2.65 | 0.018 |

**Example 7: Assessment of recurrent AF with circulating NT-proBMP10**

The GISSI AF study relates to patients in sinus rhythm (SR) with a history of atrial fibrillation (AF), but without significant left ventricular dysfunction or heart failure. All patients underwent biochemistry NT-proBMP10 assessment and electrocardiography at 3 times during 1 year follow up.

Circulating NT-proBMP10 levels have been determined in samples of n=281 patients with blood sampled and biomarkers assayed, in SR at 6-month visit and in samples of n=33 patients with blood sampled and biomarkers assayed, with ongoing AF at 6-month visit. Antibodies against pro-peptide BMP10 were used.

**Table 9 Measurement of circulating NT-proBMP10 in GISSI AF patients at 6-month visit in SR and with ongoing AF**

|  | GISSI AF | | |
|---|---|---|---|
|  | SR (n=28 1) | AF (n=33) | p-value |
| NT- | 1,97 | 2,31 | < 0,0001 |
| proBMP10 (median; ng/ mL) | [1,75 - 2,33] | [2,04-2,67] |  |

As shown in table 9, NT-proBMP10 was observed in patients with ongoing AF at sampling versus patients in SR at the time of blood sampling at the 6 - month visit of the GISSI AF study.

It is obvious, that for NT-proBMP10 small, but highly significant delta changes of marker elevations in AF pts vs SR pts could be detected. In the 33 patients with ongoing AF at the time of sampling median NT-proBMP10 values of 2,31 [2,04-2,67] vs 1,97[1,75 - 2,33] ng/mL were observed versus the 281 patients in SR at the time of blood sampling.

Samples of n= 105 patients were in SR at 6-month visit, but had experienced more than one recurrence of AF from randomization to the 6 - month visit. All 105 patients spontaneously converted to SR.

- 0-7 days        n=11 patients
- 8-30 days      n=17 patients
- >30 days       n=77 patients

**Table 10 Measurement of circulating NT-proBMP10 in GISSI AF patients in SR; Case control of patients experiencing recurrent AF several days before sampling**

|  | Days after AF | | |
|---|---|---|---|
|  | 1 - 7 (n=11) | 8 - 30 (n=17) | >30 (n=77) |
| NT-proBMP10 (median; ng/ mL) | 2,30 [1,65 - 2,45] | 1,90 [1,67-2,50] | 1,90 [1,75-2,25] |

As shown in table 10, NT-proBMP10 titers were observed to be elevated for up to 7 days after AF in patients, that spontaneously converted to SR. In the 11 patients with preceding AF up to 7 days before sampling median values of 2,30 [1,65 - 2,45] versus 1,90 [1,75-2,25] ng/mL were observed versus the 77 patients with preceding AF more than 30 days before blood sampling. It is striking, that the very same median values for NT-proBMP10 were observed up to 7 days after preceding AF before sampling as in patients with ongoing AF. In the 11 patients in SR after AF up to 7 days ago at the time of sampling median NT-proBMP10 values of 2,30 [1,65 - 2,45] ng/mL were observed. As shown in table 9 in the 33 patients with ongoing AF at the time of in SR at the time of blood sampling median NT-proBMP-10 values of 2,31 [2,04 - 2,67] ng/ml were observed.

Data evaluation showed that patients with NT-proBMP10 levels (independent from other biomarkers) above a reference value (in the study > 2,0 ng/mL) are suspicious to have recurrent atrial fibrillation after therapeutic interventions, e.g. after cardioversion. The differentiation of poor and good therapy responders supports decision making, which patient may not profit from a therapy in order to avoid costly therapies and associated burden, but poor outcome to the patient.

It is even shown, that elevated NT-proBMP10 levels may be detected in patients presenting in sinus rhythm up to 7 days after a preceding AF episode.

In summary, the diagnosis of paroxysmal atrial fibrillation in patients presenting within 7 days later in sinus rhythm may be achieved with detection of enhanced levels of NT-proBMP10 alone or in combination with a marker of cardiac injury (eg. cTNThs) and/or a marker of heart failure (NT-ProBNP).

**Example 8:** Detection of NT-proBMP10 versus detection of mature BMP10

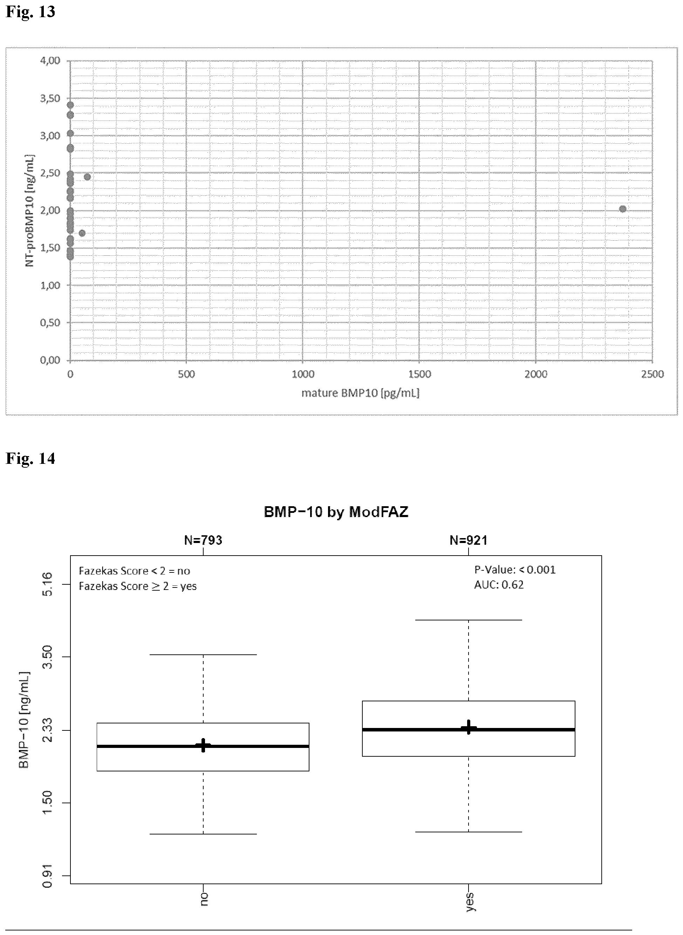

For comparison of detecting mature BMP10 and NT-proBMP10 the Elecsys prototype detection method as described in example 14 was measured in a head to head analysis with BMP-10 ELISA (R&Dsystems DuoSet DY2926-05) detecting mature BMP-10 homodimer (aa 317-424). Samples that were measured are deriving from the mapping cohort as described in example 1. Diagnosed patients with atrial fibrillation as compared to patients based on their different circulating BMP10 levels applying novel method for detection of NT-proBMP10 in comparison to commercial immunoassays for detection of mature BMP10 from R&Dsystems. Serum samples from patients were electrophysiologically characterized using high-density epicardial mapping with multi-electrode arrays (MAPPING study) (Figure 13, or Table below).

| sample | Mature BMP10 [pg/ml] | Diagnosis |
|---|---|---|
| Patient 1 | 52.4 | Sinus rhythm |
| Patient 2 | 605 | Sinus rhythm |
| Patient 3 | 552 | Sinus rhythm |
| Patient 4 | 217 | Persistent AF |
| Patient 5 | 2374 | Sinus rhythm |
| Patient 6 | 74.5 | Paroxysmal AF |

For only six out of 52 samples mature BMP10 levels could be detected. These are reflecting 4 in sinus rhythm, 1 paroxysmal and 1 persistent AF patient. Whereas NT-proBMP10 levels could be detected for all samples as described in example 1.

This finding of only 11.5 % of samples having detectable levels of mature BMP10 suggests that physiologically the mature form is underrepresented in circulation due to internalization upon receptor binding. Thus, NT-proBMP10 is circulating in a more stable form and higher detectable concentration levels. Allowing clinical decision making based on circulating NT -proBMP 1 0 levels.

**Example 9: Immunization of rabbits for generation of antibodies against BMP-10**

Here we describe the development antibodies with the ability to bind Bone Morphogenetic Protein-10 (NT-proBMP10). For the generation of such antibodies, we immunized 12-16-weeks old NZW rabbits with rec. NT-proBMP10, a polypeptide comprising the first 312 amino acids of preproBMP10. All rabbits were subjected to repeated immunizations. In the first month the animals were immunized weekly. From the second month onward the animals were immunized once per month. For the first immunization, we dissolved 500 µg of the immunogen in 1 mL 140 mM NaCl and we emulsified the solution in 1 ml CFA. For all following immunizations, CFA was replaced by IF A.

**Example 10: Development of antibodies binding NT-proBMP10**

For the development of antibodies binding to BMP-10, B-cell cloning as described in Seeber et al. (2014), PLoS One. 2014 Feb 4;9(2) was used. Firstly, the PBMC pool of cells was prepared from whole blood of the immunized animals by ficoll gradient centrifugation. For the enrichment of antigen reactive B-cells from the PBMC pool randomly biotinylated NT-proBMP10 was immobilized on streptavidin coated magnetic beads (Miltenyi). For the coating of the beads the protein was used at a concentration of 1 µg/ml. Therefore, we incubated the prepared PBMC pool of the immunized animals with the NT-proBMP10-coated beads for 1h. For the enrichment of antigen-reactive B-cells MACS columns (Miltenyi) were used. B-cell sorting and incubation was done as described in Seeber et al. (2014), PLoS One. 2014 Feb 4;9(2). To identify NT-proBMP10 reactive clones by ELISA we immobilized NT-proBMP10 on the surface of 96well plates and the concentration used for immobilization was 250 ng/ml. After washing the plates were blocked with 5% BSA to reduce background signals. The plates were washed again and 30 µl of the primary rabbit B-cell supernatant were transferred to the 96 well plates and incubated for 1h at room temperature. For the detection of antibodies bound to the screening peptides, HRP-labeled F(ab')2 goat anti-rabbit Fcγ (Dianova) and ABTS (Roche) as a substrate were added. Clones binding to plate bound NT-proBMP10 were selected for subsequent molecular cloning as described in Seeber et al. (2014), PLoS One. 2014 Feb 4;9(2).

**Example 11: Kinetic Screening**

Information antigen: Pro-peptide BMP10 (R&D-Systems) and in-house construct "312" (pre-pro-peptide BMP10), both represent the prodomain of BMP10 ± 19 aa N-terminal leader peptide. Recombinant human BMP-10, R&D-Systems, Cat.No 2926-BP/CF, Lot: Qual0518031, disulfide-linked homodimer, MW 24.40 kDa.

**Kinetic Screening**

The kinetic screening was performed at 37°C on a GE Healthcare Biacore 4000 instrument. A Biacore CM5 Series S sensor was mounted to the instrument, hydrodynamical addressed and preconditioned according to the manufacturer's instructions. The system buffer was HBS-EP (10 mM HEPES, 150 mM NaCl, 1 mM EDTA, 0.05 % (w/v) P20). The system buffer supplemented with 1 mg/mL CMD (Carboxymethyldextran, Fluka) was used as sample buffer.

A rabbit antibody capture system was immobilized on the sensor surface. A polyclonal goat anti-rabbit IgG Fc capture antibody GARbFcγ (Code-No. 111-005-046; Jackson Immuno Research) was amine coupled using the EDC/NHS-chemistry according to the to the manufacturer's instructions.

30 μg/mL GARbFcγ in 10 mM sodium acetate buffer (pH 4.5) were preconcentrated to the spots 1, 2, 4 and 5 in the flow cells 1, 2, 3 and 4 and covalently bound to the CMD-surface with densities of approximately 10000 RU. Free activated carboxyl groups were subsequently saturated with 1 M ethanolamine pH 8.5.

The spots 1 and 5 were used for the interaction measurements and spots 2 and 4 served as references. Each rabbit antibody supernatant suspension was diluted 1:5 in sample buffer and was injected at a flow rate of 10 μL/min for 2 minutes. The rabbit antibody Capture Level (CL) in resonance units RU was monitored.

Construct "312" was injected with a single concentration c = 150 nM to the respective surface displayed anti-pro-peptide BMP10 rabbit mAb at 30 μL/min. The association and dissociation phase was monitored for 5 minutes each. After each cycle of kinetics determination the rabbit clones were completely washed from the sensor surface by an injection of 10 mM Glycine pH 1.5 at 20 μL/min for 30 seconds. Report points Binding Late (BL), shortly before the end of the pro-peptide BMP 10 injection and Stability Late (SL), shortly before the end of the dissociation were extracted from obtained sensorgram. They are used to characterize the antibody/antigen binding stability. Furthermore, the dissociation rate constant $k_d[s^{-1}]$ was calculated according to a Langmuir 1:1 model. The antigen/antibody complex stability halftime (minutes) was calculated according to the formula $\ln(2)/60*k_d$. The Molar Ratio, representing the binding stoichiometry, was calculated with the formula:

$$\text{MW (antibody)} / \text{MW (antigen)} * \text{BL (antigen)} / \text{CL (antibody)}$$

280 rabbit antibodies were tested using this approach. 18 Abs were identified with suitable kinetic properties meeting the criteria for the Elecsys-platform.

**Kinetic characterization**

Detailed kinetic investigations were performed using the BIAcore 8K instrument from GE Healthcare. The rabbit mAbs< propeptid BMP10> clones 2H8, 3H8, 8G5, 9F7, 11A5, 11C10, 14C8 and 13G6 - identified via the kinetic screening- were kinetically characterized in detail for binding pro-peptide BMP10 at 37°C.

A Biacore CM5 Series S sensor (Lot #10281824 / 10276998) was mounted to the instrument.

Amine coupling of capture molecules

A rabbit antibody capture system was immobilized on the sensor surface. A polyclonal goat anti-rabbit IgG Fc capture antibody GARbFcy (Code-No. 111-005-046, Lot #131053; Jackson Immuno Research) was amine coupled using the EDC/NHS-chemistry according to the manufacturer's instructions: running buffer: HBS-N buffer (10 mM HEPES, 150 mM NaCl,pH 7.4), activation by mixture of EDC/NHS, the capture-Ab was diluted in coupling buffer NaAc, pH 5.0, c = 30 μg/mL; finally remaining activated carboxyl groups were blocked by injection of 1 M Ethanolamime pH 8.5; Ab densities reached between 11200 - 12700 RU

Kinetic characterization for pro-peptide BMP10 binding to a selection of mAbs at 37°C

The system and sample buffer was HBS-EP (10 mM HEPES, 150 mM NaCl, 1 mM EDTA, 0.05 % (w/v) P20, pH 7.4).

Flow cells 2 of channels 1, 2, 3, 4, 5, 6, 7 and 8 were used for the interaction measurements and flow cells 1 of each channel served as references. Each rabbit antibody was diluted to 3 nM in sample buffer and was injected at a flow rate of 5 $\mu$L/min for 2 minutes. The rabbit antibody Capture Level (CL) in resonance units RU was monitored.

A series of increasing concentrations c = 3.7-300 nM pro-peptide BMP10 "BMP10(312)" was injected to the respective surface displayed anti-pro-peptide BMP10 rabbit mAb at 60 $\mu$L/min with replicate for the concentration c = 33.3 nM. The association phase was monitored for 3 minutes; dissociation phase was monitored for 10 minutes. After each cycle of kinetics determination, the rabbit clones were eluted from the capture system by an injection of 10 mM Glycine pH 2.0 for 1 minute, followed by two consecutive injections of 10 mM Glycine pH 2.25 at 20 $\mu$L/min for 1 minute.

The dissociation rate constant $k_d$ was evaluated using a Langmuir 1:1 fit model according to the BIAcore™ evaluation software Insight SW V 2.0 from GE Healthcare. The antigen/antibody complex stability half-life time (minutes) was calculated according to the formula $\ln(2)/60 \ast k_d$.

The Molar Ratio, representing the binding stoichiometry, was calculated with the formula:

$$MW \ (antibody) \ / \ MW \ (antigen) \ast BL \ (antigen) \ / \ CL \ (antibody)$$

Since the pro-peptide BMP10 is a dimeric molecule, the obtained affinities are avidity-burdened, therefore, representing apparent data.

**Results**

Kinetic screening

280 rabbit antibodies were tested using the kinetic screening approach. 18 Abs with suitable kinetic properties were identified and further characterized.

Detailed kinetic characterization

From 280 kinetically screened rabbit monoclonal antibodies 18 antibodies were selected.

Detailed concentration-dependent kinetic investigations showed, that the pro-peptide BMP 10 interaction does not behave according to a Langmuir 1:1 interaction.

The pro-peptide BMP10 is a dimeric molecule, the interactions are probably avidity burdened. The kinetic data represents apparent data, but can be characterized by visual inspection of the sensorgrams and by a quantification of the antibody linear dissociation phases. The complex half-lifes vary between t/2 diss = 6 and > 115 minutes. The binding stoichiometries are between 1.2 to 1.4 and indicate a 2:1 binding stoichiometry.

All Abs with exception of 14C8 and 8G5 show suitable kinetic profiles: comparable fast complex formation velocity and complex half-lifes t/2 diss > 15 minutes. The Molar Ratios for all Abs indicate a 2:1 binding stoichiometry. Clone 8G5 and 14C8 show a slightly slower association and less complex stability than other clones. Both cover the same epitope region. Clones 2H8 and 3H8, each covers a unique epitope region.

Clone 13G6 shows the slowest dissociation ($k_d$ < 1.0E-04 s$^{-1}$), resulting in the complex-half-life t/2-diss > 115 minutes. Clone 11A5 shows a suitable kinetic signature with fast complex formation velocity and a complex half-life of 30 minutes. The Molar Ratio indicates fully functional Ab with a 2:1 binding stoichiometry.

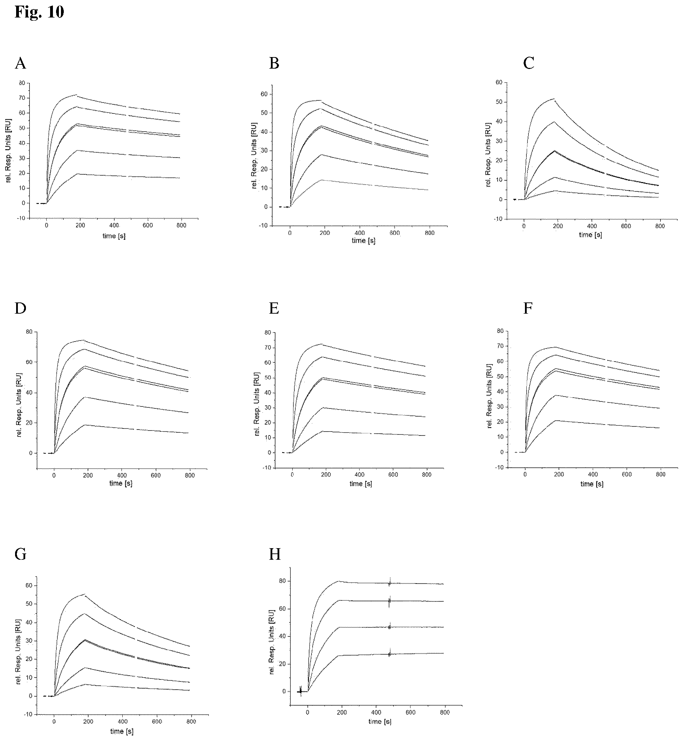

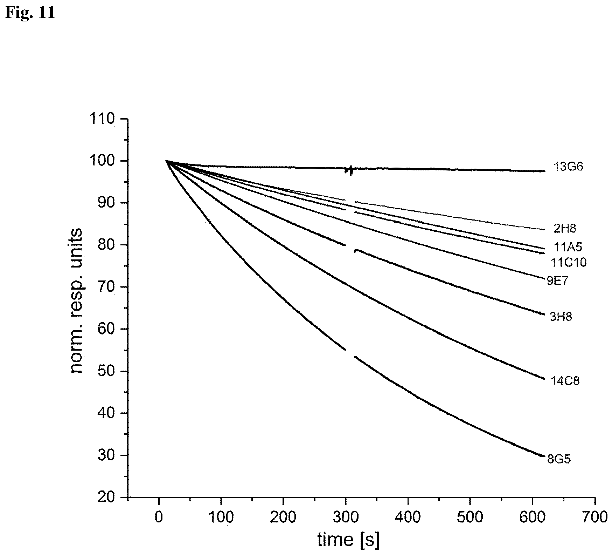

Results are summarized in the Table 11, Sensorgram overlays are shown in Figure 10. The normalized antibody dissociation phases for the 8 clones are shown in Figure 11

**Table 11:** Kinetic constants and affinity data for the pro-peptide BMP10-binding to a selection of mAbs<pro-peptide BMP10> measured by SPR (BIACORE® 8K) at 37 °C

| mAb | CL [RU] | $k_d$ [s$^{-1}$] | t/2 diss [min] | R$_{max}$ [RU] | MR [ ] |
|---|---|---|---|---|---|
| 2H8 | 208 | 3.0E-04 ± < 0.1 % | 38 | 65 | 1.2 |
| 3H8 | 162 | 7.8E-04 ± < 0.1 % | 15 | 53 | 1.3 |

(continued)

| mAb | CL [RU] | $k_d$ [s$^{-1}$] | t/2 diss [min] | R$_{max}$ [RU] | MR [ ] |
|---|---|---|---|---|---|
| 8G5 | 164 | 2.1E-03 $\pm$ < 0.1 % | 6 | 52 | 1.2 |
| 9E7 | 189 | 5.4E-04 $\pm$ < 0.1 % | 21 | 70 | 1.4 |
| 11A5 | 187 | 3.9E-04 $\pm$ < 0.1 % | 30 | 68 | 1.4 |
| 11C10 | 187 | 4.5E-04 $\pm$ < 0.1 % | 26 | 64 | 1.3 |
| 14C8 | 155 | 1.2E-03 $\pm$ < 0.1 % | 10 | 53 | 1.3 |
| 13G6 | 219 | <1.0E-04 $\pm$ 1.1 % | >115 | 74 | 1.3 |

$k_d$ dissociation rate constant [s$^{-1}$]
t$_{/2\text{-diss}}$ antigen/antibody complex stability half-life [min], calculated according to the formula ln(2)/60*$k_d$
R$_{max}$ maximum analyte response [RU]
MR Molar Ratio: Ratio R$_{max}$ experimental vs. theoretical, bound analyte per mAb

**Example 12:** Epitope Mapping using Peptide Microarrays

The epitope mapping of antibody clones was carried out by means of a library of overlapping immobilized peptide fragments (length: 15 amino acids, 14 amino acids overlap) corresponding to the sequence of human bone morphogenetic protein 10. Peptides were synthesized with an automated synthesizer (Intavis MultiPep RS) on modified cellulose disks which were dissolved after synthesis. The solutions of the individual peptides were then spotted onto coated microscope slides. The synthesis was carried out stepwise utilizing 9-fluorenylmethoxycarbonyl (Fmoc) chemistry on amino-modified cellulose disks in a 384-well synthesis plate. In each coupling cycle, the corresponding amino acids were activated with a solution of DIC/HOBt in DMF. Between coupling steps, un-reacted amino groups were capped with a mixture of acetic anhydride, diisopropylethyl amine and 1-hydroxybenzotriazole. Upon completion of the synthesis, the cellulose disks were transferred to a 96-well plate and treated with a mixture of trifluoroacetic acid (TFA), dichloromethane, triisoproylsilane (TIS) and water for side chain deprotection. After removal of the cleavage solution, the cellulose bound peptides were dissolved with a mixture of TFA, TFMSA, TIS and water, precipitated with diisopropyl ether and re-suspended in DMSO. These peptide solutions were subsequently spotted onto Intavis CelluSpots™ slides using an Intavis slide spotting robot.

For epitope analysis, the prepared slides were washed with ethanol and then Tris-buffered saline (TBS; 50 mM Tris, 137 mM NaCl, 2.7 mM KCl, pH 8) before a blocking step was carried out for 1 h at 37 °C with 5 mL 10x Western Blocking Reagent (Roche Applied Science), 2.5 g sucrose in TBS, 0.1% Tween 20. After washing (TBS + 0.1% Tween 20), the slides were incubated with a solution (1 μg/mL) of antibody clones in TBS + 0.1% Tween 20 at 37 °C 1 h. After washing, the slides were incubated for detection with an anti-rabbit secondary HRP-antibody (1:20000 in TBS-T) followed by incubation with DAB substrate. Positive SPOTs were assigned to the corresponding peptide sequences.

**Table 12: Epitopes**

| Analyzed Antibodies | Position in the protein (UniProtKB O95393, SEQ ID NO: 1 | Detected Epitope |
|---|---|---|
| MAB<BMP10>rRb-3H8 HC3/LC3 (p10qx/p10qy)-IgG(SPA) | 37 to 47 | S-L-F-G-D-V-F-S-E-Q-D (SEQ ID NO: 2) |
| MAB<BMP10>rRb-8G5 HC3/LC5 (p10qz/p10ra)-IgG(SPA) | - | no linear epitope detected |
| MAB<BMP10>rRb-9E7 HC12/LC7 (p10rw/p10rx)-IgG(SPA) | | no linear epitope detected |
| MAB<BMP10>rRb-11A5 HC6/LC3.2 (p10se/p10sf)-IgG(SPA) | 171 to 185 | L-E-S-K-G-D-N-EG-E-R-N-M-L-V (SEQ ID NO: 3 |

(continued)

| Analyzed Antibodies | Position in the protein (UniProtKB O95393, SEQ ID NO: 1) | Detected Epitope |
|---|---|---|
| MAB<BMP10>rRb-2H8 HC7/LC6 (p10qv/p10qw)-IgG(SPA) | 291 to 299 | S-S-G-P-G-E-E-A-L (SEQ ID NO: 5) |
| MAB<BMP10>rRb-14C8 HC2/LC3 (p10th/p10ti)-IgG(SPA) | | no linear epitope detected |
| MAB<BMP10>rRb-13G6 (p10tf/p10tg)-IgG(SPA) | 173 to 181 | S-K-G-D-N-E-G-E-R (SEQ ID NO: 4) |

**Example 13:** Selection of antibodies for sandwich assays

Biotinylated and Ruthenylated clones 2H8, 3H8, 8G5, 9E7 11A5, 11C10, 14C8, 13G6 were tested in multiple sandwiches for identification of sandwich partners for further Elecsys immunoassay development. Relation of recognition of recombinant NT-proBMP10 (22-312) at 0,01 ng/ml with blank values across sandwich combinations (Table 13) reflects a signal-to-noise ratio of 1,16 and 1,22 for the combination of clones 11A2 and 11C2 either on the biotinylated or ruthenylated side. The combination 13G6 and 11C10 reflects ratios of 1,22 and 1,23, indicating high grade of performance independent of the orientation. A similar observation is made for the combination of 3H8 and 9E7 with ratios of 1,22 and 1,23 respectively but higher blank values compared to the combination of 11C10 and 13G6. The other combinations described achieve good ratios, yet only for one sandwich orientation.

Table 13: Antibody sandwich characteristics regarding maximum signal readout on Elecsys ECLIA measuring cell for concentration levels of recombinant NT-proBMP10 (22-312) at 0,01ng/ml and the signal-to-noise ratio of 0,01ng/ml divided by the blank value without spiked-in recombinant protein (S/N).

| [ng/ml] BMP10 (312) | Clone-IgG-Ru/Clone-IgG-Bi | | | | | | | | | |
|---|---|---|---|---|---|---|---|---|---|---|
| | 11A5/ 11C10 | 11A5/ 3H8 | 13G6/ 11C10 | 11C10/ 11A5 | 3H8/ 9E7 | 13G6/ 3H8 | 11C10/ 13G6 | 9E7/ 3H8 | 3H8/ 11C10 | 11C10/ 3H8 |
| 0,01 | 1147 | 1193 | 865,5 | 1012 | 1668 | 922 | 1005 | 1087,5 | 1756,5 | 1114,5 |
| 0 | 987,5 | 1015 | 712 | 827 | 1363 | 748 | 815 | 884,5 | 1430 | 859,5 |
| SIN | 1,16 | 1,18 | 1,22 | 1,22 | 1,22 | 1,23 | 1,23 | 1,23 | 1,23 | 1,30 |

Recognition of 20 native samples derived from healthy donors (Table 14) shows the range of detection for NT-proBMP10 in native serum samples, allowing to determine baseline values for clinical sample measurement. These are in a comparable range for both Ru- and Bi- orientations of 11C10 and 13G6. A similar range is detected by 11C10 and 11A5 also in both orientations.

Table 14: Antibody sandwich characteristics regarding signal readout on Elecsys ECLIA measuring cell for concentration levels of 20 native healthy human serum samples depicted are minimum, maximum, and median values.

| | Clone-IgG-Ru/Clone-IgG-Bi | | | |
|---|---|---|---|---|
| | 11C10/ 13G6 | 11C10/ 11A5 | 3G6/ 11C10 | 11A5/ 11C10 |
| median | 35546 | 42507 | 21968 | 31176,5 |
| min | 22175 | 20474 | 15786 | 23059 |
| max | 52515 | 63268 | 29712 | 44245 |

**Example 14:** Biomarker measurement (Exemplary method for the detection of NT-proBMP10)

Serum and plasma concentrations of the biomarker NT-proBMP10 were measured with the commercialized Elecsys® reagents from Roche Diagnostics (Mannheim, Germany). The biomarker NT-proBMP10 was measured with prototype Elecsys® reagents from Roche Diagnostics (Mannheim, Germany).

NT-proBMP10-Assay using biotinylated rabbit MAB<NT-proBMP10>F(ab')2-Bi and ruthenylated MAB<NT-proBMP10>-Ru :

An electrochemiluminescence immunoassay (ECLIA) for the specific measurement of NT-proBMP10 in particular in human serum or plasma samples was developed using the Elecsys® cobas analyzer e601. The Elecsys NT-proBMP10 immunoassay is an electrochemiluminescence im-munoassay (ECLIA) that functions via the sandwich principle. There are two antibodies included in the assay, namely a biotinylated monoclonal antibody F(ab')2-fragment MAB<BMP10> (MAB<BMP10_22-312>Bi; capture antibody, such as 11C10) and a ruthenylated monoclonal anti-BMP10 antibody MAB<BMP10> (MAB<BMP10_22-312>-Ru; detection antibody, such as 13G6), which form sandwich immunoassay complexes with NT-proBMP10 in the sample. The complexes are then bound to solid-phase streptavidin-coated microparticles. These are captured magnetically onto the electrode surface, leading to chemiluminescence emission upon application of voltage to the electrode, which is measured by a photomultiplier. Results are determined via an instrument-specific calibration curve determined by series of 6 calibrators with different concentrations of NT-proBMP10 across the measuring range. Samples are measured applying assay protocol 2 with pipetting volumes of 20 ul of the sample, 75 ul of reagent 1 (R1), 75 $\mu$l of reagent 2 (R2) and 30 $\mu$l of magnetic beads. R1 is containing MAB<BMP10>F(ab') 2-Bi in phosphate reaction buffer and reagent 2 (R2) is containing MAB<BMP10>-Ru in the same reaction buffer.

**Example 15: Prediction of silent brain infarcts (LNCCI and SNCI) based on circulating BMP-10 levels**

BMP-10 in the assessment of silent brain infarcts provides a method to

1. Predicting the risk of silent brain infarcts in patients with atrial fibrillation based on circulating BMP-10 levels in serum/plasma (SWISSAF study, Table 15 + 16)

2. Improving the prediction of clinical accuracy of clinical stroke risk scores for silent brain infarcts based on circulating BMP-10 levels in serum/plasma (e.g. $CHA_2DS_2$-VASc, CHADS2 score) (SWISS AF study, Table 17)

The ability of circulating BMP-10 to predict the risk for the occurrence of silent infarcts was assessed in the SWISS AF study (Conen D., Forum Med Suisse 2012;12:860-862; Conen et al., Swiss Med Wkly. 2017;147). Patients of the SWISS AF cohort have a median age of 74 years, a rate of prior clinical strokes or TIA of 20%, a rate of vascular diseases of 34% and a history of diabetes of 17%.

BMP-10 was measured in the complete SWISS AF study with a pre-commercial assays were used for bone morphogenetic protein 10 (BMP-10) (high-throughput Elecsys® immunoassays; Roche Diagnostics, Mannheim, Germany). For detection of BMP-10 a sandwich-immunoassay was developed for the cobas Elecsys® ECLIA platform.

Because the estimates from a naive proportional hazard model on the case control cohort would be biased (due to the altered proportion of cases to controls), a weighted proportional hazard model was used. Weights are based on the inverse probability for each patient to be selected for the case control cohort. In order to get estimates for the absolute survival rates in the two groups based on the dichotomized baseline BMP-10 measurement (<=median vs > median) a weighted version of the Kaplan-Meier plot was created.

In order to assess the ability of BMP-10 to improve existing risk scores for the prognosis of stroke the $CHADS_2$ the $CHA_2DS_2$-VASc and the ABC score were extended by BMP-10 (log2 transformed). Extension was done by creating a portioned hazard model including BMP-10 and the respective risk score as independent variables.

The c-indices of the $CHADS_2$, the $CHA_2DS_2$-VASc and ABC score were compared to the c- indices of these extended models. For the calculation of the c-index in the case-cohort setting a weighted version of the c-index was used as proposed in Ganna (2011).

Results

**Table 15:** Significant altered circulating levels of BMP-10 in patients with brain lesions (SWISS AF study). Brain lesions include LNCCI and SNCI. Values are median (1st; 3rd Quartile).

| Any brain lesions on bMRI | No | Yes | | All |
|---|---|---|---|---|
| | n=1078 | n=631 | p | n=1709 |
| BMP-10 ng/ml | 2.17 (1.87; 2.51) | 2.32 (1.98; 2.73) | <0.001 | 2.23 (1.91; 2.60) |

Patients with LNCCI or SNCI on the bMRI were older (75.0 vs 68.1 years, p<0.0001), had more often permanent AF (28.4 vs 17.8%, p=0.0002), higher systolic BP levels (136.7 vs 131.3 mmHg, p<0.0001) and a higher CHA2DS2-VASc score (3.2 vs 2.1 points, p<0.0001), but showed no difference in the rate of oral anticoagulation (90.3 vs 88.5%, p=0.32). As shown in Table 15, BMP-10 has significantly higher levels in patients with brain lesions.

As demonstrated in Table 15, the risk of silent brain infarcts in patients with atrial fibrillation can be assessed based on circulating BMP-10 levels in serum/plasma.

**Table 16:** Significant multivariable-adjusted hazard ratios (HR) (95% confidence intervals (CI)) for BMP-10 associated with silent infarcts (presence of LNCCI and SNCI)

| Biomarkers | Presence of LNCCI | |
|---|---|---|
| Bone morphogenetic protein 10 | OR (95% CI) | p-value |
| Model 1 | 1.73 (1.17; 2.56) | 0.006 |
| Model 2 | 1.57 (1.05; 2.34) | 0.028 |
| Model 1 was adjusted for age and sex. Model 2 was additionally adjusted for systolic blood pressure, prior major bleeding, diabetes, peripheral vascular disease, BMI, smoking status, use of oral anticoagulation and antiplatelet medication. | | |

Biomarkers were logarithmized.

As shown in Table 16, BMP-10 was significantly associated with LNCCI after multivariable adjustments for age and sex (Model1) or for age, sex, systolic blood pressure, prior major bleeding, diabetes, peripheral vascular disease, BMI, smoking status, use of oral anticoagulation and antiplatelet medication.

Therefore the risk of silent brain infarcts in patients with atrial fibrillation can be assessed based on circulating BMP-10 levels in serum/plasma.

**Table 17:** Significant improvement of the CHAD2DS2-VASc score with BMP-10 for the relation to large non-cortical infarcts.

| Predictor variables were logarithmized biomarkers in addition to CHADS2-VA2SC score, the outcome variable was presence/absence of large non-cortical and cortical infarcts. | |
|---|---|
| **Biomarkers** | **Presence of LNCCI or SNCI** |
| | AUC |
| CHA2DS2-VASc | 0.696 (0.67-0.721) |
| CHA2DS2-VASc + Bone morphogenetic protein 10 | 0.699 (0.673-0.724) |

When we added individual biomarkers to the CHA2DS2-VASc score, the AUC (95% CI) was improved by BMP-10 0.699 (0.673-0.724) as demonstrated in Table 17.

The combination of BMP-10 with clinical parameters of the CHA2DS2-VASC score well predicted clinically silent brain infarcts and outperformed the CHA2DS2-VASc score. Early clinical identification of patients at risk of cognitive decline might allow for better diagnostic and preventive measures.

**Example 16: Prediction of White Matter Lesions based on circulating BMP-10 levels**

Data in the SWISS-AF data shows that BMP-10 correlates with existence of large non-cortical and cortical infarcts (LNCCI) in patients.

The extent of matter lesions can be expressed by the Fazekas score (Fazekas, JB Chawluk, A Alavi, HI Hurtig, and RA Zimmerman American Journal of Roentgenology 1987 149:2, 351-356). The Fazekas score is ranging from 0 to 3. 0 indicates no WML, 1 mild WML, 2 moderate WMI, and 3 severe WML.

In order to compare the association of BMP-10 with large non-cortical and cortical infarcts (LNCCI) patients were classified in two groups, Fazekas Score < 2 (no) vs Fazekas Score ≥ 2 (yes). Figure 14 shows that BMP-10 is increased in patients with moderate or severe WMLs versus patients with mild or no WMI,s.

WML extent can be caused by clinical silent strokes (Wang Y, Liu G, Hong D, Chen F, Ji X, Cao G. White matter injury in ischemic stroke. Prog Neurobiol. 2016;141:45-60. doi:10.1016/j.pneurobio.2016.04.005). This further advocates the usefulness of BMP-10 to predict the risk for clinical stroke.

The ability of circulating BMP-10 to discriminate between patients with Fazekas Score < 2 (no) versus Fazekas Score ≥ 2 (yes) is indicated by the AUC of 0.62. White matter changes in the brain of dementia patients. Advanced age and changes in WMI, scores have been described to be associated with severity of dementia in Alzheimers disease patients (Kao et al., 2019).

Age is also an important predictor of clinical stroke. Therefore, it is plausible that data of significantly increased BMP-10 levels in the circulation indicate not only moderate or severe large non-cortical and cortical infarcts (LNCCI), but also indicate age related brain diseases, e.g. vascular dementia.

**Claims**

1. A method for assessing the extent of white matter lesions in a subject, said method comprising:

   a) determining the amount of one or more BMP10-type peptides in a blood, serum or plasma sample from the subject, and
   b) assessing the extent of white matter lesions in a subject based on the amount determined in step a),

   wherein step a) comprises contacting the sample with at least one agent that is capable of binding within amino acid region 37 to 299 of the polypeptide shown in SEQ ID NO: 1.

2. A method for predicting dementia in a subject, said method comprising:

   a) determining the amount of one or more BMP10-type peptides in a blood, serum or plasma sample from the subject,
   b) comparing the amount determined in step a) to a reference, and
   c) predicting the risk of dementia in said subject,

   wherein step a) comprises contacting the sample with at least one agent that is capable of binding within amino acid region 37 to 299 of the polypeptide shown in SEQ ID NO: 1.

3. The method of claim 2, wherein said dementia is vascular dementia and/or Alzheimer's disease.

4. A method for the assessment whether a subject has experienced one or more silent strokes, said method comprising

   a) determining the amount one or more BMP10-type peptides in a blood, serum or plasma sample from the subject,
   b) comparing the amount determined in step a) to a reference, and
   c) assessing whether a subject has experienced one or more silent strokes,

   wherein step a) comprises contacting the sample with at least one agent that is capable of binding within amino acid region 37 to 299 of the polypeptide shown in SEQ ID NO: 1.

5. The method of any one claims 1 to 4, wherein the BMP10-type peptide is NT-proBMP10.

6. The method of any one of claims 1 to 5, wherein said agent is a monoclonal antibody, or antigen binding fragment thereof.

7. The method of claim 6, wherein the monoclonal antibody, or antigen-binding fragment thereof, is capable of binding

   a) to an epitope contained in amino acid region 171 to 185 of SEQ ID NO: 1 (LESKGDNEGERNMLV, SEQ ID NO:

3), such as an epitope contained in amino acid region 173 to 181 of SEQ ID NO: 1 (SKGDNEGER, SEQ ID NO: 4),

b) to an epitope contained in amino acid region 37 to 47 of the polypeptide shown in SEQ ID NO: 1 (SLFGDVFSEQD, SEQ ID NO 2), or

c) to an epitope contained in amino acid region 291 to 299 of SEQ ID NO: 1 (SSGPGEEAL, SEQ ID NO: 5).

8. The method of claims 6 or 7, wherein the monoclonal antibody, or antigen-binding fragment thereof, comprises

I. a heavy chain variable domain having a sequence as shown in SEQ ID NO: 7, and a light chain variable domain having a sequence as shown in SEQ ID NO: 16;

II. a heavy chain variable domain having a sequence as shown in SEQ ID NO: 8, and a light chain variable domain having a sequence as shown in SEQ ID NO: 17;

III. a heavy chain variable domain having a sequence as shown in SEQ ID NO: 9, and a light chain variable domain having a sequence as shown in SEQ ID NO: 18;

IV. a heavy chain variable domain having a sequence as shown in SEQ ID NO: 10, and a light chain variable domain having a sequence as shown in SEQ ID NO: 19;

V. a heavy chain variable domain having a sequence as shown in SEQ ID NO: 11, and a light chain variable domain having a sequence as shown in SEQ ID NO: 20;

VI. a heavy chain variable domain having a sequence as shown in SEQ ID NO: 12, and a light chain variable domain having a sequence as shown in SEQ ID NO: 21;

VII. a heavy chain variable domain having a sequence as shown in SEQ ID NO: 13, and a light chain variable domain having a sequence as shown in SEQ ID NO: 22;

VIII. a heavy chain variable domain having a sequence as shown in SEQ ID NO: 14, and a light chain variable domain having a sequence as shown in SEQ ID NO: 23; or

IX. a heavy chain variable domain having a sequence as shown in SEQ ID NO: 15, and a light chain variable domain having a sequence as shown in SEQ ID NO: 24.

9. The method of any one of claims 6 to 8, wherein the monoclonal antibody, or antigen-binding fragment thereof, comprises

(a) the heavy chain CDRH1 of SEQ ID NO: 28 (NYAMS), the heavy chain CDRH2 of SEQ ID NO: 46 (YISASGNTYYASWVKG) and the heavy chain CDRH3 of SEQ ID NO: 64 ( GYSGWISGTWA), the light chain CDRL1 of SEQ ID NO: 37 (QSSQSVVNNNRLS), the light chain CDRL2 of SEQ ID NO: 55 (RASTLAS) and the light chain CDRL3 of SEQ ID NO: 73 (LGDYVSYSEAA) or

(b) the heavy chain CDRH1 of SEQ ID NO: 27 (RNLMS), the heavy chain CDRH2 of SEQ ID NO: 45 (SINFRNITWYASWAKG), the heavy chain CDRH3 of SEQ ID NO: 63 (GVYVNSNGYYSL), the light chain CDRL1 of SEQ ID NO: 36 (QASQSVSNLLA), the light chain CDRL2 of SEQ ID NO: 54 (GASKLES), the light chain CDRL3 of SEQ ID NO: 72 (QTYWGGDGTSYLNP).

**Figure 1**

**Figure 2**

Figure 3

Figure 4

**Figure 5**

Figure 6

Figure 6

## Figure 7

Figure 8

Kaplan Meier Plot

**Figure 9**

Fig. 10

A

B

C

D

E

F

G

H

Fig. 11

**Fig. 12**

```
        10          20          30          40    3H8   50
MGSLVLTLCA LFCLAAYLVS GSPIMNLEQS PLEEDMSLFG DVFSEQDGVD
        60          70          80          90          100
FNTLLQSMKD EFLKTLNLSD IPTQDSAKVD PPEYMLELYN KFATDRTSMP
       110         120         130         140         150
SANIIRSFKN EDLFSQPVSF NGLRKYPLLF NVSIPHHEEV IMAELRLYTL
       160         170    13G6 180   11A5 190         200
VQRDRMIYDG VDRKITIFEV LESKGDNEGE RNMLVLVSGE IYGTNSEWET
       210         220         230         240         250
FDVTDAIRRW QKSGSSTHQL EVHIESKHDE AEDASSGRLE IDTSAQNKHN
       260         270         280         290    2H8  300
PLLIVFSDDQ SSDKERKEEL NEMISHEQLP ELDNLGLDSF SSGPGEEAIL
```

Amino acid region 1 to 300 of SEQ ID NO: 1

**Fig. 13**

**Fig. 14**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8287868 B
- US 5932216 A
- US 2013209490 A
- US 20120213782 A
- EP 2019072042 W
- WO 02089657 A
- WO 02083913 A
- EP 0648228 B1
- US 4016043 A
- US 4424279 A
- US 4018653 A
- WO 2012107419 A

### Non-patent literature cited in the description

- **CHUGH S.S. et al.** *Circulation*, 2014, vol. 129, 837-47
- **HART et al.** *Ann Intern Med*, 2007, vol. 146 (12), 857-67
- **GO AS et al.** *JAMA*, 2001, vol. 285 (18), 2370-5
- **LATINI R. et al.** *J Intern Med.*, February 2011, vol. 269 (2), 160-71
- **DAVID et al.** *Blood*, 2007, vol. 109 (5), 1953-61
- **KIENAST**. *J. Biol. Chem.*, 2016, vol. 293 (28), 10963-10974
- **SUSAN-RESIGA et al.** *J Biol Chem.*, 01 July 2011, vol. 286 (26), 22785-94
- **YADIN et al.** *CYTOGFR*, 2016, vol. 27, 13-34
- **RICARD et al.** *Blood*, 21 June 2012, vol. 119 (25), 6162-6171
- **HUANG et al.** *J Clin Invest.*, 2012, vol. 122 (10), 3678-3691
- **SUN et al.** *J Cell Biochem.*, 2014, vol. 115 (11), 1868-1876
- **JIANG et al.** *JBC*, 2016, vol. 291 (6), 2954-2966
- **KAHR et al.** *Plos ONE*, 2010, vol. 6 (10), e26389
- **TILLET**. *J. Biol. Chem.*, 2018, vol. 293 (28), 10963-10974
- **WANG Y ; LIU G ; HONG D ; CHEN F ; JI X ; CAO G.** White matter injury in ischemic stroke.. *Prog Neurobiol*, 2016, vol. 141, 45-60
- **DOWDY ; WEARDEN**. Statistics for Research. John Wiley & Sons, 1983
- **FUSTER V. et al.** *Circulation*, 2006, vol. 114 (7), e257-354
- **YADIN et al.** *CYTOGFR 2016*, 2016, vol. 27, 13-34
- **BONOW RO. et al.** *Circulation*, 1996, vol. 93, 1946-1950
- **BONOW RO et al.** New Insights into the cardiac natriuretic peptides.. *Circulation*, 1996, vol. 93, 1946-1950
- **PEETERS et al.** *Biochem. J.*, 1991, vol. 276, 203-207
- **STORCH et al.** *Biochem. Biophys. Acta*, 2000, vol. 1486, 28-44
- **UHLÉN M. et al.** *Science*, 2015, vol. 347 (6220), 1260419
- **BECHARD D et al.** *J Biol Chem*, 2001, vol. 276 (51), 48341-48349
- **LASSALE P. et al.** *J. Biol. Chem.*, 1996, vol. 271, 20458-20464
- **MAISONPIERRE et al.** *Science*, 1997, vol. 277, 55-60
- **RICHTER, M.M.** *Chem. Rev.*, 2004, vol. 104, 3003-3036
- **ZWEIG MH. et al.** *Clin. Chem.*, 1993, vol. 39, 561-577
- *J Am Heart Assoc.*, 2013, vol. 2, e004549
- **HIJAZI**. *The Lancet*, 2016, vol. 387, 2302-2311
- **FUSTER V et al.** *Circulation*, 2011, vol. 123, e269-e367
- **FAZEKAS ; JB CHAWLUK ; A ALAVI ; HI HURTIG ; RA ZIMMERMAN**. *American Journal of Roentgenology*, 1987, vol. 149 (2), 351-356
- **CONEN et al.** *J Am Coll Cardiol*, 2019, vol. 73, 989-99
- *J. Am. Coll. Cardiol.*, 2001, vol. 38, 2101-2113
- *J. Am. Coll. Cardiol.*, 2005, vol. 46, e1-e82
- **HUNT**. *Journal of the American College of Cardiology*, 20 September 2005, vol. 46 (6), e1-e82
- **KABAT et al.** Sequences of Proteins of Immunological Interest. US Dept. of Health and Human Services, 1991, 3242
- **NEEDLEMAN ; WUNSCH**. *J. Mol. Biol.*, 1970, vol. 48, 443

- **PEARSON** ; **LIPMAN**. *Proc. Natl. Acad. Sci.*, 1988, vol. 85, 2444
- **NEEDLEMAN** ; **WUNSCH**. *J. Mol. Biol.*, 1970, vol. 48, 444-453
- **RICE, P.** ; **LONGDEN, I.** ; **BLEASBY, A.** EMBOSS: The European Molecular Biology Open Software Suite. *Trends in Genetics*, 2000, vol. 16 (6), 276-277
- **CONEN D.** *Swiss Med Wkly.*, 10 July 2017, vol. 147, w14467
- **KNECHT S**. *International Journal of Cardiology*, 2014, vol. 176 (3), 645-650
- **ZELJKOVIC I.** *Biochem Med.*, 2019, vol. 29, 020902
- **SEEBER et al.** *PLoS One.*, 04 February 2014, vol. 9 (2)
- **SEEBER et al.** *PLoS One*, 04 February 2014, vol. 9 (2)
- **CONEN D.** *Forum Med Suisse*, 2012, vol. 12, 860-862
- **CONEN et al.** *Swiss Med Wkly.*, 2017, vol. 147
- **WANG Y** ; **LIU G** ; **HONG D** ; **CHEN F** ; **JI X** ; **CAO G.** White matter injury in ischemic stroke.. *Prog Neurobiol.*, 2016, vol. 141, 45-60